# EUROPEAN PATENT SPECIFICATION

(11) **EP 1 537 214 B1**
(45) Date of publication and mention of the grant of the patent: **01.03.2006**
(21) Application number: 03770256.0
(22) Date of filing: 29.08.2003
(51) Int. Cl.: C12N 15/64, A61K 39/00

(54) **REGULATED BACTERIAL LYSIS FOR GENE VACCINE VECTOR DELIVERY AND ANTIGEN RELEASE**
KONTROLLIERTE BAKTERIELLE LYSE ZUR VERABREICHUNG VON DNA VAKZINVEKTOREN UND IMPFANTIGEN
LYSE BACTERIENNE REGULEE DESTINEE A L'ADMINISTRATION D'UN VECTEUR VACCINAL GENETIQUE ET A LA LIBERATION D'UN ANTIGENE

(30) Priority: 01.09.2002 US 407522 P
(43) Date of publication of application: 08.06.2005
(73) Proprietor: Washington University, St. Louis, MO 63130 (US)
(72) Inventor: CURTISS, Roy, III, St. Louis, MO 63112-1009 (US); KONG, Wei, St. Louis, MO 63105 (US)
(74) Representative: Brasnett, Adrian Hugh
(86) International application number: PCT/US2003/026883
(87) International publication number: WO 2004/020643

(56) References cited:
- WO-A-02/059292
- KONG W ET AL: "Construction and application of host-vector systems for DNA vaccine vector delivery." ABSTRACTS OF THE GENERAL MEETING OF THE AMERICAN SOCIETY FOR, vol. 103, 2003, pages Z-016, XP009026097 103rd American Society for Microbiology General Meeting;Washington, DC, USA; May 18-22, 2003, 2003 ISSN: 1060-2011 (ISSN print)
- KONG W ET AL: "Regulated bacterial lysis for antigen release." ABSTRACTS OF THE GENERAL MEETING OF THE AMERICAN SOCIETY FOR, vol. 103, 2003, pages E-031, XP001179186 103rd American Society for Microbiology General Meeting;Washington, DC, USA; May 18-22, 2003, 2003 ISSN: 1060-2011 (ISSN print)
- TACKET C O ET AL: "Safety and immunogenicity in humans of an attenuated Salmonella typhi vaccine vector strain expressing plasmid-encoded hepatitis B antigens stabilized by the asd-balanced lethal vector system" INFECTION AND IMMUNITY, AMERICAN SOCIETY FOR MICROBIOLOGY. WASHINGTON, US, vol. 65, no. 8, August 1997 (1997-08), pages 3381-3385, XP002239901 ISSN: 0019-9567
- NAKAYAMA K ET AL: "CONSTRUCTION OF AN ASD+ EXPRESSION-CLONING VECTOR: STABLE MAINTAINANCE AND HIGH LEVEL EXPRESSION OF CLONED GENES IN A SALMONELLA VACCINE STRAIN" BIO/TECHNOLOGY, NATURE PUBLISHING CO. NEW YORK, US, vol. 6, no. 6, 1 June 1988 (1988-06-01), pages 693-697, XP000578379 ISSN: 0733-222X

## Description

### Reference to Government Grant

This invention was made with government support under Grant Numbers NIH DE06669 and USDA 099-35204-8572. The government has certain rights in this invention.

### Related Applications

This application claims priority to provisional application no. 60/407,522, entitled "Regulated Bacterial Lysis for Genetic Vector Delivery and Antigen Release", filed on September 1, 2002.

### BACKGROUND

The invention generally relates to regulated host-vector systems for delivery of genetic vaccine vectors or desired gene products to a eukaryotic host, preparations of bacterial genetic vaccines, genetically engineered microorganisms that are useful for delivery of genetic vaccine vectors or desired gene products, and methods of use for the host-vector system.

Vaccination is a means for preparing the immune system to reduce disease symptoms, prevent horizontal transmission of infectious agents and reduce disease mortality. The immune system of a recipient host will generate an immune response to foreign antigens.

Standard vaccines include the administration of carbohydrates, peptides, polypeptides, proteins, glycosylated polypeptides, lipids, glycolipids and lipoproteins against which an immune response in a host is desired. An alternative to such standard immunization is the passive administration of antibodies to the host.

Passive immunization procedures for humans have limitations. These limitations include the cost of antibody production and the requirement for continuous administration of these antibodies. In addition, although polyclonal or monoclonal antibodies can be readily produced by routine techniques, production and purification of antibody compositions that are sufficiently safe for *in vivo* delivery is relatively expensive and time consuming.

When soluble proteins or polypeptides (*e.g.,* proteins presented directly into the bloodstream), serve as antigens, the proteins or polypeptides induce a humoral immune response in the form of circulating antibodies. In contrast, cellular immunity is generally elicited by intracellular antigens, such as antigens produced by parasites or viruses, and can also be used by the body to eliminate solid tumors. Cellular immunity involves presentation of the target antigen on the surface of antigen presenting cells by complexing with MHC class I proteins. If an antigen is synthesized in a cell and presented by both Class I and Class II molecules, both antibody production and cell-mediated immunity may result.

Genes from bacteria, viruses, fungi, and parasites have been cloned into a variety of bacteria for the purpose of directing the bacteria to express the foreign antigen or impart on the bacteria certain desired properties for use as a live vaccine. Examples include cloning the genes of *Shigella* into *E. coli* rendering the *E. coli* invasive and therefore more suitable for use as a vaccine strain, or cloning of *P. falciparum* malaria genes into *Salmonella typhimurium* which subsequently express these malaria proteins (Sadoff *et al.,* Science 240:336-338(1988); Aggrawal *et al.,* J. Exp. Med. 172:1083-1090 (1990)). All of these bacterial delivery systems require the bacteria itself to produce the antigen or the functional molecule.

*Salmonella* vector vaccines have been extensively studied. *Salmonella* vectors are capable of eliciting humoral and cellular immunity against bacterial, viral, fungal, and parasitic antigens (See e.g. Powell et al., US patent No. 5,877,159 citing Formal *et al.,* Infect. Immun., 34:746-751 (1981); Aggarwal *et al.,* J. Exp. Med 172:1083-1090 (1990); Cardenas and Clements, Clin. Microbiol. Rev. 5:328-342 (1992); Curtiss *et al.,* Dev. Biol. Stand., 82:23-33 (1994); Chatfield *et al.,* Bio/Technology, 10:888-892 (1992); and Curtiss *et al.,* J. Clin. Invest. 110:1061-1066 (2002)). These humoral responses occur in the mucosal (Cardenas *et al., supra*), and systemic compartments (Gonzalez *et al.,* J. Infect. Dis., 169:927-931 (1994); Cardenas *et al. supra;* Curtiss *et al., supra*)*.* Live oral *Salmonella* vector vaccines also elicit T cell responses against foreign antigens (Wick *et al.,* Infect. Immun., 62:4542-4548 (1994)). These include antigen-specific cytotoxic CD8⁺ T cell responses (Gonzalez *et al., supra;* Aggarwal *et al., supra;* Flynn *et al.,* Mol. Microbiol., 4:2111-2118 (1990); Turner *et al.,* Infect. Immun., 61:5374-5380 (1993); and Gao *et al.,* Infect. Immun., 60:3780-3789 (1992)).

DNA vaccines have been proposed as a means to induce *in vivo* cellular immunity by taking advantage of the recipient host's transcription and/or translation machinery. For example, a eukaryotic host is immunized with a eukaryotic expression plasmid encoding the antigen. The eukaryotic host expresses the antigen on the plasmid. DNA vaccination allows for the possibility of co-expressing immunomodulatory molecules like cytokines, co-stimulatory molecules, or antisense RNA to steer the immune response to the preferred direction. It also allows flexibility in manipulation of the DNA encoding the antigen. For additional general discussion of DNA vaccines, see for example, Pardoll and Beckerleg, Immunity, 3:165-169 (1995); Lietner *et al.,* Vaccine 18:765-777 (1999); and Lewis and Babiuk, Adv. Virus Res. 54:129-188 (1999).

Recently, naked DNA vaccines carrying eukaryotic expression cassettes have been used to immunize against influenza both in chickens (Robinson *et al.,* Vacc., 11:957-960 (1993)) and ferrets (Webster *et al.,* Vaccine 12:1495-1498 (1994)); against *Plasmodium yoelii* in mice (Hoffman *et al.,* Vacc., 12:1529-1533; (1994)); against rabies in mice (Xiang *et al.,* Virol., 199:132-140 (1994)); against human carcinoembryonic antigen in mice (Conry *et al.,* Canc. Res., 54:1164-1168 (1994)) and against hepatitis B in mice (Davis *et al.,* Vacc., 12:1503-1509 (1994); and Davis *et al*., Hum. Molec. Gen., 2:1847-1851 (1993)).

DNA vaccines may be delivered directly to the host, such as by injection into the muscles of the host. Alternatively, the DNA vaccines may be delivered to the host cells through bacterial carriers, such as *Shigella, Listeria,* or *Salmonella.*

Processing of exogenous antigens involves endocytosis, partial degradation within the endocytic vacuole, and binding to class II MHC molecules. Processing of class I-restricted antigens also appears to involve proteolysis and recognition of antigen-derived peptides bound to MHC class I molecules. Antigens synthesized within host cells, or antigens derived from intracellular bacteria that have the ability to exit the endocytic vacuole (e.g., *Listeria monocytogenes* and *Shigella* spp.), are processed and then preferentially associate with MHC class I molecules (*See e.g.* Galan *et al.,* U.S. Patent No. U.S. Patent No. 6,306,387 B1).

In the studies by Sizemore *et al.,* Science 270:299-302 (1995) the *Shigella flexneri* strain delivering the DNA vaccine vector possessed a Deltaasd mutation that imposes a requirement for diaminopimelic acid (DAP), an essential constituent of the rigid layer of the bacterial cell wall. Since DAP is only synthesized by bacteria and is unavailable in animal tissues, these bacteria commence to lyse as a consequence of DAP-less death immediately upon inoculation into an immunized animal host. Thus, many of the bacteria lyse before they enter cells of the immunized animal host. These bacteria, such as *Shigella,* most likely enter only epithelial cells lining mucosal tissues. *See e.g.* Branstrom et al. (U.S. Patent No. 5,824,538). DAP-requiring strains of *Escherichia coli* also lyse too prematurely in the immunized animal host and thus, are unable to systemically colonize the lymphoid tissues. *Listeria monocytogenes* have been genetically modified to express phage lysis genes, but the *Listeria* also lyse before the bacteria can gain access to internal lymphoid tissues. Furthermore, *L. monocytogenes* is not particularly invasive when delivered orally requiring over 10⁹ CFU to cause systemic infection in mice as compared to S. *typhimurium* cells, which require only 10⁵ CFU.

In previous studies of attenuated *Salmonella* as a carrier of DNA vaccine vectors, the *Salmonella* were attenuated by DeltaaroA mutations that enable the bacteria to invade internal lymphoid tissues. However, three or four immunizations were needed to induce a desired level of immunity. Repeated immunizations were required perhaps because a release of the DNA vaccine vector may depend on the host lysing and destroying the attenuated *Salmonella,* an action that may lead to the degradation of the DNA vaccine vector by endogenous *Salmonella* nucleases, such as endonuclease I, and/or by host enzymes that are involved in active bacterial degradation.

Once internalized into the host cells, *Salmonella* are translocated through the epithelial cells to the lamina propria where they are later taken up by macrophages. About 95% of *Salmonella* bacteria initially gain entry into the internal lymphoid tissues by attachment to and invasion of the M cells overlying the gut associated lymphoid tissue (GALT), nasal associated lymphoid tissue (NALT) and bronchial associated lymphoid tissues (BALT) after which they can gain access to local lymphoid nodes such as the mesenteric lymph nodes and subsequently to the liver and spleen. About 5% of the *Salmonella* bacteria invade through the epithelial cells to the lamina propria. During the translocation process, *Salmonella* transit inside endocytic vesicles within infected epithelial cells where they undergo limited replication similar to replication within endocytic vesicles in macrophages. Within infected animal hosts, about half of the bacteria reside outside of the cells in fluids of the interstitial space, in lymph and in blood.

Unlike other facultative intracellular pathogens such as *Listeria* or *Shigella* spp., which gain access to the cytosol shortly after entry, *Salmonella* spp. remain inside the endocytic vesicle in macrophages and epithelial cells but escape late in the cell infection when the cell succumbs either by *Salmonella*-induced necrosis or apoptosis, and the *Salmonella* are released to infect other cells. Efficient stimulation of the class-I-restricted immune response, which is known to be important for protection against viruses and a variety of intracellular pathogens (Gao *et al.,* Infect. Immun., 60:3780-3789 (1992); Yang et al., J. Immunol., 145:2281-2285 (1990)), has not been very successful. For example, mice vaccinated with avirulent strains of *Salmonella* expressing the influenza virus NP failed to mount a significant class I-restricted T cell response against the NP, although they successfully induced class II-restricted responses. (Brett *et al.,* Immunol., 80:306-312 (1993).

Galan et al. used avirulent *Salmonella* to deliver a peptide epitope fused to a *Salmonella* effector protein encoded by a nucleic acid molecule such that the chimeric protein containing the antigen was secreted by the *Salmonella* and translocated into cells within the immunized animal host dependent on a Type III secretion system (TTSS).

The location of antigens in a recombinant attenuated *Salmonella* vaccine significantly influences the level of induced immune response, especially antibody production, upon immunization of an animal. Thus, if the antigen is retained in the cytoplasm and must be released by the actions of the immunized host, immune responses are poor (Kang and Curtiss, FEMS Immunol. Med. Microbiol. Lett. 37:99-104 (2003)). On the other hand, immune responses, especially antibody responses, are much higher when the antigen is secreted into the periplasm and more so if released into the supernatant fluid (Kang *et al.,* Infect. Immun. 70:1739-1749 (2002)). In Tacket et al. 1997, Infection and Immunity, 65:3381 an Asd-balanced lethal vector system is disclosed. An Asd mutation in the Salmonella chromosome is complemented by the wild type Asd gene on a plasmid. When the cells lose the plasmid the cells lyse and liberate their antigenic content.Thus, the release of an expressed antigen by lysis of the recombinant attenuated antigen delivery strain in lymphoid tissues within the immunized animal would further enhance the immune response to the expressed antigen. This can be readily achieved by using an antigen delivery strain that exhibits a regulated delayed lysis phenotype.

Based on the discussion above, there is a need for a regulated bacterial lysis for delivery of genetic vaccine vectors and for antigen release. The regulated bacterial lysis may be used to produce a live vaccine that can effectively colonize the lymphoid tissues of the eukaryotic host without causing disease and can result in large amounts of antigen, synthesized either by the prokaryote or the eukaryotic host, to induce an effective immune response.

**SUMMARY**

In general, described herein is a host-vector system exhibiting regulated bacterial lysis to enable delivery of genetic vaccine vectors or desired gene products to a eukaryotic host. Also described herein are microorganisms comprising the host-vector system and uses of the host-vector system, such as for immunization of a eukaryotic host.

The host-vector system possesses novel and surprising features for attenuation, biological containment and immunogenicity.

Briefly, therefore, the host-vector system comprises a modified host chromosome and at least one modified vector. The host chromosome comprises an activatable control sequence, which can be activated by an inducer, a sequence that encodes a repressor, and at least one essential gene operably linked to a regulatable promoter sequence. In one embodiment, the essential gene is operably linked to a prokaryotic activator-promoter sequence. The essential gene encodes a polypeptide that is necessary for synthesis of a rigid layer of a cell wall of a prokaryote. In one embodiment, the essential gene is inactivated. In another embodiment, the essential gene is operably linked to an activatible sequence, which may be the same or different from the activatible sequence that is operably linked to the repressor. In one embodiment, where two essential genes Thus, one or more activatible sequences may be included, and one or more inducers may be used. The inducers may be the same or different.

Further, where the eukaryotic host expresses the polynucleotide encoding the desired gene product, the vector of the host-vector system comprises a eukaryotic expression cassette that comprises a eukaryotic promoter sequence, a site for insertion of a gene encoding a desired gene product, and a polyadenylation sequence.

In another embodiment, a microorganism expresses the polynucleotide encoding the desired gene product or a fusion gene product. The polynucleotide is operably linked to a regulatable promoter sequence.

The vector also comprises a prokaryotic activator-promoter sequence, at least one origin of replication (*ori*), a regulatable promoter, at least one essential gene, at least one transcription terminator sequence, and at least one CpG sequence motif that enhances immunogenicity. The regulatable prokaryotic promoter sequence may be regulated by a variety of molecules, including, but not limited to, a repressor, activator, inducer, or enhancers. One or more regulatable prokaryotic promoter sequences is included. The regulatable prokaryotic promoter sequences may be the same or different and can be regulated by the same or different molecules. The essential gene is a gene necessary for synthesis of a rigid layer of a cell wall of a prokaryote. One or more essential genes is included. The essential genes are operably linked to at least one regulatable promoter sequence, which may be the same or different. One or more transcription terminator sequences is included.

In one embodiment, the host-vector system comprises a modified host chromosome and two modified vectors. The first vector comprises i) a eukaryotic expression cassette comprising 1) a eukaryotic promoter sequence; 2) a site for insertion of a gene encoding a desired gene product; and 3) a polyadenylation sequence; ii) a prokaryotic activator-promoter sequence; iii) at least one origin of replication (ori); iv) a regulatable prokaryotic promoter, which is repressible by the repressor; v) an essential gene, wherein the essential gene is necessary for synthesis of a rigid layer of a cell wall of a prokaryote; vi) at least one transcription terminator sequence; and vii) at least one CpG sequence motif, wherein the CpG sequence motif enhances immunogenicity. The second vector comprises i) a prokaryotic activator-promoter sequence; ii) at least one origin of replication (ori); iii) a first regulatable prokaryotic promotor sequence, wherein the first regulatable prokaryotic promotor sequence is repressible by a first repressor; iv) a second regulatable prokaryotic promotor sequence, wherein the second regulatable prokaryotic promotor sequence is repressible by a second repressor; v) at least one essential gene, wherein the essential gene is necessary for synthesis of a rigid layer of a cell wall of a prokaryote; vi) at least one transcription terminator sequence; and vii) a site for insertion of a gene encoding a desired gene product.

The host-vector system may further comprise a polynucleotide encoding a desired gene product. The desired gene product may be an antigen. The antigen may derive from a variety of pathogens, including, but not limited to, a bacterium, a virus, a fungus, or a parasite. *Eimeria,* HBV and *Streptococcus* are non-limiting examples. The antigen may be a protein, polypeptide, or epitope encoded by a polynucleotide derived from the pathogen.

Further, a microorganism, such as a bacterium, preferably, *Salmonella,* may comprise the host-vector system to deliver the genetic vaccine vector or desired gene product to a vertebrate, such as a mouse, rat, bird, or human. The microorganism may deliver the genetic vaccine vector to a eukaryotic host, who then expresses the polynucleotide encoding the antigen. Alternatively, the microorganism expresses the polynucleotide encoding the desired gene product and upon lysis, delivers the desired gene product to the eukaryotic host. In an embodiment, where at least two vectors is used in the host-vector system, both the eukaryotic host expresses the desired gene product from the first vector and a bacterial host express the desired gene product from the second vector. The gene product may be an antigen.

In either instance, the microorganism, possessing the host-vector system, may be used in a prophylactic or a therapeutic composition, such as a vaccine. The vaccine may be administered to a eukaryotic host, such as a vertebrate. The vertebrate may be a mouse, rat, bird, or human. Methods of delivery of a polynucleotide encoding a desired gene product and methods of delivery of a desired gene product are also provided herein. For example, the host-vector system may be used to vaccinate poultry against coccidiosis or humans against pneumonia.

In addition to the attenuation and containment features described throughout, the host-vector system comprises additional advantageous features that enhance its effectiveness and efficiency in delivery of genetic vaccine vectors or desired gene products. For example, a modification or multiple modifications (no particular order is required) to the host-vector system may be made to delay lysis of the microorganism, to preserve the vector after lysis of the microorganism, to deprive the microorganism of other unintended means of survival, to allow protein synthesis to either continue or be inhibited while the microorganism undergoes lysis, to reduce undesirable effects associated with vaccination, to eliminate or minimize toxicity to the eukaryotic host, to enhance means for the microorganism to escape the endosomes, and to enhance desired means of antigen processing and presentation. It is intended that the host-vector system and uses thereof may contain any number of these and other features.

In addition to using the host-vector system to confer protective immunity to bacterial, viral, fungal and parasite pathogens, it can be appreciated that the host-vector system may be used for other applications, including, but not limited to, development of anti-cancer vaccines, anti-fertility treatment, and growth enhancing effects on agriculturally important animals, such as, but not limited to, poultry, swine, sheep, and cattle.

**FIGURES**

Figure 1 shows the structure of the *araC* P_{BAD} SD*-asd* vector pYA3450.

Figure 2A shows the structure of the *araC* P_{BAD} SD-GTG *asd* vector pYA3530.

Figure 2B shows the structure of the *araC* P_{BAD} P22 c2 SD-GTG *asd* vector pYA3531.

Figure 3 shows the growth of Chi8645 DeltaPmurA7::araC P_{BAD} *murA* in 1 % rodent chow, 1 % chicken feed and 1 % chicken breast meat broths with or without 0.5 % arabinose.

Figure 4 shows diagrammed structures of all defined deletion mutations and deletion-insertion mutations indicating detailed aspects of gene changes.

Figure 5A shows the structure of DNA vaccine vector pYA3650 with regulatable delayed lysis attributes and SD-GTG *murA* and SD-GTG *asd* sequences.

Figure 5B shows structure of DNA vaccine vector pYA3651 with regulatable delayed lysis attributes and SD-GTG *murA* and SD-ATG *asd* sequences.

Figure 6 shows structures of the suicide vectors pMEG-443, pMEG-611 and pMEG-902 for introducing the Delta*asdA16,* Delta*asdA19* ::TT *araC* P_{BAD} *c2* and DeltaP_{murA7}::*araC* P_{BAD} *murA* mutations, respectively.

Figure 7 shows a diagram of the transductional method of moving unmarked deletion mutations from one strain of bacteria to another using suicide vectors.

Figure 8 shows a diagram of the suicide vectors with the Delta*asdA33* and Delta*asdA183*::TT *araC* P_{BAD} *c2* constructions for use in *S. typhi* and *S. paratyphi* A.

Figure 9 shows a diagram of the construction of the suicide vector for Delta*araBAD1923.*

Figure 10 shows a diagram of the construction of the suicide vector for Delta*araE25.*

Figure 11 shows a diagram of construction of the suicide vector for Delta*araBAD23.*

Figure 12 shows a diagram of the construction of the suicide vectors for Delta*araBAD23 c2 lacI.:rrfG* TT, Delta*araBAD23 c2::rrfG* TT and Delta*araBAD lacI::rrfG* TT.

Figure 13 shows a diagram of the construction of the suicide vector for Delta*endA2311.*

Figure 14 shows a diagram of the construction of the suicide vector for Delta*endA23*::TT *araC* P_{BAD} *lacI* with improved *lacI* expression.

Figure 15 shows a diagram of the construction of the suicide vector for Delta*gmd-11.*

Figure 16 shows a diagram of the construction of the suicide vector for Delta(*gmd-fcl)-26.*

Figure 17 shows a diagram of the construction of the suicide vector for Delta*relA1123.*

Figure 18 shows a diagram of the construction of the suicide vector for the Delta*relA11:*:TT *araC* P_{BAD} *lacI* with improved *lacI* expression.

Figure 19 shows a diagram of the construction of the suicide vector for Delta*msbB48.*

Figure 20 shows a diagram of the construction of the suicide vector for Delta*fliC825.*

Figure 21 shows a diagram of the construction of the suicide vector for Delta*fljB217.*

Figure 22 shows a diagram of the construction of the suicide vector for Delta*fliC-*Var mutation.

Figure 23 shows a diagram of the construction of the suicide vector for Delta*fljB-*Var mutation.

Figure 24 shows a diagram of the construction of the suicide vector to generate the in-frame Delta*sifA26* mutation.

Figure 25 shows a diagram of the construction of the suicide vector to introduce the DeltaP_{sifA196}::TT *araC* P_{BAD} *sifA* mutation-insertion into the chromosome.

Figure 26 shows a diagram of the construction of the suicide vector to introduce the Deltaalr-3 mutation into the chromosome.

Figure 27 shows a diagram of the construction of the suicide vector to introduce the Delta*dadB4* mutation into the chromosome.

Figure 28 shows a diagram of the construction of the suicide vector to introduce the improved DeltaP_{murA11}::a*raC* P_{BAD} *murA* deletion-insertion mutation into the chromosome.

Figure 29 shows a diagram of the construction of pYA3607 with P22 P_{R} to generate anti-sense RNA for the *asd* gene.

Figure 30 shows a diagram of the construction of the regulatable lysis system vector pYA3646 from pYA3531 and pYA3607 with all intervening steps and PCR cloning reactions.

Figure 31 shows steps in the construction of pYA3646. Figure 31A shows a diagram of the construction of pYA3608. Figure 31B shows a diagram of the construction of pYA3609. Figure 31C shows a diagram of the construction of pYA3610. Figure 31D shows a diagram of the construction of pYA3624. Figure 31E shows a diagram of the construction of pYA3613. Figure 31F shows a diagram of the construction of pYA3645. Figure 31G shows a diagram of the construction of pYA3646.

Figure 32 shows a diagram of the cloning of *araC* P_{BAD} from *E*. *coli* K-12 for regulation.

Figure 33 shows the DNA nucleotide sequence of *araC* P_{BAD} region from Chi289 in pYA3624 and amino acid sequence of AraC protein.

Figure 34 shows the DNA nucleotide alignment of nucleotide sequences of the *E*. *coli* K-12 *araC* P_{BAD} region and the *E. coli B*/*r araC* P_{BAD} region.

Figure 35 shows a diagram of the construction of the regulatable lysis system vector pYA3647.

Figure 36 shows a diagram of the construction of the DNA vaccine vector pYA3650 specifying a regulatable lysis system from pVAX1 and pYA3608 with intervening steps and PCR cloning reactions.

Figure 37 shows the steps in the construction of pYA3650. Figure 37A shows a diagram of the construction of pYA3587. Figure 37B shows a diagram of the construction of pYA3611. Figure 37C shows a diagram of the construction of pYA3614. Figure 37D shows a diagram of the construction of pYA3650.

Figure 38 shows the DNA sequence of the DNA vaccine vector pYA3650. Figure 38A shows base pairs 1 to 3300. Figure 38B shows base pairs 3301 to 6759.

Figure 39 shows an oligonucleotide sequence of *rrfG* TT and multiple cloning sites in pYA3650.

Figure 40 shows the DNA and amino acid sequences of the GTG*-murA* gene of pYA3650.

Figure 41 shows the DNA and amino acid sequences of the GTG*-asd* gene of pYA3650.

Figure 42 shows a diagram of the construction of the DNA vaccine vector pYA3651 specifying a regulatable lysis system.

Figure 43 shows the DNA sequence of the DNA vaccine vector pYA3651. Figure 33A shows base pairs 1 to 3300. Figure 33B shows base pairs 3301 to 6759.

Figure 44 shows the DNA and amino acid sequences of the ATG*-asd* gene of pYA3651.

Figure 45 shows an immuno-blot analysis on *araC* P_{BAD} *asd* vectors using rabbit anti-Asd serum.

Figure 46 shows the arabinose-dependent, DAP-dependent growth of Chi8888 and the arabinose-dependent growth of Chi8888 with either pYA3650 or pYA3651.

Figure 47 shows changes in body temperature as a consequence of oral immunization of eight-week old female BALB/c mice with live host-vector systems for delivery of DNA vaccine vectors by regulatable cell lysis *in vivo.*

Figure 48 shows a diagram of the construction of pYA3674 (pYA3650 specifying expression of the *Eimeria acervulina* EASZ-240 sporozoite antigen with fusion of FLAG peptide) and pYA3675 (pYA3651 with the same insert construction).

Figure 49 shows a diagram of the construction of pYA3677 (pYA3650 specifying expression of the *Eimeria acervulina* EAMZ-250 merozoite antigen with fusion of the FLAG peptide) and pYA3678 (pYA3651 with the same insert construction).

Figure 50 shows the DNA and amino acid sequences of EASZ-240 with FLAG fusion in pYA3674 and pYA3675.

Figure 51 shows the DNA and amino acid sequences of EAMZ-250 with FLAG fusion in pYA3677 and pYA3678.

Figure 52 shows the IgG immune responses to *Salmonella* LPS and SOMPs and the *Eimeria* EASZ240 antigens in orally immunized mice.

Figure 53 shows the IgG immune responses to *Salmonella* LPS and SOMPs and the *Eimeria* EASZ240 antigens in orally immunized chickens.

Figure 54 shows a diagram of PCR cloning of the *sipB* gene in the Asd vector pYA3332.

Figure 55 shows a diagram of the construction of the pYA3646 derivative with the Ptrc - MCS - TT - pBR *ori* cassette and nucleotide sequence of Ptrc, SD and cloning sites to generate pYA3681.

Figure 56 shows a diagram of the construction of the pYA3647 derivative with the Ptrc - MCS - TT - pBR *ori* cassette to generate pYA3682.

Figure 57 shows the arabinose-dependent, DAP-dependent growth of Chi8888 and the arabinose-dependent growth of Chi8888 with either pYA3681 or pYA3682.

Figure 58 shows the data on the release or non-release of beta-galactosidase in a Chi8888 derivative with the *lacZ* gene and containing the pYA3681 plasmid with regulated delayed lysis attributes, depending on the presence or absence of arabinose in the medium.

Figure 59 diagrams the construction of pYA3712 encoding the alpha-helical domain of the *S. pneumoniae* RX1 PspA antigen using a codon-optimized sequence encoding for PspA.

Figure 60 diagrams the construction of pYA3713 encoding the alpha-helical domain of the *S. pneumoniae* RX1 PspA antigen.

Figure 61 shows a diagram of the construction of the pYA3646 derivative expressing the HBV core pre S1, S2 sequences.

Figure 62 shows the DNA and amino acid sequences of HBV core gene with pre S1 and pre S2 epitopes in pYA3646 derivative.

Figure 63 shows the construction of Alr⁺ plasmid vectors with pSC101 *ori* and p15A *ori,* respectively.

Figure 64 shows the construction of the Alr⁺ plasmid vector with pSC101 *ori* to enable fusion of antigens with T-cell epitopes to the N-terminal end of the Type III effector protein SopE.

Figure 65 diagrams the construction of a BAC vector with IncI-alpha genes and Alr⁺ in place of antibiotic resistance genes.

**DETAILED DESCRIPTION**

In the description that follows, a number of terms used in recombinant nucleic acids research and in the fields of bacteriology, virology, microbiology, immunology, genetics and animal science are utilized. In order to provide a clear and consistent understanding of the disclosure herein, including the scope to be given such terms, the following definitions are provided.

Definitions.

An "activator" refers to a DNA-binding molecule that regulates one or more genes by increasing the rate of transcription.

"Attenuated" refers to a pathogen having mutations that reduce the ability of the pathogen to elicit disease symptoms and disease in an animal, but which do not eliminate the potential of the attenuated bacterium to attach to, invade and persist in appropriate lymphoid tissues within the animal. Attenuated microbes are useful, for example, to expose an organism to a particular genetic vaccine vector or gene product, such as an antigen or a therapeutic protein, over an extended time period. "Attenuated" does not mean that a microbe of that genus or species cannot ever function as a pathogen, but that the particular microbe being used is attenuated with respect to the particular animal being tested. Attenuated strains are incapable of inducing a full suite of symptoms of the disease that is normally associated with its pathogenic counterpart. Sometimes "avirulent" is used as a substitute term for attenuated.

A "Bacterial Artificial Chromosome" (BAC) vector is a vector derived from a low copy number plasmid (one to two copies per chromosome DNA equivalent), such as mini-F, that can be stably replicated and maintained in a bacterial host. A BAC vector can be used for insertion of up to about 600 kb of DNA using gene cloning methods. This inserted DNA may come from a single source or multiple sources.

"Biological containment", as used herein, refers to a restriction on the survival of a microorganism to a targeted environment, such as within a eukaryotic host, due to modifications to the microorganism provided by the host-vector system.

A polynucleotide sequence is "codon optimized" when the codons for amino acids in the original polynucleotide that the host-vector strain rarely used for expression of highly expressed genes are replaced by codons that the host-vector strain preferentially used for expression of highly expressed genes. Usually, the codons for the amino acids arginine, leucine, isoleucine, glycine and proline are changed.

In general, "control sequences" are DNA sequences that are necessary to effect the expression of coding sequences to which they are operably linked. As such, control sequences provide sites for the action of repressors, activators, enhancers, RNA polymerase, and other transcription factors. Nonlimiting examples of such control sequences are promoters and ribosome binding sites.

Control sequences permitting expression of gene products in bacteria are distinctly different from control sequences necessary for gene expression in eukaryotic organisms such that prokaryotic control sequences generally do not function in eukaryotic cells and vice versa. The term "control sequence" can encompass those sequences from prokaryotes or eukaryotes.

An "activatible control sequence" refers to a DNA sequence that can be activated by an inducer.

Cytotoxic T lymphocytes (CTL), also known as killer T lymphocytes, are able to kill target cells that are infected with intracellular pathogens that can be bacteria, viruses, fungi, parasites or tumor cells. Macrophages, dendritic cells, and similar cells that present T-cell epitopes in association with MHC class I antigens stimulate production of CTLs.

A "derivative" is a molecule, plasmid, vector or strain, including a host-vector strain, that is derived from a parental molecule, plasmid, vector or strain, including a host-vector strain. A derivative may have less or more genetic information than the parental element from which it is derived. In some instances, a derivative might be a hybrid derived from two or more parental elements.

An "essential gene", as used herein, refers to a gene that is necessary for the prokaryote to synthesize the rigid layer of its cell wall. Examples of essential genes include, but are not limited to, *murA, murB, murC, murD, murE, murF, murG, murH* and, *murI* (which are necessary for synthesis of the murein rigid cell wall layer), *dapA, dapB, dapC, dapD, dapE, dapF* and *asd* (which are necessary for synthesis of diaminopimelic acid (DAP)), *alr* and *dadB* (which are necessary for synthesis of D-alanine), *ddlA,* and *ddlB* (which are necessary for synthesis of D-alanyl-D-alanine). Muramic acid, DAP and D-alanine are unique constituents of the rigid layer of the cell wall and are not incorporated into any other bacterial structure or component. Even though gram-positive bacteria, other than *Mycobacterium* species and several other acid-fast gram-positive bacterial genera, lack DAP in the rigid layer of their cell wall, there are numerous other genes for synthesis of muramic acid, D-alanine or D-alanyl-D-alanine to select for regulation using a regulatory sequence such as the *araC* P_{BAD} regulatory system to achieve bacterial cell lysis *in vivo.*

The term "genetic vaccine vector" may be a DNA or RNA vaccine vector. The DNA or RNA encodes at least one antigen. The antigen may derive from various sources, including, but not limited to bacteria, viruses, fungi, and parasites and from animals, including humans. "DNA vaccine vector" refers to a plasmid DNA molecule propagated in a bacterial cell that has a gene sequence encoding a desired gene product operably linked to a eukaryotic control sequence, so that the desired gene product is expressed only after introduction of the DNA vaccine vector internally into eukaryotic cells by vaccination (immunization). The DNA vaccine vector can be administered to individuals to be immunized by injection, air gun, or preferably, by use of attenuated bacteria that liberate the DNA vaccine vector on entrance into host cells of the immunized individual.

A "gene" is a biological unit of heredity. Generally, a gene is a polynucleotide sequence that encodes an RNA molecule or a polypeptide, or a mutation of said polynucleotide sequence. The gene may be a naturally occurring sequence that is capable of being expressed into an active or inactive polypeptide. The gene may also comprise a mutation, for example a point mutation, insertion, or deletion, such that it is not capable of being expressed, or such that it expresses an altered or truncated polypeptide or RNA molecule. A gene may be created by recombinant DNA methodologies. Alternatively, the gene may be synthesized by well-known synthetic methods.

A gene may be "inactivated" by any method that eliminates or substantially impairs the expression of its gene product. For example, inactivation can be achieved by mutations.

As used herein, "immune system" refers to anatomical features and mechanisms by which a multicellular animal reacts to an antigen. Such a system has both humoral and cellular components. The humoral component produces antibodies that specifically bind to the antigen. There are five types of antibodies or immunoglobulins (Ig), i.e., IgA, IgD, IgE, IgG or IgM. The cellular component or response to antigens has been well studied and widely reported. T cells can either directly kill pathogens or secrete cellular hormones, cytokines, to direct other cells to kill pathogens. A more complete description of both the humoral and cellular components is given in Roitt, Brostoff and Male, Immunology: Fourth Edition, C.V. Mosby International Ltd., London(1998).

By using vaccines, physicians and veterinarians have successfully induced humoral and cellular immunity to human pathogens, e.g. smallpox, rubella, etc., and to animal pathogens such Marek's disease virus, Newcastle disease virus, *etc.* Some vaccines may include live bacteria that express antigens or carry gene material encoding antigens to which immune responses are desired.

To initiate an immune response to pathogens or vaccines, the human body often requires dendritic cells (DC). Related to macrophages, these cells express large numbers of MHC class I/II-peptide complexes which prime helper and killer-T lymphocytes in vivo to the offending pathogens. DC's become even more effective in the presence of inflammatory cytokines, including interferon-gamma. Such cytokines cause DCs to upregulate adhesion and costimulatory molecules and, thus, to become more potent, terminally differentiated stimulators of T-cell immunity. DC's can acquire antigens along mucosal surfaces, travel to T cell-rich lymphoid tissue (such as GALT, MALT, spleen, etc.(Chen et. al., Mol. Microbiol. 21:1101-1115 (1996)), and stimulate T cell activation, proliferation, and differentiation. Because of unique surface markers and monoclonal antibodies against them, activities of DC's can be discerned in populations of other cells and can also be purified for use in various studies.

"Immunization" is a process of inducing in an animal an immune response to an antigen by administration of a vaccine. The term "vaccination" is often used interchangeably with the term immunization.

"Incompatibility" is an attribute of plasmids that governs their ability or inability to be stably maintained within the same bacterial host cell. This property can be governed by one or more genes and/or non-coding DNA sequences on the plasmid. The abbreviation "Inc", with a capital letter modifier, is used to designate the "incompatibility group" of the particular plasmid.

An "individual" treated with a vaccine of the invention is defined herein as including all vertebrates, for example, mammals, including domestic animals and humans, various species of birds, including domestic birds, particularly those of agricultural importance. In addition, mollusks and certain other invertebrates have a primitive immune system, and are included as an "individual".

As used herein, an "inducer" is an extracellular stimulus that causes an activatible control sequence to become active. The inducer provides the signal that allows the DNA sequence to be transcribed into mRNA. Non-limiting examples of inducers are small molecules (e.g. arabinose and lactose), inorganic molecules (e.g. Fe, Mg, or P), temperature alteration, osmotic stress, and starvation.

As used herein, "microbe" or "microorganism" includes bacteria, viruses, protozoa, and fungi.

A "mutation" is an alteration of a polynucleotide sequence, characterized either by an alteration in one or more nucleotide bases, or by an insertion of one or more nucleotides into the sequence, or by a deletion of one or more nucleotides from the sequence, or a combination of these.

The term "operably linked", as used herein when describing the relationship between two nucleic acid or polypeptide regions simply means that they are functionally related to each other. For example, a pre-sequence is operably linked to a peptide if it functions as a signal sequence, participating in the secretion of the mature form of the protein most probably involving cleavage of the signal sequence. A promoter is operably linked to a coding sequence if it controls the transcription of the sequence; a ribosome binding site is operably linked to a coding sequence if it is positioned so as to permit translation.

"Pathogen-associated molecular patterns" (PAMPs) are compounds like lipopolysaccharide (LPS), teichoic acid, lipoteichoic acid, peptidoglycan, flagella, bacterial CpG containing oligonucleotides, etc. that are recognized by "Toll-like receptors" (TLRs) and serve to stimulate the innate immune system, which is necessary to induce acquired immunity in an immunized individual.

A "polynucleotide" refers to a covalently-linked sequence of nucleotides in which the 3' and 5' ends on each nucleotide are joined by phosphodiester bonds. The polynucleotide may encode a gene or fragments of a gene. For example, a polynucleotide may encode the HBV pre SI and S2 epitopes or the *M tuberculosis* ESAT-6 T-cell epitopes.

The term "promoter sequence" as used herein refers to the minimal sequence sufficient to direct transcription. Also included in the invention is an enhancer sequence which may or may not be contiguous with the promoter sequence. Enhancer sequences influence promoter-dependent gene expression and may be located in the 5' or 3' regions of the native gene. Optionally, expression is cell-type specific, tissue-specific, or species specific.

A "prokaryotic activator-promoter" is a promoter that is derived from a prokaryote and/or can be utilized by a prokaryote to transcribe the DNA sequence into RNA. Activation can be achieved, for example, by an inducer.

A "regulatable promoter" is any promoter whose activity is affected by a cis or trans acting factor (e.g., an inducible promoter, such as an external signal or agent). The regulatable promoter is considered a "repressible promoter" if it can be repressed by a repressor. Non-limiting examples of repressible promoters are lambda P_{L}, lambda P_{R}, P22 P_{L}, P22 P_{R}, P_{lac}, P_{tac} and P_{trc}.

A "replicon" is a unit of replication that possesses an origin of replication termed *ori* and one or more sequences that encode proteins and/or RNA molecules that regulate initiation of replication.

As used herein, a "repressor" is a protein that is synthesized by a regulator gene and binds to an operator locus, blocking transcription of that operon.

A "transcription terminator" is an element or sequence at the 3' end of a gene that causes transcription with synthesis of RNA to cease and often is accomplished by the RNA taking on a stem-loop structure followed by a short sequence of U's.

"Transfection" is a process for the delivery of nucleic acids, usually from viruses, into cells to result in infection of those cells and the production of viruses. If the virus nucleic acid has mutations or other modifications, the transfection may not produce viruses.

"Ubiqutination" is a multi-step process whereby proteins or peptides are covalently attached to "ubiqutin" and, thus, marked for degradation in the proteosome in eukaryotic cells. This process, if rapid, can enhance presentation of T-cell epitopes to facilitate induction of a strong CTL response. If slow or absent, the process can result in higher antibody titers to a delivered or synthesized antigen.

By "vaccine" is meant an agent designed to stimulate the immune system of a living organism so that protection against future harm is provided. A particular vaccine may or may not be effective in any particular animal. Immunization refers to the process of rendering an organism immune to a disease.

"Vector" as used herein denotes a genetically engineered nucleic acid construct capable of being modified by gene recombinant techniques to incorporate any desired foreign nucleic acid sequence, which may be used as a means to introduce said sequence in a host cell, replicate it, clone it, and/or express said nucleic acid sequence, wherein said vector comprises all the necessary sequence information to enable the vector to be replicated in host cells, and/or to enable the nucleic acid sequence to be expressed, and/or to enable recombination to take place, and/or to enable the vector to be packaged in viral particles. This recitation of the properties of a vector is not meant to be exhaustive. Those skilled in the art will understand that the use of the term "vector", and its plural "vectors", can be used interchangeably, and where appropriate refer to one or more vectors as described herein.

Vectors, optionally containing a foreign nucleic acid, may be "introduced" into a host cell, tissue or organism in accordance with known techniques such as transformation, transfection using calcium-phosophate precipitated DNA, electroporation, particle bombardment, transfection with a recombinant virus or phagemid, infection with an infective viral particle, injection into tissues or microinjection of the DNA into cells or the like. Both prokaryotic and eukaryotic hosts may be employed, which may include bacteria, yeast, plants and animals, including human cells.

The term "expression vector", as used herein refers to a nucleic acid construct containing a nucleic acid sequence, which is operably linked to a suitable control sequence capable of effecting the expression of said nucleic acid in a suitable subject. Such control sequences include a promoter to effect transcription, an optional operator sequence to control such transcription, a sequence encoding suitable mRNA ribosome binding sites, and sequences that control termination of translation and transcription. The vector may be a plasmid, a phage, or a combination of sequences from both plasmids and phages, such as cosmids and phasmids. Once transformed into a suitable subject, the vector may replicate and function independently of the subject's genome, or may, in some instances, integrate into the genome itself. In the present specification, "plasmid" and "vector" are sometimes used interchangeably as a DNA plasmid vector is the most commonly used form of vector at present. However, the invention includes such other forms of expression vectors, which serve equivalent functions and which are, or become, known in the art.

"Virus-like particles" (VLPs) are particles that assemble, usually in bacteria, due to expression of one or more viral genes whose proteins self-assemble into multi-protein structures that resemble virus particles. These VPLs are non-infectious because they lack the viral genome and one or more gene products that would be necessary for infection of eukaryotic cells or animals.

The term "pathogen" as used herein refers to viruses, bacteria, fungi, protozoa, parasites, or other microbes, organisms and agents that infect cell(s) and tissues thereby causing disease or other adverse symptoms. As particularly used herein, the term "pathogen" preferably refers to agents that have the capability to infect, or avoid destruction by, macrophages. Examples of such agents include, but are not limited to, *E*. *acervulina, E. tenella, E. maxima, P. acnes, L. monocytogenes, S. typhimurium, N. gonorrhoea, M. avium, M. tuberculosis, M. leprae, B. abortus, C. albicans, L. major. Plasmodium falciparum,* Marek's disease virus, influenza virus and HBV.

**Description**

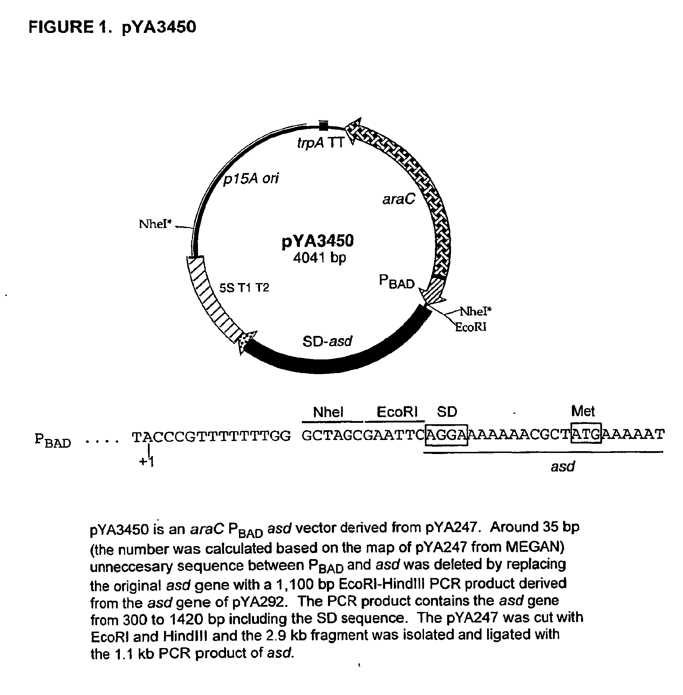

We commenced to develop a DNA vaccine delivery system using *Salmonella typhimurium* UK-1 that would colonize internal lymphoid tissues before commencing to lyse to liberate a DNA vaccine vector, without degradation, into the cytoplasm of host cells capable of active protein synthesis. We genetically modified genes encoding enzymes needed for synthesis of essential constituents of the rigid layer of the bacterial cell wall so that their expression would be turned off *in vivo* with dilution of enzyme concentration at each cell division to result in lysis after 5 to 10 cell division cycles. First, we determined whether we could use the *araC* P_{BAD} activator-promoter system on a multicopy plasmid vector to regulate synthesis of the *asd* gene product. We, thus, constructed the plasmid vector pYA3450 (Figure 1) with a p15A *ori.* The p15A *ori* causes synthesis of some 20 to 30 copies of plasmid DNA per chromosome DNA equivalent. For a DNA vaccine vector, it is desirable to have a much higher plasmid copy number such as controlled by the pUC *ori* to yield 200 to 300 plasmid copies per chromosome DNA equivalent. Nevertheless, it was prudent to work out necessary parameters for controlled cell lysis using a less demanding system with a lower plasmid copy number.

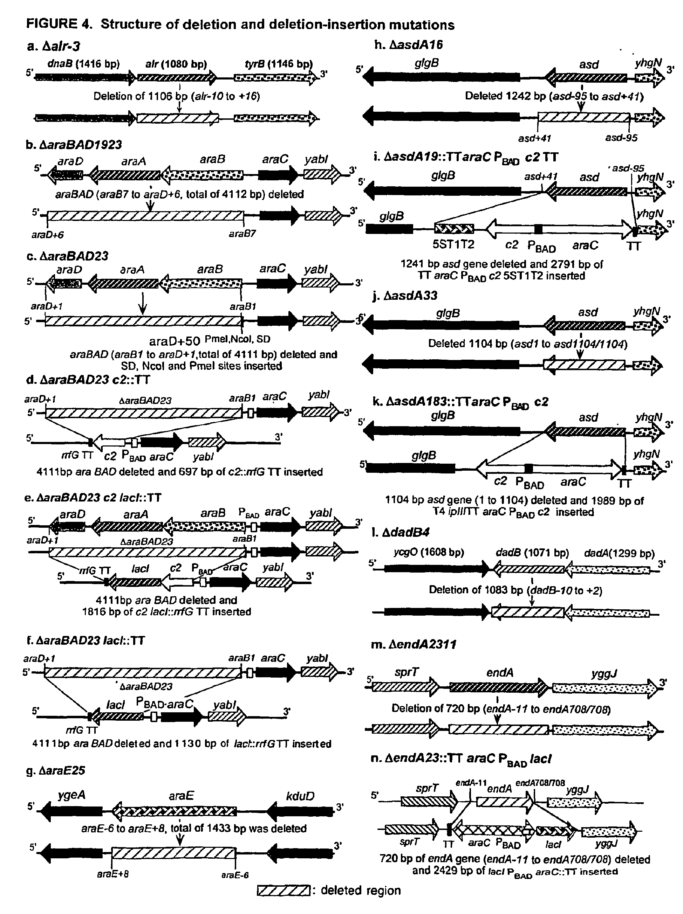

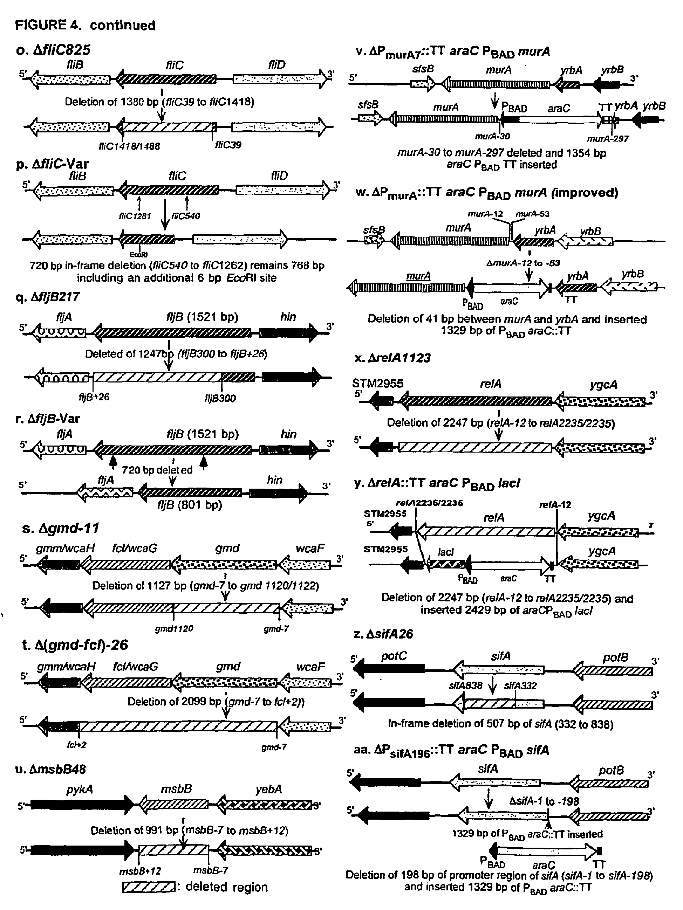

When pYA3450 was introduced into Chi8276 (Table 1), a *S. typhimurium* UK-1 strain with a Delta*asdA16* mutation (see Figure 4 h) by electroporation, the strain did not exhibit arabinose-dependent growth but instead grew very well in the absence of arabinose even after subculturing for numerous generations. This indicated that the *araC* P_{BAD} activator-promoter derived from *E. coli* B/r was leaky with a baseline of transcription sufficient to make enough Asd enzyme to catalyze the synthesis of sufficient DAP needed for cell wall biosynthesis. This Chi8276 (pYA3450) strain also retained wild-type virulence for mice with an LD₅₀ of about 10⁵ CFU, thus, rendering it unsuitable for a DNA vaccine delivery system.

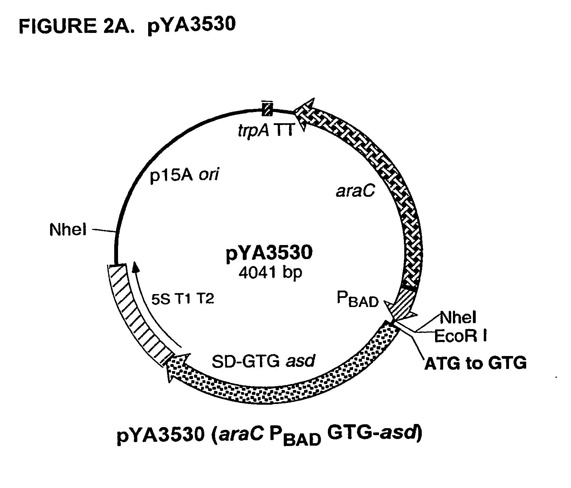

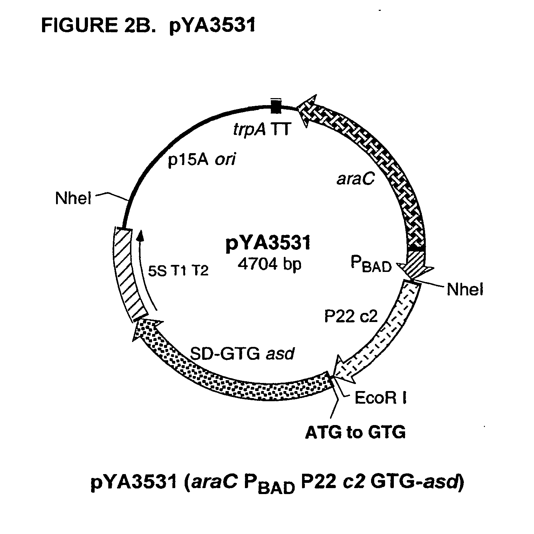

Because translation efficiency is known to be influenced by the start codon specifying the N-terminal methionine, we used site-directed mutagenesis to change the start codon for the *asd* gene from ATG to GTG in pYA3450. GTG is used to initiate translation by some 8% of the genes in *E. coli* and *Salmonella.* This generated plasmid pYA3530 (Figure 2a) that was introduced into Chi8276 by electroporation. Another derivative from pYA3450 had an insertion of the phage P22 c2 gene that was introduced into pYA3450 to yield pYA3488 (Table 2) and an ATG to GTG mutation to change the start codon for the *asd* gene. This yielded pYA3531 (Figure 2b). These recombinant strains exhibited arabinose-independent growth in a diversity of media and would not display DAP-less death and lysis in media devoid of arabinose. If the origin of replication for the plasmid specifies a high copy number, *e.g*. pBR *ori* or pUC *ori,* and not a low copy number replication origins, e.g. pSC101 *ori* or p15A *ori,* then the Asd⁺ plasmid vectors could function to complement Deltaasd chromosomal mutations after deletion of the plasmid *asd* promoter, but leaving a sequence specifying the Shine-Dalgarno (SD) ribosome binding site and the open reading frame for the Asd enzyme. (Kang et al., Infect. Immun. 70:1739-1749 (2002); Functional Balanced-Lethal Host-Vector system PCT/US01/42527, filed on 10/5/2001). Our results with *araC* P_{BAD} GTG-*asd* constructions indicate that arabinose-independent transcription of the *asd* gene is at a higher frequency on these p15A *ori* plasmids than on a plasmid lacking a *araC* P_{BAD} sequence, but has the SD*-asd* sequence.

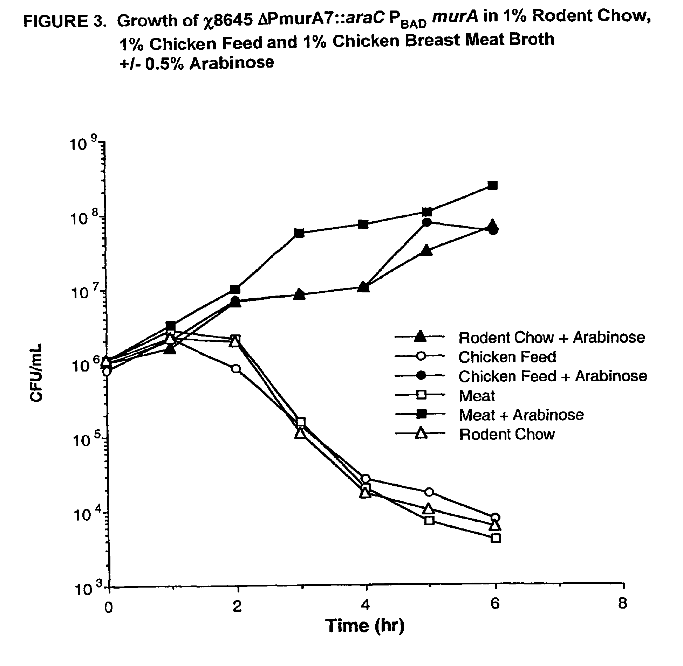

We next determined whether use of the *araC* P_{BAD} activator-promoter to regulate expression of a chromosomal gene encoding an enzyme for synthesis of an essential constituent of the rigid layer of the bacterial cell wall might not result in a strain that would exhibit arabinose-dependent growth with lysis occurring in media devoid of arabinose. We chose a construction in which the promoter for the *murA* gene was deleted and replaced by the *araC* P_{BAD} activator-promoter. The *murA* gene encodes the enzyme UDP-N-acetylglucosamine enolpyruvyltransferase (EC 2.5.1.7), an enzyme critical for the biosynthesis of muramic acid that serves as one of two repeating sugars of the rigid layer of the bacterial cell wall. The resulting strain Chi8645 (Table 1) with the DeltaP_{murA7}:: *araC* P_{BAD} *murA* construction (see Figure 4 v) exhibited arabinose-dependent growth in a diversity of media and lysis when inoculated into growth medium devoid of arabinose. As demonstrated by the results depicted in Figure 3, Chi8645 grew well when media made by resuspension of rodent chow or chicken feed or macerated chicken breast meat was supplemented with arabinose but commenced to die and lyse two hours after inoculation into the same media that was not supplemented with arabinose. These results demonstrate that free arabinose does not exist in either chicken or rodent feed and is also not present in animal tissues.

Chi8645 was quite attenuated since 3 of 4 female BALB/c mice survived a dose of 1 x 10⁹ CFU with the one mouse dying on day 14, which is relatively late compared to deaths caused by wild-type *S*. *typhimurium* UK-1 Chi3761. In challenge studies of mice immunized with Chi8645, 8 of 13 mice survived challenge with 1 x 10⁹ CFU of Chi3761. Thus Chi8645 exhibited some ability to induce protective immunity to challenge with virulent *S. typhimurium.* We next examined the ability of Chi8645 to colonize lymphoid tissues following oral inoculation of female BALB/c mice. In an experiment in which the inoculating dose was 7.9 x 10⁸ CFU, the total mean titers of Chi8645 recovered from Peyer's patches (8 to 11/mouse) on days 1, 2 and 3 following oral inoculation were 1.1 x 10², 63, and 3.3 CFU, respectively. Viable Chi8645 cells were never recovered from the spleen or any other internal lymphoid organs. Elimination of the ability to metabolize arabinose and alteration of its uptake and retention can prolong the time when arabinose is maintained in the cytoplasm of the cell to activate the AraC gene product to cause transcription of genes under the control of the P_{BAD} promoter. We introduced the Delta*araE25* mutation that eliminates a low-affinity arabinose uptake protein and the Delta*araBAD1923* mutation preventing utilization and breakdown of arabinose into Chi8645. Both the Delta*araE25* and Delta*araBAD1923* mutations (see Figures 4 g and 4 b) were introduced into Chi8645 by using a co-transductional method for moving unmarked deletion mutations by using the suicide vector originally used to generate the defined deletion mutation (Kang et al., J. Bacteriol. 184:307-312 (2002)). The resulting strain Chi8654 thus possessed the DeltaP_{murA7}:: *araC* P_{BAD} *murA* Delta*araBAD1923,* and Delta*araE25* mutations. Unfortunately, this strain was also unable to colonize internal lymphoid tissues after oral inoculation into BALB/c mice even though colonization of Peyer's patches occurred at a low level for a three-day period.

The results with strains possessing an arabinose-dependent regulatable chromosomal essential gene for cell wall biosynthesis indicated the need for a higher number of copies of the essential gene so that cells would have a sufficient enzyme content to permit 5 to 10 generations of growth prior to commencing cell wall-less death leading to lysis. In other words, the bacterial strain had to be capable of a sufficient number of generations of growth in the absence of arabinose to enable colonization of internal lymphoid organs such as the liver and spleen before lysis and liberation of the DNA vaccine vector into the cytoplasm of cells within the immunized host. We considered two approaches to enable creating a DNA vaccine delivery strain with these desired attributes. In one approach, the *araC* P_{BAD} activator-promoter could be modified to achieve less leakiness and a decreased occurrence of transcription under control of the P_{BAD} promoter in the absence of arabinose. Alternatively, we could screen other bacterial strains taken from the various species within the *Enterobacteriaceae* capable of arabinose utilization and therefore possessing an arabinose operon including the *araC* P_{BAD} activator-promoter to identify one with a greater dependence on arabinose for inducing transcription. Although we discovered a number of potential candidates, we used PCR cloning to recover the *araC* P_{BAD} activator-promoter from the selected *E. coli* K-12 strain Chi289. As a second means to preclude low-level expression of plasmid genes encoding essential cell wall enzymes, we decided to use a regulatable promoter to synthesize anti-sense mRNA to interfere with transcription of and/or block translation of mRNA transcribed from the plasmid genes encoding essential cell wall enzymes. As an additional means to ensure lysis after 5 to 10 generations of growth in the absence of arabinose, we included two essential genes necessary for synthesis of the rigid layer of the bacterial cell wall, *murA* and *asd.* As an added safety feature, we retained in the bacterial host strain for the DNA vaccine plasmid vector the chromosomal DeltaP_{murA}:: *araC* P_{BAD} *murA* deletion-insertion mutation (Figure 4 v). We used the phage P22 P_{R} to cause synthesis of the anti-sense mRNA for the *asd* and *murA* genes. P_{R} is repressed by the C2 repressor encoded by the phage P22 c2 gene. We therefore made use of the Delta*asdA19:: araC* P_{BAD} c2 deletion-insertion mutation (Figure 4 i) so that synthesis of the C2 repressor would be dependent on the presence of arabinose in the growth medium. In other words, when the DNA vaccine plasmid host strain is grown in the presence of arabinose, C2 repressor is synthesized to cause repression of P_{R} to preclude transcription of anti-sense mRNA for the *asd* and *murA* genes. As an additional means to delay onset of bacterial cell lysis, the chromosomal Delta*araBAD1923* and Delta*araE25* mutations (Figures 4 g and 4 b) were introduced into the bacterial plasmid host strain.

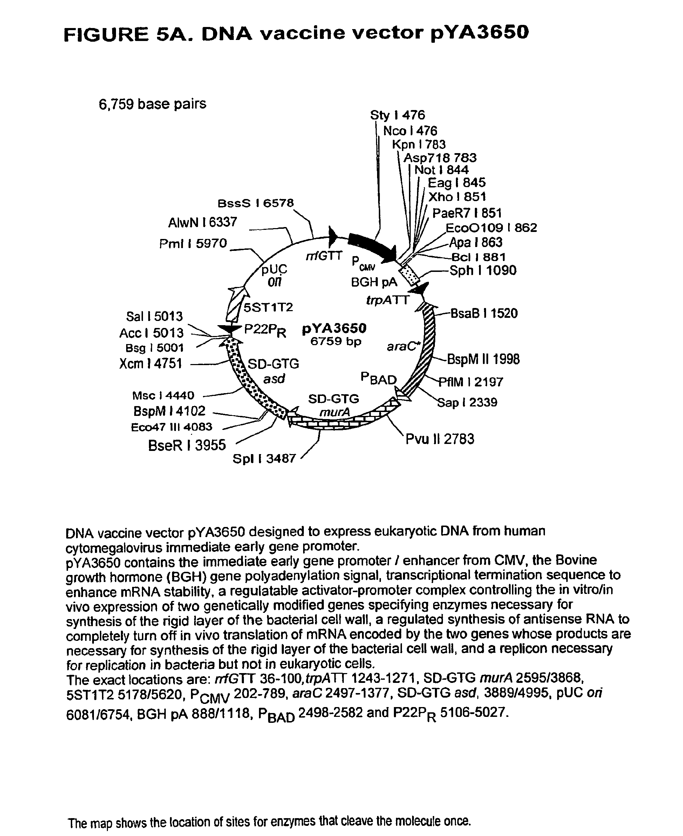

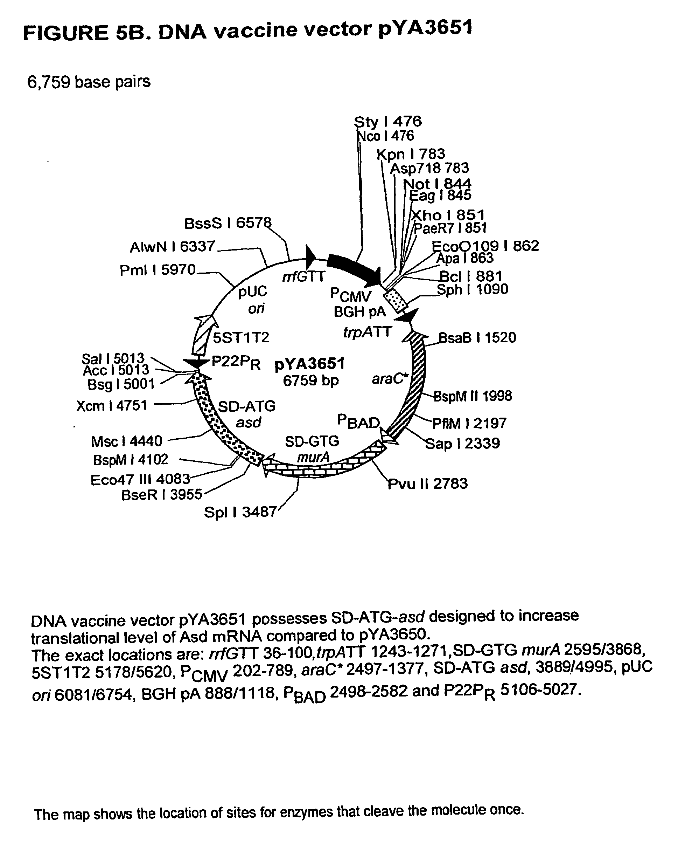

To deliver a DNA vaccine vector, we engineered the bacterial delivery strain to inhibit or preclude degradative damage to the circular DNA vaccine vector plasmid. This was accomplished by the introduction of a defined deletion mutation Delta*endA2311* (see Figure 4 m) to eliminate the periplasmic endonuclease I enzyme that is capable of cutting circular plasmid DNA. This resulted in *S. typhimurium* UK-1 strain Chi8854 which possessed the genotype *DeltaasdA19:: araC* P_{BAD} *c2* DeltaP_{murA7}:: *araC* P_{BAD} *murA* Delta*araBAD1923* Delta*araE25* Delta*endA2311.* The DNA vaccine plasmid vectors pYA3650 and pYA3651 are diagrammed in Figure 5. Both plasmids possess the pUC *ori* so that the bacterial host will possess 200 to 300 copies per chromosomal DNA equivalent. Both plasmids have a eukaryotic expression cassette including the CMV promoter, a multiple cloning site, and the bovine growth hormone gene polyadenylation sequence. Both plasmids have a less-leaky *araC* P_{BAD} activator-promoter from *E. coli* K-12. This reduces rare transcription of the *murA* and *asd* genes when arabinose is absent, thus making transcription more dependent on the presence of arabinose. Both plasmids have two genes for essential enzymes for synthesis of the rigid layer of the bacterial cell wall, *asd* and *murA.* These genes have both been modified in pYA3 650 to substitute the normal ATG start codons with the GTG codon for both the *murA* and *asd* genes and to decrease the efficiency of translation of the mRNA molecules specified by the *murA* and *asd* genes. In pYA3651, we retained the ATG start codon for the distally transcribed *asd* gene.

We have determined that for optimal lysis of the bacterial DNA vaccine vector delivery strain it is important that the concentrations of both enzymes encoded by the *murA* and *asd* genes be depleted in the same or nearly same generation of bacterial growth. Since in two-gene operons there can be differences in the translation efficiency of the mRNA and it is also possible that transcription of a two-gene operon ceases before the second gene sequence is fully transcribed, we considered that having the *asd* gene with an ATG start would enhance the likelihood that the amounts of the *asd* and *murA* encoded enzymes would be nearly the same. The synthesis of anti-sense mRNA also might be more effective in preventing expression of the proximal *asd* gene that the distal *murA* gene. Currently, optimal results are achieved with the GTG codon for the *murA* gene and either the GTG or ATG start codon for the *asd* gene. Both plasmids have the P22 P_{R} that is repressed by the C2 repressor whose synthesis is regulated by the *araC* P_{BAD} *c2* construction inserted within the inactivated chromosomal *asdA19* mutation in Chi8854. This results in synthesis of anti-sense mRNA for the *asd* and *murA* genes when arabinose is absent. Both plasmids possess three transcription terminator sequences to preclude interference in expression of gene information in one domain by expression in another domain. The pUC *ori* domain for replication, the prokaryotic regulatory domain with the *araC, murA* and *asd* genes, and the eukaryotic expression domain are all separated by transcription terminators. The two DNA vaccine plasmid vectors pYA3650 and pYA3651 (Figure 5) also possess more immuno-stimulatory CpG sequences than present in commonly used DNA vaccine vectors. The details in construction of Chi8854 and the DNA vaccine plasmid vectors pYA3650 and pYA3651 will be presented in the Examples along with presentation of data to support the host-vector system for DNA vaccine delivery to immunized animals.

Described herein is a regulated bacterial lysis for delivery of genetic vaccine vectors and desired gene products to a eukaryotic host. It is discovered that a host-vector system can be used to efficiently and effectively deliver gene vectors and desired gene products to a eukaryotic host.

As described herein, a host-vector system provides a means for regulated lysis of bacteria, which involves a novel and unobvious means for delivery of genetic vaccine vectors and desired gene products to eukaryotic hosts. The host-vector system involves both attenuation and biological containment of the bacteria. The attenuated bacteria allows for preparation of vaccines that are avirulent in eukaryotic hosts, including mice, chickens, and presumably humans. Some researchers attenuate bacteria by introducing mutations that permit environmental regulation of surface molecule synthesis such as lipopolysaccharides in gram-negative microorganisms as affected by a *galE* mutation. Bacteria can also be attenuated by introduction of mutations that impose specific nutritional requirements, such as for constituents of nucleic acids such as purines, constituents of the cell wall such as diaminopimelic acid (DAP) or that impose requirements for aromatic amino acids and vitamins derived therefrom, such as caused by aro mutations. Still other means of attenuation are achieved by mutating genes affecting global regulation of other genes. Through biological containment, the vaccine shed, for example, in feces is unable to survive in nature to cause immunization of other unintended individuals.

In an embodiment, the host-vector system has a number of desirable features. First, the host-vector system comprises a host chromosome that has been modified to comprise an activatible control sequence, such as *araC* P_{BAD}), that controls the synthesis of a regulatable gene. The *araC* P_{BAD} activator-promoter drives the arabinose-dependent expression of regulatory genes at this chromosome location. The regulatable gene encodes, for example, a repressor that represses a regulatable control sequence on a vector. In the presence of an inducer, such as arabinose (which can be supplemented in the culture media), the activatible control sequence is activated to transcribe a gene encoding the repressor, such as C2 or LacI. In one aspect, the sequence that encodes the repressor is operably-linked to the activatible control sequence. The repressor represses a regulatable promoter sequence, such as P22 P_{R} or P_{trc}, thereby preventing synthesis of the genes under the control of the regulatable promoter sequence. In the absence of the inducer, the activatible control sequence is not activated, and the repressor is not produced. Without the repressor, the regulatable promoter sequence is not repressed. Thus, synthesis of mRNA for genes (or its anti-sense strand) under the control of the regulatable promoter can occur.

In the host-vector system, the host chromosome has at least one essential gene that has been modified. In one embodiment, two essential genes are modified. In one embodiment, the essential gene is inactivated.. In another embodiment, the essential gene is operably linked to an activatible control sequence, which may be the same as or different from the activatible control sequence that controls expression of the repressor. Thus, there may be multiple activatible control sequences. These activatible control sequences may be activated by the same or different inducers. For example, a first activatible control sequence is operably linked to the sequence that encodes the repressor. The inducer may be arabinose or lactose. A second activatible control sequence may be operably linked to the essential gene.

The essential gene encodes a polypeptide that is necessary for synthesis of a rigid layer of a cell wall of a prokaryote. Any number of essential genes may be modified, such as by inactivation or regulation under the control of an activatible control sequence. For example, *murA, murB, murC, murD, murE, murF, murG, murH,* and *murI* are necessary for synthesis of the murein rigid cell wall layer. *dapA, dapB, dapC, dapD, dapE, dapF* and *asd* are necessary for synthesis of diaminopimelic acid (DAP)), *alr* and *dadB* (which are necessary for synthesis of D-alanine), *ddlA,* and *ddlB* (which are necessary for synthesis of D-alanyl-D-alanine). Muramic acid, DAP and D-alanine are unique to bacteria and are not either synthesized by or available in animals, including humans.

In one embodiment, the essential gene is *asd.* The *asd* gene encodes aspartic semialdehyde dehydrogenase, an enzyme necessary for the synthesis of diaminopimelic acid (DAP), which is an essential constituent of the rigid layer of the bacterial cell wall (Nakayama et al., 1988, Bio/Tech. 6:693-697). Inactivation of *asd* prevents the bacteria from synthesizing the rigid layer of its cell wall, which leads to lysis of the bacteria, unless either DAP is supplied in the growth medium or a wild-type *asd*^{*+*} gene is present on a plasmid within the mutant bacteria.

In another embodiment, the essential genes are *alr* and *dadB.* The *alr* gene encodes alanine racemase whereas the *dadB* gene encodes a catabolic enzyme enabling bacteria to grow on D-alanine but which in the reverse direction can cause synthesis of D-alanine from L-alanine. D-alanine is a unique constituent of the rigid layer of the bacterial cell wall. Inactivation of both the *alr* and *dadB* genes prevents the bacteria from synthesizing the rigid layer of the cell wall, which leads to lysis of the bacteria, unless either D-alanine is supplied in the growth medium or a wild-type *alr*^{*+*} or a wild-type *dadB*^{*+*} gene is present on a plasmid within the mutant bacteria.

The vector of the host-vector system comprises several advantageous features for delivery of a genetic vaccine vector. For example, the vector comprises a eukaryotic expression cassette, which comprises a eukaryotic promoter sequence, a site for insertion of a polynucleotide encoding a desired gene product, and a polyadenylation sequence. The eukaryotic promoter sequence, for example, may be P_{CMV}, which is derived from the human cytomegalovirus. A polynucleotide encoding a desired gene product may be inserted into a site for insertion of a polynucleotide encoding a desired gene product. Insertion of a polynucleotide at this site places the polynucleotide under the control of the eukaryotic promoter. If a sequence to be expressed lacks a Kozak sequence to facilitate mRNA binding to ribosomes, one is provided immediately upstream of the start codon. Through the eukaryotic promoter, the eukaryotic host expresses the desired gene product. The polyadenylation sequence may derive from a variety of sources, such as from the gene for bovine growth hormone. The polyadenylation sequence enhances the stability of mRNA and prevents its breakdown by ribonuclease.

In one embodiment, the prokaryote expresses the polypeptide encoding the desired gene product using a vector with a prokaryotic expression cassette. In this instance, the eukaryotic expression cassette is not used. Thus, the eukaryotic expression cassette need not be in the vector.

In another embodiment, the vector of the host-vector system has a prokaryotic activator-promoter sequence, at least one origin of replication (*ori*), a regulatable promoter, which is repressible by a repressor, at least one essential gene, at least one transcription terminator sequence, and at least one CpG sequence motif.

When the prokaryotic activator-promoter is activated by, for example, an inducer, such as arabinose or lactose, the prokaryote expresses a gene under the control of the prokaryotic activator-promoter. The prokaryotic activator-promoter may be *araC* P_{BAD}. In one embodiment, the prokaryotic activator-promoter is operably linked to at least one essential gene.

Further, replication of the vector can commence at the origin of replication. The *ori* may be pUC, pBR, p15A, pSC101, or pBAC. One can also include an *ori* from plasmids, such as BAC vectors, with only one or two plasmid copies per chromosomal DNA equivalent. In one embodiment, the *ori* is a pUC *ori.*

Still further, the regulatable promoter of the vector may be regulated by a repressor, such as C2 or LacI. In one embodiment, a regulatable promoter sequence may be repressed by a repressor. In another embodiment, multiple regulatable promoter sequences may be used. The regulatable promoter sequences may be repressed by the same or different repressors. In still another embodiment, a first regulatable promoter sequence is operably linked to a site for insertion of a polynucleotide encoding a desired gene product. In this way, expression of the polynucleotide inserted at this site would be under the control of the regulatable promoter sequence. The regulatable promoter may a phage P22 P_{R} or P_{trc}.

In another embodiment, the regulatable promoter sequence allows synthesis of anti-sense mRNA for the essential genes, such as *asd* and *murA* genes, in the vector, when the phage P22 repressor protein C2 is absent. This repressor protein is synthesized under the control of the *araC* P_{BAD} activator-promoter as described above. To increase repression of the regulatable promoter, multiple copies of the gene encoding the repressors may be present. For example, the amount of C2 repressor specified by a single chromosomal gene might be insufficient to repress completely a plasmid localized P_{R} such that low-level transcription leading to anti-sense mRNA for the *asd* and *murA* genes might occur. Thus, multiple copies of regulatable *c2* and/or *lacI* genes may be inserted into the chromosome.

The essential gene on the vector may complement the essential gene that has been modified on the chromosome. For example, the *asd* gene may be inactivated in the bacterial chromosome. The bacteria can no longer make DAP, which is necessary for the bacteria to synthesize a rigid layer of its cell wall. Thus, when the *asd* gene is inactivated, the bacteria undergo DAP-less death through lysis. Bacterial lysis releases genetic vaccine vectors or desired gene products to the eukaryotic host. As explained further below, this lysis may be delayed to allow the bacteria to first effectively colonize the lymphoid tissues, where macrophages and dendritic cells are abundant, and allow the bacteria to undergo 5 to 10 generations of replication, which produces a large number of genetic vaccine vectors or amounts of desired gene products. With a large number of genetic vaccine vectors, the eukaryotic host can express a large amount of antigen, which enhances the induction of a desired immune response. In another embodiment, the prokaryote expresses the gene encoding the desired gene products. Delayed lysis would also allow the bacteria time to colonize the lymphoid tissue, replicate the vector, and express the desired gene product, such as an antigen, in large amount before lysis. Upon lysis, a large amount of the desired gene product is presented to the cells of the eukaryotic host.

The essential gene on the vector allows the bacteria to survive in culture. The essential gene may be operably linked to a prokaryotic activator-promoter sequence on the vector.

In vaccination, whether the eukaryotic host or the prokaryote expresses the antigen on the vector of the host-vector system, delayed lysis allows production of a large amount of antigen for presentation to the immune system of the eukaryotic host to elicit an effective immune response. After expression of the antigen, the cells (e.g. dendritic cells) of the eukaryotic host processes (e.g. by complexing the antigen to MHC I and MHC II proteins) and presents the antigen to the immune system of the eukaryotic hosts, thereby eliciting an efficient and effective immune response. In most cases, Class I molecules present foreign proteins synthesized in a cell. For presentation by Class II, the foreign protein either can be synthesized in the cell or taken up by the cell from the outside (*i.e*., presented in the form of a free protein or peptide). If an antigen is synthesized in a cell and presented by both Class I and Class II molecules, both antibody producing B cells and cytotoxic T cells are produced. However, if an antigen originated outside of a cell and is presented only by Class II, the specific immune response is largely limited to T helper cells and antibody production (Robinson, *et al.,* Eds., THE SCIENTIFIC FUTURE OF DNA FOR IMMUNIZATION, American Academy of Microbiology, pp. 1-29, (1997)). For example, *Salmonella* can enter the host cell and lyse within the endosome or in the cytoplasm. The antigens released in the endosome are processed and presented with MHC class II molecules to recruit Th2 cells to enhance induction of antibodies by B cells whereas antigens released into the cytoplasm are processed and presented with MHC I proteins to Th1 cells to enhance induction of cellular immunity and CTLs. Alternatively, *Salmonella* can enter the host cell, lyse, and release gene vectors. Using the released gene vectors, the host cell expresses the antigens, which are processed and presented with MHC I proteins to Th1 cells. Still further, *Salmonella* can exist outside of the host cells, such as in body fluids. Through regulated lysis, *Salmonella* can release the antigens outside of the host cells. The antigens are then taken up by endosomes, which can lead to a Class II presentation for recruitment to Th2 cells that enhances antibody production.

Still further, the host-vector system comprises transcription terminator sequences. As transcription of the several genes on the vector may interfere with the effectiveness of the host-vector system, transcription terminator sequences are used to keep separate the transcription of the genes under the different promoters. For example, the transcription terminator sequence avoids *araC* P_{BAD} driven transcription beyond the inserted gene, such as the c2 and/or *lacI* gene, that might attenuate or alter the physiology of the bacteria. The transcription terminator sequence may be *rrfG.*

CpG sequence motifs may be used to enhance immunogenicity of the host-vector system. (Krieg, Annu. Rev. Immunol. 20:709-760 (2002)). The motifs are prevalent in bacteria, but are absent in vertebrates. The motifs are unmethylated in bacteria, which makes them appear foreign to vertebrates, which lack such sequences. These CpG sequence motifs enhance immunogenicity by acting like adjuvants and stimulate the human immune system. In humans, preferably GTCGTT is used to activate the human immune cells. The GTCGTT motif is also optimal for stimulation of lymphocyte proliferation in several species including cattle, sheep, goats, horses, pigs, dogs, cats, and chickens, while the GACGTT motif is preferred for rabbits and mice (Rankin *et al.,* Antisense Nucl.Acid Drug Devel. 11:333-340 (2001)). CpG DNA sequences within bacterial DNA possess adjuvant activity that enhance induction of immunity by DNA vaccines.

Any microorganism may be used to carry the host-vector system for delivery of the genetic vaccine vector or desired gene product. Preferably, the microorganism has an ability to invade and colonize the lymphoid tissue. *Salmonella* is an example of such a microorganism. *Shigella,* enteroinvasive *E. coli, Yersinia* and *Listeria* are other examples of bacteria that could be modified to exhibit regulated delayed lysis for the delivery of antigens or genetic vaccines. The lymphoid tissue may be in a liver, spleen, or a regional lymph node.

A number of advantageous modifications may be made to the host-vector system to further enhance its effectiveness and efficiency. The host-vector system may have one modification or multiple modifications, which can occur in any order.

For example, after growth in an arabinose-enriched environment and then transferred to an environment without arabinose, the bacterium still retains some arabinose. The limited amount of arabinose allows the bacterium to survive for a short period of time. This delay allows the bacterium time to replicate and colonize the lymphoid tissue. In one embodiment, to further delay lysis of the bacterium, the *araBAD* gene is mutated. The enzymes encoded by the *araBAD* genes degrades arabinose. For example, inclusion of the Delta*araBAD1923* mutation as diagrammed in Figure 4 b delays the onset of lysis because the arabinose used to induce gene expression is retained in the bacterium for a little longer.

In another embodiment, the *araE* gene is mutated. The *araE* gene encodes a transport protein. For example, the mutation of *araE* further delays onset of lysis because the arabinose taken up into cells during permissive growth does not leak out of cells via the AraE transport protein.

In still another embodiment, the genes encoding endonuclease I enzyme may be mutated. Endonuclease I enzyme degrades nucleic acids, especially circular plasmid DNA molecules that can be digested by endonucleases but not by exonucleases. When the host strain possesses a mutation that inactivates the periplasmic endonuclease I enzyme, recovery of circular plasmid DNA is improved and the efficiency of plasmid transfer to a host strain by either transformation or electroporation is enhanced. It would also not be subject to attack and breakdown when released by a bacterium undergoing regulated lysis in an immunized animal or individual.

In yet another embodiment, the *gmd* or *gmd-fcl* gene may be mutated. Bacteria experiencing perturbations in the synthesis of their cell walls, due to either inhibition of cell wall synthesis by antibiotics or by metabolic limitations as a result of mutational alterations, frequently respond to this type of stress by synthesizing the extracellular polysaccharide colanic acid. Deletion mutation of the gmd gene precludes colanic acid synthesis that could enable bacterial cell to survive, while undergoing death by lysis due to the presence of mutations in its essential genes. The mutation would block the conversion of GDP-mannose to GDP-fucose. GDP-fucose is necessary for the synthesis of colanic acid since fucose represents one-third of the sugar mass of colanic acid.

In a further embodiment, the *relA* gene is mutated. Bacterial strains that undergo lysis as a result of their inability to synthesize the rigid layer of the bacterial cell wall may cease to lyse if protein synthesis is arrested. To uncouple this interdependence for continued protein synthesis for lysis to occur, the *relA* gene was inactivated by mutation. Although protein synthesis within bacteria *in vivo* is not likely to impair genetic vaccine vector delivery by lysis, the bacterial strain might encounter some environments, following excretion in feces, for example, in which protein synthesis might be inhibited. In this instance, bacterial cells for genetic vaccine or antigen delivery might survive. For example, Figure 4 x depicts a Delta*relA1123* mutation that uncouples lysis from protein synthesis and ensures ultimate lysis and death of the DNA vaccine vector delivery host strain.

In another embodiment, the *msbB* gene is mutated. The lysis of gram-negative bacteria *in vivo* leads to the release of lipopolysaccharide (LPS) that contains endotoxin due to the presence of lipid A. Although lipid A serves as an adjuvant to stimulate the innate immune system to produce interferon-gamma, which enhances the induction of a desired cellular immune response, this inflammatory response can also induce fever and have an adverse consequence on an immunized animal or human host. There are numerous mutations that can alter the myristillation of lipid A with alterations of attached fatty acids to alter the degree of toxicity (Raetz and Whitfield, Annu. Rev. Biochem. 71:635-700 (2002)). Mutation of the *msbB* gene decreases LPS toxicity without altering the virulence of the bacterial strain possessing this mutation. Figure 4 u diagrams the Delta*msbB48* mutation.

In another embodiment, it is contemplated that the host-vector system may be modified to encode a SipB protein. SipB has homology to the *Shigella flexneri* IpaB protein (Hume *et al. ,* Mol. Microbiol. 49:425-439 (2003)) that is responsible for enabling *Shigella* to rapidly exit the endosome and replicate freely in the cytoplasm. Therefore, since SipB can partially substitute for IpaB in *Shigella,* an increased amount of SipB production by *Salmonella* may permit endosome escape to enhance induction of a CD8-dependent CTL response. It is further contemplated, that the *sifA* gene may be inactivated to facilitate *Salmonella's* exit from the endosome. The *sifA* gene product is required to maintain *Salmonella* cells within the endosome, and inactivation of this gene enables *Salmonella* cells to escape the endosome and multiply in the cytoplasm (Brummel *et al.,* Infect. Immun. 70:3264-3270 (2002); Beuzon *et al.,* Microbiology 148:2705-2715 (2002)). Because the bacteria entering the cytoplasm are often killed, preferably, the promoter for the *sifA* gene is deleted and replaced with the *araC* P_{BAD} activator-promoter so that non-expression of the *sifA* gene is delayed until after the DNA vaccine delivery host-vector has undergone some 5 to 10 generations of growth and adequately colonized lymphoid tissues. The DeltaP_{sifA196}::TT *araC* P_{BAD} *sifA* deletion-insertion construction diagrammed in Figure 4 can be introduced into strains to achieve these desired results. In this case, DNA vaccine vectors will be delivered more efficiently into the cytoplasm with enhanced antigen synthesis leading to increased MHC class I presentation and a resulting superior cellular immune response, especially a CTL response.

The host-vector system has multiple advantageous applications. In one embodiment, the host-vector system further comprises a polynucleotide encoding a desired gene product. The polynucleotide may encode an auto-antigen, such as a sperm-specific or egg-specific antigen to induce an immunity that may confer immunocontraception or infertility. The antigens may represent protective antigens encoded by genes from a pathogen that render the pathogen virulent. Other antigens may be tumor antigens that can be used to develop a vaccine against cancer. Depending on the type of immunity desired, any person of ordinary skill in the art would know the appropriate antigen to select to develop a vaccine or vaccination regimen that would stimulate that type of immunity. Antigens can be specified by polynucleotides cloned from pathogens or as cDNA clones from animals, including humans. The polynucleotides can be synthesized with codons to optimize expression either in bacteria or in vertebrate animals, including humans. In developing a vaccine against HBV, for example, the protective pre S1 and pre S2 epitopes can be expressed as fusions to the HBV core, which self-assembles into a particle in bacteria (Schodel et al., Infect. Immun. 62:1669-1676 (1994)). These particles are highly immunogenic and can be delivered to the immunized individual by regulated delayed lysis of the bacterial host-vector system. Because the expression of the core genes for the woodchuck and duck hepatitis viruses (that are closely related to HBV) occurs in bacteria and the synthesized core proteins self-assemble into core particles, it would be feasible to use core particles from these viruses for presentation of B-cell and T-cell epitopes representing protective antigens from various bacterial, viral, fungal, and parasite pathogens.

The host-vector system displaying regulated delayed lysis can be used to deliver to an immunized individual a bolus of antigen synthesized in the host-vector strain prior to its lysis *in vivo.* Such antigen synthesis can be controlled using a regulatable promoter such as P_{trc}. P_{trc} is regulated by the production of a LacI repressor which is encoded by an *araC* P_{BAD} *lacI* that exists either in the chromosome or on a low copy number plasmid in the host-vector strain. Growth of such a strain in the presence of arabinose causes synthesis of the LacI repressor to repress antigen synthesis controlled by P_{trc}. However, LacI synthesis ceases following immunization since arabinose is not present. The amount of LacI is diluted out with each cell division of the host-vector strain in the immunized animal or individual. This leads to a gradual de-repression of P_{trc}-controlled antigen synthesis. Because the plasmid copy number encoding essential genes for cell wall synthesis outnumbers the copy number encoding the *araC* P_{BAD} *lacI,* synthesis of antigen encoded by the gene regulated by P_{trc} commences several generations (*i. e.,* cell divisions) prior to the onset of cell lysis. Enhancement of the immunogenicity of the antigen synthesized by the host-vector system can be achieved by partial exportation of the antigen out of the host-vector cells, either as soluble antigen or contained in membrane vesicles. Export of antigens outside of host-vector cells can be accomplished by fusing the sequence encoding the antigen to a sequence encoding the beta-lactamase signal sequence and additional amino acids to facilitate secretion (Kang *et. al.,* Infect. Immun. 70:1739-1749 (2002)). This type of construction has been accomplished for the alpha-helical portion of the *S*. *pneumoniae* PspA antigen encoded by the original sequence from *S. pneumoniae* strain RX1 and a codon sequence with codons optimized for high-level expression in *Salmonella*, *Shigella* or *E. coli.*

In an embodiment, the host-vector strain delivers the antigen(s) by utilizing the Type III secretion system, which allows delivery of T-cell epitopes into the cytoplasm of cells within the immunized animal or individual. This type of antigen delivery results in MHC class I presentation and induction of a Th1-dependent CTL immune response. To achieve this result, an additional plasmid vector system is used. *Salmonella* strains with the *alr* and *dadB* mutations require D-alanine for growth. D-alanine is an essential constituent of the rigid layer of the bacterial cell wall and is not present in animals, including humans. A wild-type *alr*^{*+*} gene in a plasmid vector enables selective maintenance of the plasmid in a bacterial strain in an immunized animal that has mutations in the *alr* and *dadB* genes. Use of such a plasmid vector encoding a fusion between a Type III effector protein, e.g. SptP, SopE or SopE2, and an antigen sequence containing T-cell epitopes, e.g. the ESAT-6 antigen from *M*. *tuberculosis* or the LLO antigen from *L. monocytogenes,* enables induction of CTL responses to protect against infection with *M*. *tuberculosis* and *L. monocytogenes,* respectively.

Other combination vaccines are also contemplated. A fusion between codon-optimized sequences encoding the alpha-helical domains of PspA antigens from S. *pneumoniae* strains (*e.g.* RX1 and EF5668) ensure induction of immunity to the majority of *S. pneumoniae* strains of diverse serotypes (Hollingshead *et. al.,* Infect. Immun. 68:5889-5900 (2000)). This fusion can be fused to beta-lactamase secretion sequence and placed under the P_{trc} control in the regulated delayed lysis vectors, such as pYA3681 or pYA3682. PspA antigens is an adhesin necessary for *S*. *pneumoniae* colonization. Psa A is specified as a fusion to the beta-lactamase signal sequence with the sequence inserted in an Alr⁺ vector. The strain also possesses mutations in the *alr* and *dadB* genes to impose a requirement for D-alanine. The *psaA* sequence could be PCR amplified from *S. pneumoniae* TIGR-4. As a result, a vaccine displaying regulated delayed cell lysis can be engineered to synthesize two protective pneumococcal antigens to stimulate immunity that could both block colonization and systemic infection by diverse *S*. *pneumoniae* serotypes. The antigens would initially be secreted or released in membrane vesicles during cell divisions *in vivo* after immunization and then released as a bolus of antigens when the cells commence to lyse.

Other potential protective antigens are from viruses, such as HIV, influenza, and papilloma. HIV antigenic proteins that could be expressed in a DNA vaccine vector to be delivered to an immunized host by the host-vector system with regulatable lysis *in vivo* as described comprises gp120, gp140, gp160 (Env), Nef, Pol, Tat, Rev and Gag/Pol. These antigens or peptide epitopes with B-cell and/or T-cell stimulatory properties derived therefrom could also be presented by HBV core particles to be released from a host-vector system having the regulatable lysis *in vivo* phenotype as described to stimulate an immune response in a vaccinated individual to these HIV epitopes. Thus, it is contemplated that the papillomavirus gene for the L1 core protein, which assemble into virus-like particles (VLPs) in the cytoplasm of *Salmonella* vaccine strains, can be expressed. (Benyacoub et al., Infect. Immun. 67:3674-3679 (1999)). These VLPs could be released by a host-vector system with regulated lysis occurring *in vivo.* The delivery system may be further modified to deliver a protective T-cell epitope encoded by the papillomavirus E7 gene (Nonn et al., J. Cancer Res. Clin. Oncol. 2:381-389 (2003). This can be accomplished with an Alr⁺ plasmid vector designed to deliver the N-terminal portion of the SptP, SopE or SopE2 proteins, which can be delivered by the TTSS, a protein encoded by genes in *Salmonella* Pathogenicity Island 1 (SPI-1). Such a vaccine should be useful to prevent papilloma-induced cervical cancer.

The host-vector system with regulated delayed lysis can have additional uses in addition to those described. Vaccines against many viral diseases of animals, including humans, make use of live attenuated viruses. These viruses must each be propagated in specific cell lines or animal hosts, including embryonated eggs. A producer of such vaccines must maintain all these diverse cell lines and propagation systems. This can result in considerable expenses because cell line storage and cell culture media are expensive. The complexities and costs increase further when one also considers that many of these live viral vaccines use attenuated virus derivatives that protect against a particular strain of a given virus that are most prevalent in a portion of the world. This is particularly true in the poultry industry. Thus, the propagation and delivery of attenuated viral genomes in a bacterial host-vector system of the present invention would be at a considerable savings in reduced vaccine production and delivery costs. Such bacterial strains can be stored in -70°C freezers, grown in inexpensive media and preserved by lyophilization.

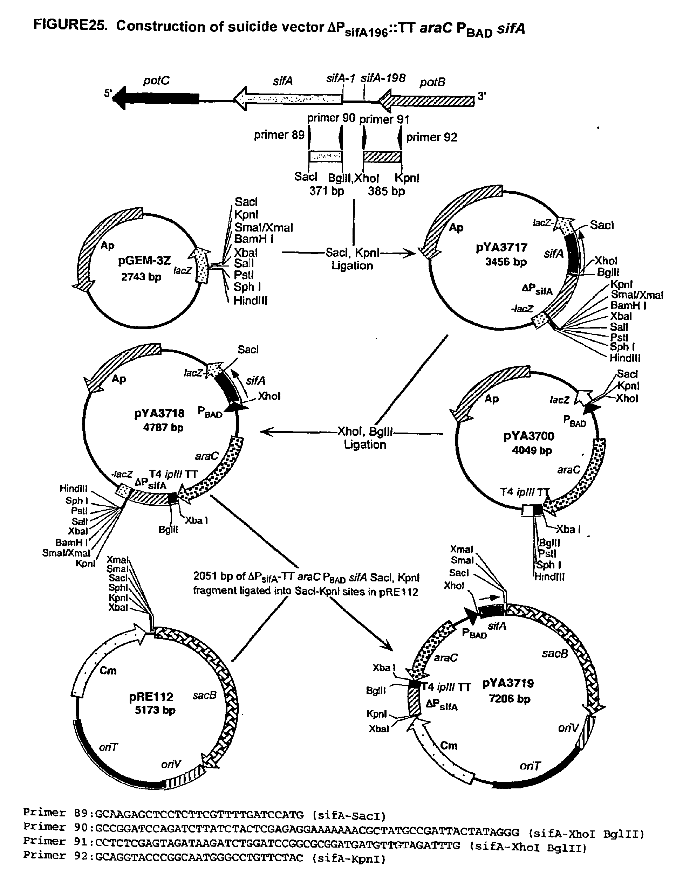

Artificial bacterial chromosomes (BACs) generated from mini-F plasmids have been very useful for cloning (and sequencing) large segments of DNA (up to 600 kb) from various sources into strains of *E. coli* (Shizuya *et al*., Proc. Natl. Acad. Sci. USA 89:8794-8797 (1992)). BACs have been used to clone the entire genome of a herpesvirus and transfected into eukaryotic cells to cause a productive infection with release of infectious virus particles (Messerle *et al*., Proc. Natl. Acad. Sci. USA 94:14759-14763 (1997)). More recently, Tischer *et al.* (J. Gen. Virol. 83:2367-2376 (2002)) have generated a DNA vaccine based on the cloning of the entire genome of an attenuated Marek's disease virus vaccine into a BACs vector and have been able to deliver this recombinant viral DNA vaccine to chickens. Some chickens (depending on the delivery means, dose and timing of immunization) developed immunity and did not develop tumors after challenge with a virulent Marek's disease virus strain. A much more cost-effective and efficacious means to deliver such a recombinant BAC to chickens is to introduce the recombinant BAC into a *S*. *typhimurium* host-vector system with regulated delayed lysis, and Chi8888 is further modified to display a regulated delayed means to escape the endosome to release the recombinant BACs into the cytoplasm of cells within the immunized chickens. The Delta P_{sifA196}::TTaraCP_{BAD}sifA mutation in Chi8925 is introduced into Chi8888 or its derivative using the suicide vector pYA3719 (Figure 25) to accomplish this regulated escape from the endosome.

Currently available BAC vectors are derived from the mini-F plasmid, which possesses IncF genes that are incompatible for maintenance of this plasmid in a strain that possesses another plasmid with the same or closely similar incompatibility genes. The S. *typhimurium* virulence plasmid is a stable low-copy number plasmid that is also IncF (Tinge and Curtiss, J. Bacteriol. 72:5266-5277 (1990)) and is, therefore, incompatible with the currently available IncF BAC vectors. To circumvent this problem, the genes for the IncF in the BAC vector can be replaced with IncI-alpha (Nozue et al., Plasmid 19:46-56 (1988)). This generates an IncI-alpha BAC vector with a low copy number replicon that will be compatible with the *S. typhimurium* virulence plasmid. This IncI-alpha BAC vector with the attenuated Marek's virus genome could then be efficiently and cost effectively delivered to day-of-hatch chicks using the bacterial host-vector system of the present invention. The vaccine may be administered by course spray. (Sandra Kelly, WO 00/04920 or PCT/US99/15843) DNA copies of RNA virus genomes can be used in the present invention.

Another use of a modified IncI-alpha BAC would be to specify synthesis of a large number of protective antigens from one or more pathogens. One could employ such a system as a means to screen for genes encoding protective antigens from uncharacterized pathogens.

The antigens may also derive from *Plasmodium* species that cause malaria, *Eimeria* species that cause coccidiosis, *Shistosoma* species that cause shistosomiasis, and *Trypanosome* species that cause a variety of diseases. These antigens can be encoded by a DNA vaccine vector to be expressed by the immunized host to induce systemic and cellular immunities or epitopes derived from these antigens can be delivered either as B-cell epitopes by fusion to HBV cores to stimulate antibody production or as T-cell epitopes by fusion to effector proteins such as SptP, SopE or SopE2 to be delivered by Type III secretion to stimulate cellular immunity.

In an embodiment, the host-vector system is used in a vaccine. The vaccine may be administered to a eukaryotic host, such as a vertebrate. The vaccine may be administered via oral, rectal, intravaginal and intrapenile routes of administration. Still further, the vaccine can be administered by intranasal inoculation or by injection intradermally, intramuscular, subcutaneously, intraveinously and intraperitonealy. The vaccine is generally suspended in a suitable excipient and is administered at appropriate doses. Optimal doses are determined by clinical trials. The vertebrate may be, for example, a mouse, rat, bird, or human.

The host-vector system can be applied to develop vaccines to protect against a diversity of pathogens, which may be bacterial, viral, fungal, or parasitic. In an embodiment, the host-vector system is applied to immunize a eukaryotic host against coccidiosis. Here, the eukaryotic host is typically a poultry species, such as a chicken. *Eimeria* species cause coccidiosis in poultry, a bloody diarrhea with severe negative economic consequences for the poultry industry. The industry spends some $500,000,000 annually for anti-coccidial drugs. No effective safe vaccine presently exists. Since coccidiosis is a parasitic disease with a one-week life cycle with oocysts that germinate into sporozoites that differentiate into merozoites, it is important to induce cellular immunity to both the sporozoite and merozoite stages. Since *Eimeria* is an intracellular pathogen, induction of a CD8⁺ dependant response generating CTLs would be most protective. This can be achieved by DNA vaccines since protein antigens are synthesized in the cytoplasm of host cells, which leads to MHC class I presentation that results in inducing a CTL response. The delivery of a DNA vaccine would stimulate other branches of the immune system to result in production of mucosal and systemic antibody production and also stimulate CD4⁺ T cells. The nucleotide and amino acid sequences of many *Eimeria* antigens are known and can be inserted in the host-vector system for delivery of the DNA sequences encoding these antigens to the poultry to elicit an immune response for protection against coccidiosis.

All references cited herein are hereby incorporated by reference in their entirety.

**EXAMPLES**

The following Materials and Methods were used, where appropriate, throughout the examples described below.

a. Bacterial strains, media and bacterial growth. Vaccine strains for testing in mice and chickens were derived from *S. typhimurium* UK-1, which has the highest virulence for chickens and mice of any *S. typhimurium* strains we have so far tested (Curtiss, New Generation Vaccines, p. 715-740 (1990)). Table 1 lists the bacterial strains along with strains of *S. paratyphi* A and S. *typhi* to develop DNA vaccine vector and/or antigen delivery vaccines for humans. Table 2 lists the plasmids. Figure 4 depicts the defined deletion mutations with and without specific insertions that have been introduced into any of the constructed strains and which could be introduced into any other strain using suicide vectors or by the method using suicide vectors and transduction described in Example 1. Other mutations have been created using transposon mutagenesis and are available to construct strains. If a particular transposon-induced mutation proves to be significant, then a defined deletion mutation can be generated so it can be introduced into strains by allele replacement using a suicide vector in the absence of any antibiotic resistance genes. LB broth and agar (Luria and Burrous, J. Bacteriol. 74:461-476 (1957)) were used as complex media for propagation and plating of bacteria. Nutrient broth (Difco), which is devoid of arabinose, minimal salts medium, and agar (Curtiss, J. Bacteriol. 89:28-40 (1965)) were used. Some studies are done with bacterial strains grown in tissue culture medium to simulate environments to be encountered *in vivo.* MacConkey agar with 0.5% lactose (Lac) or arabinose (Ara) was used to enumerate bacteria from mice and chickens. Bacterial growth was monitored spectrophotometrically.

**TABLE 1. Bacterial Strains**

| **χ number** | **Genotype** | **Description** |
|---|---|---|
| ***Escherichia coli* K-12** | | |
| χ289 | *F supE42 λ*^{*-*} T3^{R} | Acridine orange cured F derivative of χ15 |
| χ1794 | Rts1, T6^{R} λ⁻ *his-15 lysA-4*^{*-*} T3^{R} *xyl-2*^{*-*} *arg-32*^{*-*} | Rtsl plasmid, Km^{r} Str^{s} |
| χ6212 | f80d *lacZ ΔM15 deoR Δ(lacZYA-argF)U169 supE44 λ gyrA96 recA1 relA1 endA1 ΔasdA4 Δzhf-2::*Tn*10 hsdR17* (R⁻ M⁺) | Rec⁻(UV⁵) Asd⁻ Lac⁻ Nal^{r} Tet^{s}, This is a *Δasd* derivative of DH5α providing a *recA hsd* background for the Asd⁺ vectors. It was obtained by transforming χ6101 with pYA2000 (a *recA* clone) transducing this intermediate to Tet^{r} Dap⁻ using P1L4 grown on χ2981 then plating on fusaric acid medium containing DAP to select for Tet isolate. This isolate no longer requires thiamine and appears to grow slower than either parent. (pYA2000 is not present in strain due to selection for Ap^{s} isolate). |
| χ7213 (MGN-617) | *thi-1 thr-1 supE44 tonA21 lacY1 recA RP4-2-Tc:: Mu λpir ΔasdA4* Δ*zhf-2::*Tn*10* | Kan^{r} Tet^{s} Amp^{s}, DAP Universal donor strain for conjugating pir-dependent suicide vectors |
| χ7232 (MGN-026) | *endA hisR17(rk,mk*^{*+*}*) thi-1 supE44 tonA21 lacY1 recA1 gyrA relA1 Δ(lacZYA-argF)U169 lpir deoR* (f80d *lacZ ΔM15)* | Nal^{r} UV^{s} Thi⁻, Lac⁻ DH5αlysogenized with *λpir.* This strain provides the *lacZ* complementation needed for use with the pUC based *lacZα* MCS of DH5α, with the ability to support *pir* dependent replicons. |
| DH5α | f80d *lacZ ΔM15 recA1 endA1 hisR17(rk*^{*-*}*,mk*^{*+*}*) Δ(lacZYA-argF)U169 supE44 thi-1* λ *gyrA relA1* F⁻ | This is a recombination deficient suppressing strain which is readily transformable. The f80 *lacZ* permits a-comple-mentation of the amino terminus of b-galactosidase encoded by pUC vectors. |
| ***Escherichia coli*** | | |
| χ2927 | | EPEC strain: serotype O127::K63 Strain E2348, noninvasive, nontoxigenic |
| χ6206 | | EPEC strain: serotype O26::H11 Strain H3D-produces SLT-1 |
| χ7116 | | UTEC strain: serotype O1:K1:H7 |
| ***Salmonella typhimurium* UK-1** | | |
| χ3671 | wild-type | Splenic isolate of χ3663 from a white leghorn chicken. -Day 7-9. ATCC68169 (11/3/89) |
| χ8276 | *ΔasdA16* | Defined deletion of *asd* gene by conjugation χ3761 with pMEG-443, DAP⁻ (Met⁻, Thr⁻) Tet^{S} |
| χ9289 | *ΔasdA19 (asd-17::araCP*_{*BAD*}*c2)* | Received from MEGAN Health Inc. as MGN-795 |
| χ8290 | *ΔasdA19::TTaraCP*_{*BAD*}*c2 ΔilvG3 araC*P_{BAD}*lacI* | DAP⁻ (Met⁻, Thr⁻) Tet^{s} |
| χ8448 | *ΔaraBAD1923* | Defined deletion : 4.1kb *araBAD* gene deleted, generated by conjugating χ3761 with χ7213 (pYA3484) |
| χ8477 | *ΔaraE25* | Defined deletion : 1.4kb *araE* (complete gene) deleted, generated by conjugating χ3761 with χ7213 (pYA3485) |
| χ8516 | *ΔaraBAD1923 ΔaraE25* | Generated by conjugating χ8477(Δ*araE25*) with χ7213 (pYA3484; suicide vector construct to generate *ΔaraBAD1923).* |
| χ8573 | *ΔmsbB48* | Defined deletion of *ΔmsbB* mutant generated by conjugating χ3761 with χ7213 (pYA3529) |
| χ8581 | *ΔaraBAD1923 ΔasdA16* | Generated *ΔasdA16* defined deletion by conjugating χ8448 with MGN-1765e (pMEG443) |
| χ8583 | *ΔaraBAD1923 ΔasdA16 ΔaraE25* | Generated *ΔasdA16* defined deletion by conjugating χ8516 with MGN-1765e (pMEG443) |
| χ8619 | *ΔrelA4* | Received from MEGAN Health Inc, as MGM-4785s, AT^{s} |
| χ8623 | *ΔilvG3 araC*P_{BAD}*lacI* | Defined deletion in χ3761 with MGN930e (pMEG-249), AT^{S} |
| χ8644 | *ΔP*_{*murA7*}*::araCP*_{*BAD*}*murA* | Received from MEGAN Health Inc. as MGN-5014 |
| χ8645 | *ΔP*_{*murA7*}*::araCP*_{*BAD*}*murA* | Received from MEGAN Health Inc. as MGN-5014 Tested in mice |
| χ8653 | ΔP_{murA7}::*araC*P_{BAD}*murA ΔaraE25* | *araBAD25* defined deletion by conjugating χ8645 with χ7213 (pYA3485) |
| χ8654 | *Δ*P_{murA7}::*araC*P_{BAD}*murA ΔaraBAD1923* | *araBAD1923* defined deletion by conjugating χ8645 with χ7213 (pYA3484) |
| χ8655 | *Δ*P_{murA7}::*araC*P_{BAD}*murA ΔaraE25 ΔaraBAD1923* | *araBAD1923* defined deletion by conjugating χ8653 with χ7213 (pYA3484) |
| χ8767 | *ΔaraBAD23* | *araBAD23* defined deletion by conjugating χ3761 with χ7213 (pYA3599) |
| χ8802 | *Δ*_{murA7}::*araC*P_{BAD}*murA ΔaraBAD1923 ΔasdA16* | Transduced χ8654 with P22HT_{*int*} lysate harboring *ΔasdA16* |
| χ8804 | *Δ*_{murA7}::*araC*P_{BAD}*murA ΔaraE25 ΔasdA19*::TT*araCP*_{BAD}*c2* | Transduced χ8653 with P22HT_{*int*} lysate harboring *ΔasdA19*::TT*araC*P_{BAD}*c2* |
| χ8805 | *Δ*_{murA7}::*araCP*_{BAD}*murA ΔaraBAD1923 ΔasdA19*::TT*araC*P_{BAD}*c2* | Transduced χ8654 with P22HT_{*int*} lysate harboring *ΔasdA19*::TT*araC*P_{BAD}*c2* |
| χ8806 | *Δ*P_{murA7}::*araC*P_{BAD}*murA ΔasdA19*::TT*araC*P_{BAD}*c2* | Transduced χ8644 with P22HT_{*int*} lysate harboring *ΔasdA19*::TT*araC*P_{BAD}*c2* |
| χ8807 | *Δ*P_{murA7}::*ara*CP_{BAD}*murA ΔaraE25 ΔaraBAD1923 ΔasdA 19*::*TTara*CP_{BAD}*c2* | Transduced χ8655 with P22HT_{*int*}lysate harboring *ΔasdA19*::TT*ara*CP_{BAD}*c2* |
| χ8829 | *Δgmd-11* | Defined deletion of Δ*gmd* mutant generated by conjugating χ3761 with χ7213 (pYA3628) |
| χ8831 | *Δ*(*gmd-fcl*)*-26* | Defined deletion of *Δgmd-fcl* mutant generated by conjugating χ3761 with χ7213 (pYA3629) |
| χ8844 | *ΔendA2311* | Defined deletion of *ΔendA* mutant generated by conjugating χ3761 with χ7213 (pYA3652) |
| χ8851 | *ΔP*_{murA7}::*ara*CP_{BAD}*murA ΔaraE25 ΔendA2311 ΔasdA19::TTaraCP*_{*BAD*}*c2* | Transduced χ8804 with P22HTᵢₙₜ lysate harboring *ΔendA2311* |
| χ8853 | *ΔP*_{*murA7*}*::araCP*_{*BAD*}*murA ΔendA2311 ΔasdA19::TTaraCP*_{*BAD*}*c2* | Transduced χ8806 with P22HT_{*int*}lysate harboring Δ*endA2311* |
| χ8854 | *ΔP*_{*murA7*}*::araC*P_{BAD}*murA ΔaraE25 ΔaraBAD1923 ΔendA2311 ΔasdA 19-.:TTaraCP*_{*BAD*}*c2* | Transduced χ8807 with P22HT_{*int*}lysate harboring *ΔendA2311* |
| χ8855 | *ΔP*_{*murA7*}*::araCP*_{*BAD*}*murA ΔaraBAD1923 ΔasdA16 ΔendA2311* | Transduced χ8802 with P22HT_{*int*} lysate harboring *ΔendA2311* |
| χ8859 | ΔP_{murA7}::*araC*P_{BAD}*murA ΔaraBAD1923 ΔendA2311 ΔasdA19*::TT*araC*P_{BAD}*c2* | Transduced χ8805 with P22HT_{*int*} lysate harboring *ΔendA2311* |
| χ8882 | *ΔrelA1123* | *ΔrelA* defined deletion mutant generated by conjugating χ3761 with _{χ}7213 (pYA3679) |
| χ8883 | *ΔrelA1123 ΔP*_{murA7}::*araC*P_{BAD}*murA ΔaraE25 ΔaraBAD1923 ΔendA2311 ΔasdA 19*::TT*ara*CP_{BAD}*c2* | *ΔrelA* defined deletion mutant generated by conjugating χ8854 with _{χ}7213 (pYA3679) |
| χ8884 | *ΔmsbB48* _{*Δ*murA7}::*ara*CP_{BAD}*murA ΔaraE25 ΔaraBAD1923 ΔendA2311 ΔasdA 19*::TT*ara*CP_{BAD}*c2* | *ΔmsbB* defined deletion mutant generated by conjugating χ8854 with χ7213 (pYA3529) |
| χ8885 | *Δ*(*gmd-fcl*)*-26* ΔP_{murA7}::araCP_{BAD}murA *ΔaraE25 ΔaraBAD1923 ΔendA2311 ΔasdA19::TTaraCP*_{*BAD*}*c2* | *Δ*(*gmd-fcl*)*-26* defined deletion mutant generated by conjugating χ8854 with χ7213 (pYA3629) |
| χ8887 | *ΔdadB4* | *ΔdadB* defined deletion mutant generated by conjugating χ3761 with χ7213 (pYA3668) |
| χ8888 | *ΔrelA1123 Δ*(*gmd-fcl*)*-26* ΔP_{murA7}::*araC*P_{BAD} *murA ΔaraE25 ΔaraBAD1923 ΔendA2311 ΔasdA19::*TTaraCP_{BAD}*c2* | Transduced χ8885 with P22HT_{*int*} lysate harboring Δ*relA1123* (χ8882) |
| χ8898 | *Δalr-3* | *Δalr* defined deletion mutant generated by conjugating χ3761 with χ7213 (pYA3667) |
| χ8899 | *ΔdadB4 Δalr-3* | *Δalr* defined deletion mutant generated by conjugating χ8887 with χ7213 (pYA3667) |
| χ8925 | ΔP_{sifA196}::TT*araC*P_{BAD} sifA | ΔP_{sifA}::TT *araC* P_{BAD} *sifA* deletion-insertion mutant generated by conjugating χ3761 with χ7213 (pYA3719) |
| χ8926 | *ΔsifA26* | *ΔsifA* defined deletion mutant generated by conjugating χ3761 with χ7213 (pYA3716) |
| χ8933 | *atrB13:: MudJΔrel.41123 Δ(gmd-fcl)-26* ΔP_{murA7}::araCP_{BAD}murA *ΔaraE25 ΔaraBAD1923 ΔendA2311 ΔasdA19::TTaraCP*_{*BAD*}*c2* | Transduced χ8888 with P22HTᵢₙₜ lysate harboring *atrB13:*:Mud*J* (χ4574) |
| ***Salmonella typhimurium* SL1344** | | |
| χ8600 | *ΔfliC825* | Defined deletion of *fliC* with a χ7213 (pYA3547), high-motility |
| χ8601 | *ΔfljB217* | Defined deletion of *fljB* with a χ7213 (pYA3548), low-motility |
| χ8602 | *ΔfliC825 ΔfljB217* | Defined deletion of *fljB* on χ8600 with a pYA3547, non-motile |
| ***Salmonella paratyphi A* and *Salmonella typhi*** | | |
| χ3744 | wild type *S. typhi* ISP1820 | ATCC# 55116, clinical isolate from a chilean patient Trp⁻ Cys⁻ |
| χ3769 | wild type *S. typhi* Ty2 | ATCC19430, Cys⁻ RpoS⁻ |
| χ8387 | *S*. *paratyphi A* | Derived from ATCC 9281 (χ8246) by curing of a low molecular weight ColE1-like plasmid. Kan^{S} |
| χ8438 | *S.typhi* Ty2 | ATCC# 202182, RpoS⁺ mutant of wild type χ3769 |
| ***Listeria*** | | |
| | *Listeria monocytogenes 5647B* | Serovar 4b, blood culture, 64 yr male |
| | *Listeria monocytogenes 2004E* | Serovar 4b, raw lamb |
| | *Listeria monocytogenes 1441B* | Serovar 4b, blood culture, 74 yr male |
| | *Listeria monocytogenes L4667* | Serovar 1/2b, blood culture, 52 yr male |
| | *Listeria monocytogenes L4951* | Serovar 1/2b, blood culture, 54 yr male |
| | *Listeria monocytogenes L5073* | Serovar 1/2c, blood culture, 37 yr male |
| | *Listeria monocytogenes 5647B* | Serovar 4b, blood culture, 64 yr male |
| ***Shigella*** | | |
| | *Shigella flexneri* 2a | Wild-type 2457T, Pcr⁺ Mal⁻ □^{r} |
| | *Shigella flexneri* 2a | BS98; spontaneous avirulent nonpigmented mutant derivative of wild-type 2457T, *pcr-11* |
| | *Shigella flexneri* 2a | BS109; rough phenotype |
| ***Yersinia*** | | |
| | *Yersinia pestis* KIM | |
| | *Yersinia pestis* KIM6⁺ | |
| | *Yersinia pseudotuberculosis* YPIII | Serotype III, standard lab strain |
| | *Yersinia pseudotuberculosis* 730317 | Serotype I, isolated from goat |
| | *Yersinia pseudotuberculosis* 722080 | Serotype IB, isolated from human blood |
| | *Yersinia pseudotuberculosis* 713425 | Serotype IA, isolated from monkey |
| | *Yersinia pseudotuberculosis* 77C0315 | Serotype III, isolated from domestic cat |
| | *Yersinia pseudotuberculosis* 79EA84 | Serotype III, isolated from a 57 year old human male with a shoulder wound and diarrhea |
| | *Yersinia enterocolitica* 8081V | Serotype O:8, a clinical isolate from humans |
| | *Yersinia enterocolitica* 637-83 | Serotype 05,27, a CDC reference strain |
| | *Yersinia enterocolitica* 9286-78 | Serotype 020, a human isolate |
| | *Yersinia enterocolitica* 655-83 | Serotype 018, a CDC reference strain |
| | *Yersinia enterocolitica* 634-83 | Serotype 04,32, a CDC reference strain |
| | *Yersinia enterocolitica* 642-83 | Serotype 09, isolated from a human colitis with perforation |
| | *Yersinia enterocolitica* 2455-87 | Serotype O1,2,3, isolated from human blood |
| | *Yersinia enterocolitica* 9291-78 | Serotype 06, non-pathogenic serotype, a companion to the 9286-78 |
| | *Yersinia pseudotuberculosis* YPIII | Serotype III, standard lab strain |
| | *Yersinia pseudotuberculosis* 730317 | Serotype I, isolated from goat |
| | *Yersinia pseudotuberculosis* 722080 | Serotype IB, isolated from human blood |
| ***S. pneumoniae*** | | |
| | TIGR-4 | Capsular type 4 |
| | WU2 | wild-type virulent, encapsulated type 3, PspA type 1 |
| | EF5668 | Capsular type 4, PspA type 12 |
| | Rx1 | Nonencapsulated avirulent, highly transformable variant of D39, PspA type 25 |
| ***Mycobacterium*** | | |
| | *M. tuberculosis* H37Rv | Virulent laboratory strain |
| | *M. tuberculosis* H37R_{A} | Avirulent laboratory strain |

**TABLE 2. Plasmids Need to add all new plasmids for all new constructions**

| pYA number | Description | Vector | Host strain | Marker |
|---|---|---|---|---|
| *Suicide Vector* | | | | |
| pDMS 197 | *oriV oriT sacB* | | | Tc |
| PMEG-149 | *R6K ori mob incP sacB sacR* | | | Ap |
| pMEG-249 | *ΔilvG3::araCP*_{*BAD*}/*acI* | pMEG-149 | χ7213 | Ap |
| pMEG-375 | *R6K ori mob incP sacB sacR* | | χ7232 | Cm |
| pMEG-443 | *ΔasaA16* | pMEG-375 | χ7213 | Cm, DAP |
| pMEG-611 | *ΔasdA19::*TT*araC*P_{BAD}*c2* | pMEG-375 | χ7213 | Cm, DAP |
| pMEG-902 | ΔP_{murA}7::*ara*CP_{BAD}*murA* | PRE112 | χ7213 | Cm, DAP |
| pYA3484 | *ΔaraBAD1923* | pMEG-375 | χ7213 | Cm, DAP |
| pYA3485 | *ΔaraE25* | pMEG-375 | χ7213 | Cm, DAP |
| pYA3529 | *ΔmsbB48* | pMEG-375 | χ7213 | Cm, DAP |
| pYA3547 | *ΔfliC825* | pMEG-375 | χ7213 | Cm, DAP |
| pYA3548 | *ΔfljB217* | pDMS197 | χ7213 | Tc, DAP |
| pYA3599 | *ΔaraBAD23* | pMEG-375 | χ7213 | Cm, DAP |
| pYA3628 | *Δgmd-11* | pMEG-375 | χ7213 | Cm, DAP |
| pYA3629 | *Δ(gmd-fcl)-26* | pMEG-375 | χ7213 | Cm, DAP |
| pYA3652 | *ΔendA2311* | pMEG-375 | χ7213 | Cm, DAP |
| pYA3667 | *Δalr-3* | pMEG-375 | χ7213 | Cm, DAP |
| pYA3668 | *ΔdadB4* | pMEG-375 | χ7213 | Cm, DAP |
| pYA3679 | *ΔrelA1123* | pMEG-375 | χ7213 | Cm, DAP |
| pYA3716 | ΔsifA26 | pRE112 | χ7213 | Cm, DAP |
| pYA3719 | ΔP_{sifA196}::TT *araC*P_{BAD}*sifA* | pRE112 | x7213 | Cm, DAP |
| *Vector* | | | | |
| pMEG-104 | P22P_{R}*lys13 lys19 asd* P22P_{L} | | MGN-336 | DAP |
| pMEG-242 | *araC* P_{BAD} *lacI* | | χ6212 | DAP |
| pYA3167 | pBR *ori* P_{trc} HBV *asd* | | | |
| pYA3341 | pUC *ori* P_{trc} *asd* | | χ6212 | |
| pYA3342 | pBR *ori P*_{*trc*} *asd* | | χ6212 | |
| pYA3488 | *p15A ori araC*P_{BAD} *c2* SD-ATG *asd* | | χ6212 | L-arabinose |
| pYA3450 | *p15A ori araC*P_{BAD} SD-ATG *asd* | | χ6212 | L-arabinose |
| pYA3530 | *p15A ori* ara*C*P_{BAD} SD-GTG *asd* | | χ6212 | L-arabinose |
| pYA3531 | *p15A ori araC*P_{BAD}*c2* SD-GTG *asd* | | *χ*6212 | L-arabinose |
| pYA3565 | *p15A ori araC*P_{BAD} SD-TTG *asd* | | χ6212 | L-arabinose |
| pYA3566 | *p15A ori araC*P_{BAD}*c2* SD-TTG *asd* | | χ6212 | L-arabinose |
| pYA3542 | 6 x His tag-Asd | pKK233-2 | | Ap |
| pYA3587 | pVAX-1/rrfGTT | | DH5α | Kan |
| pYA3607 | *p15A ori* Asd- P22P_{R} *anti-sense RNA* | | χ6212 | L-arabinose |
| pYA3608 | *p15A ori araC*P_{BAD} c2 SD-GTG *asd* P22P_{R} *anti-sense RNA* | | χ6212 | L-arabinose |
| pYA3609 | *p15A ori araC*P_{BAD}SD-GTG *asd* P22P_{R} *antisense RNA* | | χ6212 | L-arabinose |
| pYA3610 | *p15A ori araC*P_{BAD}*c2* SD-ATG *murA* SD-GTG *asd* P22P_{R} *antisense RNA* | | χ6212 | L-arabinose |
| pYA3611 | pUC *ori* P_{CMV}*araC* P_{BAD} c2 SD-GTG *asd* P22P_{R} *antisense RNA* | | χ6212 | L-arabinose |
| pYA3613 | *p15A ori araC** P_{BAD} SD-ATG *murA* SD-GTG *asd* P22P_{R} *antisense RNA* | | χ6212 | L-arabinose |
| pYA3614 | pUC *ori* P_{CMV}*araC**P_{BAD} SD-GTG *asd* P22P_{R} *antisense RNA* | | χ6212 | L-arabinose |
| pYA3615 | pUC *ori* P_{CMV}*araC** P_{BAD} SD-ATG *murA* SD-GTGasd P22P_{R} *antisense RNA* | | χ6212 | L-arabinose |
| pYA3624 | *p15A ori araC** P_{BAD} SD-GTG *asd* P22P_{R} *antisense RNA* | | χ6212 | L-arabinose |
| pYA3493 | pYA3342 derivative β-lactamase signal sequence-based periplasmic secretion plasmid | pYA3342 | χ6212 | |
| pYA3494 | 0.7 kb DNA encoding the α-helical region of PspA in pYA3493 | pYA3493 | χ6212 | |
| pYA3634 | Replacing pYA3494 in which base G is deleted at mature amino acid 234. | pYA3493 | χ6212 | |
| pYA3635 | pYA3494 derivative harboring codon optimized *pspA*_{*Rx1*} sequence | pYA3494 | χ6212 | |
| pYA3642 | *p15A ori araC** P_{BAD} SD-ATG *asd* P22P_{R} *antisense RNA* | | χ6212 | L-arabinose |
| pYA3643 | *p15A ori araC** P_{BAD} SD-ATG *murA* SD-ATG *asd* P22P_{R} *antisense RNA* | | χ6212 | L-arabinose |
| pYA3644 | pUC18/SD-ATG *murA* | | DH5α | |
| pYA3645 | pUC18/SD-GTG *murA* | | DH5α | L-arabinose |
| pYA3646 | *p15A ori araC** P_{BAD} SD-GTG *murA* SD-GTG *asd* P22P_{R} *antisense RNA* | | χ6212 | L-arabinose |
| pYA3647 | *p15A ori araC** P_{BAD} SD-GTG *murA* SD-ATG *asd* P22P_{R} *antisense RNA* | | χ6212 | L-arabinose |
| pYA3649 | pUC *ori* P_{CMV}*araC** P_{BAD} SD-ATG *murA* SD-ATGasd P22P_{R} *antisense RNA* | | χ6212 | L-arabinose |
| pYA3650 | pUC *ori* P_{CMV}*araC** P_{BAD} SD-GTG *murA* SD-GTGasd P22P_{R} *antisense RNA* | | χ6212 | L-arabinose |
| pYA3651 | pUC *ori* P_{CMV}*araC** P_{BAD} SD-GTG *murA* SD-ATGasd P22P_{R} *antisense RNA* | | χ6212 | L-arabinose |
| pYA3673 | pYA3650/EASZ240 | | χ6212 | L-arabinose |
| pYA3674 | pYA3650/EASZ240-FLAG | | χ6212 | L-arabinose |
| pYA3675 | pYA3651/EASZ240-FLAG | | χ6212 | L-arabinose |
| pYA3676 | pYA3650/EAMZ250 | | χ6212 | L-arabinose |
| pYA3677 | pYA3650/EAMZ250-FLAG | | χ6212 | L-arabinose |
| pYA3678 | pYA3651/EAMZ250-FLAG | | χ6212 | L-arabinose |
| pYA3680 | *p15A ori araC** P_{BAD} SD-GTG | | χ6212 | L-arabinose |
| pYA3681 | P_{trc-}P_{BR} SD-GTG*asd*, SD-GTG *murA* | | χ6212 | L-arabinose |
| pYA3682 | P_{trc-}P_{BR} SD-GTGasd, SD-GTG *murA* | | χ6212 | L-arabinose |
| pYA3712 | pYA3681/codon-optimized rPspA-Rx1 | | χ6097 | L-arabinose, Tc |
| pYA3713 | PYA3682/rPspA-Rx1 | | χ6097 | L-arabinose, Tc |
| pUC19-EASZ240(#1) | pUC-Plac-EASZ240(#1)Ap | | | Ap |
| pUC19-EAMZ250(#3) | pUC-Plac-EAMZ250(#3)Ap | | | Ap |
| pUC18 pVAX-1 | pUC-Plac-Ap pUC *ori* P_{CMV}-BGH-pA | | DH5α χ7232 | Ap Kan |

| | | | | |
|---|---|---|---|---|
| * *ara*CP_{BAD} fragment from χ289 | | | | |

b. Molecular and gene procedures. Unless indicated otherwise, standard methods for DNA isolation, restriction enzyme digestion, DNA cloning and use of PCR for construction and verification of vectors were used. (See e.g. Sambrook et al., Molecular Cloning: A Laboratory Manual, Second ed. (1989)). DNA sequence analysis was performed in the Department of Biology DNA Sequence Laboratory at Washington University. Synthesis of oligonucleotide and/or gene segment were performed by commercial entities. Phage P22HT*int* (Schmieger, Molec. Gen. Genet. 119:75-88 (1972)) was used to transduce mutations of a selectable phenotype from one S. *typhimurium* strain into other strains. Conjugational transfer of suicide vectors was performed by standard methods (Miller and Mekalanos, J. Bacteriol. 170:2575-2583 (1988)) using the suicide vector donor strain MGN-617 (also referred to as Chi7213) (Table 1). Many of the plasmids constructed possess the wild-type *asd* gene, and these recombinant constructs were introduced into Chi6212 (Table 1) by selecting for DAP-independent isolates. These constructs were sequenced and evaluated for their ability to complement various *S*. *typhimurium* mutant strains (Table 1) and for their ability to specify synthesis of Asd and MurA proteins using gel electrophoresis and western blot analysis. Chi6212, containing pYA3542 (Table 2), produced the His-tagged Asd, which was used to obtain anti-Asd rabbit antisera for western blot analyses. Table 3 lists the oligonucleotide primers used for constructions of the plasmid, for PCR reactions to confirm presence of mutations/insertions, and for DNA sequence analysis.

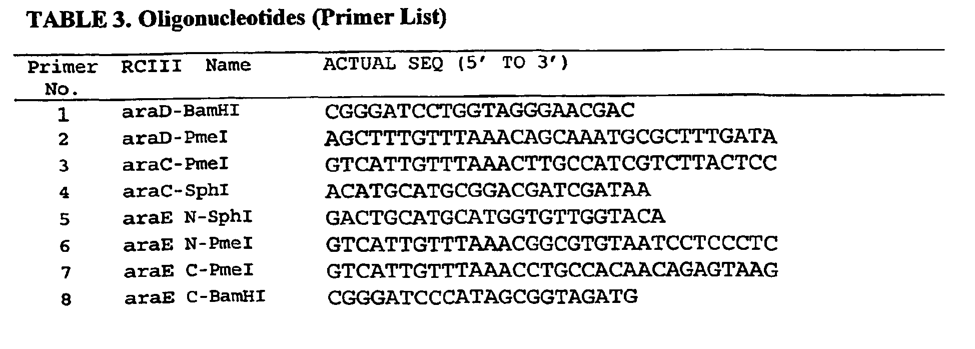

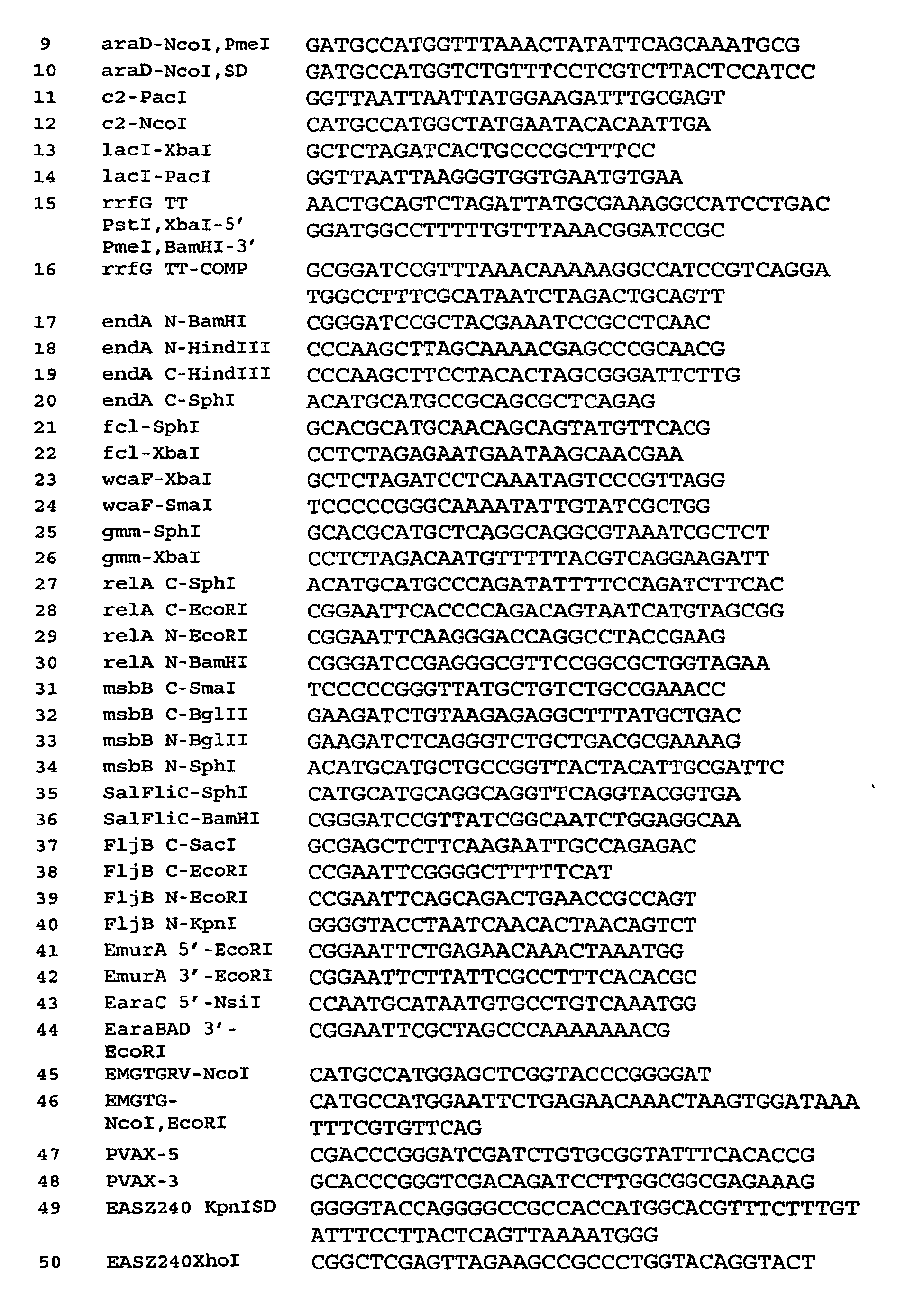

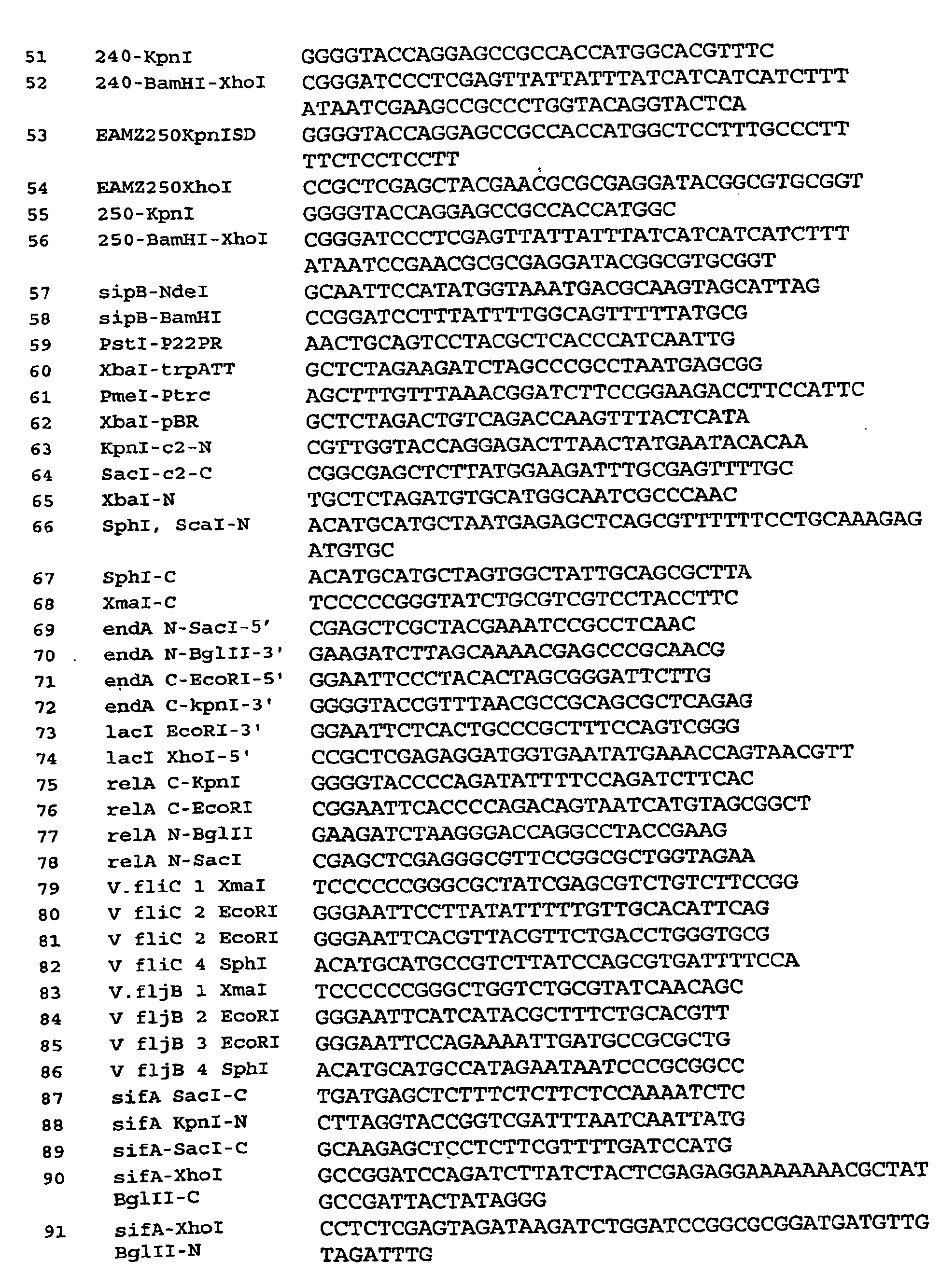

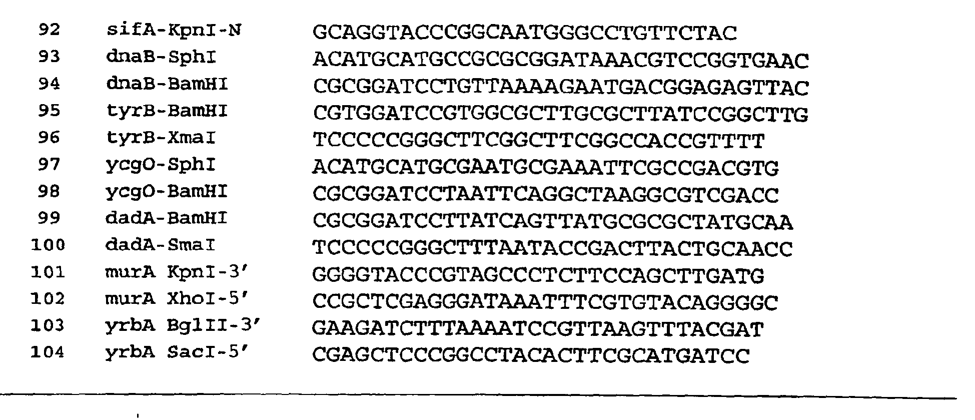

c. Strain characterization. Experimental vaccine strains were fully characterized prior to use for immunization studies. Those strains were compared with vector control strains for stability of plasmid maintenance when strains are grown in the presence of DAP over a 50 generation period. Molecular gene attributes were confirmed by use of PCR and/or Southern blot analyses with appropriate probes. Measurement of lipopolysaccharide or its absence was performed after electrophoresis using silver stained gels (Hitchcock and Brown, J. Bacteriol. 154:269-277 (1983)). Motility tests and use of specific antisera for given flagellar antigens were used to reveal presence or absence of flagella. Presence of fimbrial adhesins was assayed using agglutination of yeast and red blood cells in the presence and absence of mannose as a function of growth conditions, Congo red binding assays and by transmission electron microscopy (TEM) using negative staining with phosphotungstic acid.

d. Cell biology. The ability of various constructed *Salmonella* strains to attach to, invade into and survive in various cells in culture are quantified using well established methods (Chen et al., Mol. Micriobiol. 21:1101-1115 (1996)). Murine and human macrophage cell lines and murine bone marrow derived and human peripheral blood monocyte-derived macrophages were used. To evaluate whether engineered *Salmonella* strains are capable of escaping the endosome, we fixed infected cells, created thin sections, and examined the sections by transmission electron microscopy. These methods were previously described by Miller et al. (Alpuche-Arunda, et al., Proc. Nat. Acad. Sci. USA. 85:7972-7976 (1992)) and Finlay et al. (Mol. Microbiol. 20:151-164 (1996)).

e. Animal experimentation. BALB/c and C57BL/6 female mice, six to eight weeks of age, were used for most experiments. Inbred mice with other MHC haplotypes and Swiss Webster outbred mice were also used in some studies. Mice were held in quarantine one-week before use in experiments. They were deprived of food and water six hours before per oral immunization. No bicarbonate was administered. Food and water were returned 30 min after immunization. In initial experiments comparing multiple strains, only four to five mice were included per group. A second boosting immunization is given four weeks after the first dose, but after a retro-orbital bleed to collect serum and collection of feces and/or vaginal secretions for quantitation of SIgA. In some studies, to maximize induction of antibodies, an immunization regimen of multiple (low or increasing) doses was used during an initial seven to ten day period. In other experiments, candidate vaccine strains were quantitatively enumerated in various tissues as a function of time after inoculation. Generally, three mice were used per time point. The inoculation procedures were the same as in the immunization studies. All animals were housed in BL2 containment with filter bonnet covered cages. If high immunogenicity was observed in initial tests after primary immunization, subsequent studies were done to determine the lowest level of vaccine inocula to induce a significant immune response. Important experiments have been repeated with larger groups of mice for a given dose.

One-day-old white leghorn chicks (hatched from fertile eggs from Spafas or Sunrise Farms in our animal facility) were used for experiments in St. Louis. For infection of chicks, bacterial strains were grown as standing overnights and then diluted into appropriate prewarmed media and grown with mild aeration on a rotary shaker, generally to an OD₆₀₀ of about 0.8. Cells were sedimented by centrifugation and suspended in buffered saline with gelatin (BSG) before administration to chicks orally, intranasally or by course spray. Course spray is a method used in the hatchery to immunize day-of-hatch chicks (Kelly WO 00/04920 or PCT/US99/15843). Chicks in a box are sprayed by a regulated spray nozzle suspended above the box. Birds were euthanized as a function of time after infection and the titers of bacteria recovered from various tissues, organs and organ contents quantitated by plating on suitable media. In the case of too few to count, detection was by inoculation of 100 microl tissue samples into selenite broth for enrichment, after which cultures were streaked on MacConkey agar for identification as *Salmonella.* These methods have been previously described (Curtiss and Kelly, Infect. Immun. 55:3035-3043 (1987)). Strains recovered from birds were tested to verify the maintenance of relevant attributes, including presence of plasmids. Birds were housed in either small isolators or Horsefall isolators under BSL2 containment in our animal facility and cared for by approved procedures.

Clinical trials with human volunteers are conducted as previously described (Tacket et al., Infect. Immun. 60:536-541 (1992); Nardelli-Haefliger et al., Infect. Immun. 64:5219-5224 (1996);Tacket et al., Infect. Immun. 65:3381-3385 (1997); Frey et al., Clin. Immunol. 101:32-37 (2001)).

f. Monitoring immune responses.

i. Antigen Preparation: We have purifed antigens as His-tagged proteins from recombinant *E. coli. Salmonella* LPS O-antigen was obtained commercially. We have prepared a *S. typhimurium* outer membrane protein (SOMP) fraction and a heat killed extract from a *galE* mutant derived from the wild-type *S*. *typhimurium* UK-1 strain Chi3761. This strain grown in the absence of galactose in the medium yields SOMPs uncontaminated with LPS O-antigens. These antigens were used as controls in western blots as well as for immunoassays as described below. We isolated HBV core particles with and without inserted epitopes such as the pre S1, S2 epitopes and also the HBV S antigen to monitor immune responses to these antigens. Synthetic peptides for various T-cell epitopes to use in T-cell proliferation assays were prepared commercially. In some cases, Dr. Mark Jenkins provided the cell free extracts of *Eimeria acervulina* sporozoite antigens.

ii. ELISA: Serum antibodies are measured in blood collected by retro-orbital bleeding in mice and by veinipuncture in chickens and mucosal antibodies as extracted copro antibodies from feces (chickens) or in vaginal secretions (mice). Sera and secretions were initially pooled from all mice in a group. In later studies with successful constructs, antibody titers were monitored in individual mice. We employ a doubling dilution method with the end point titer being the dilution giving an OD₄₁₀ three times that for the reagent or unimmunized animal control. SIgA titers against the various antigens were monitored by ELISA (Hassan and Curtiss, Infect. Immun. 62:2027-2036 (1994); Hassan and Curtiss, Infect. Immun. 64:938-944 (1996)) in the same way. Since we are often interested in distinguishing between a Th1 and Th2 response, the titers of IgG1 versus Ig2A are determined. These methods have been described in previous manuscripts (Dogett, et al., Infect. Immun. 61:1859-1866 (1993); Nayak et al., Infect. Immun. 66:3744-3751 (1998); Hassan, et al., Avian Dis. 37:19-26 (1993)).

iii. ELISPOT Analysis: We routinely use ELISPOT analysis (Czerkinsky, et al., J. Immunol. Methods. 65:109-121 (1983)) to determine the titers of circulating cells secreting antibodies that are antigen-specific in human trials and occasionally, in experiments with mice and chickens.

iv. T-cell Proliferation Assays: These are performed on spleenocyte preparations obtained from groups of mice or chickens at one, two and four weeks post-vaccination. Proliferative responses to the nickel column-purified His-tagged antigens are measured by a non-radioactive colorimetric assay (Promega) with which we have had much success. Stimulation with specific peptides containing T-cell epitopes are also employed. Stimulation with *S. typhimurium* outer membrane proteins and concanavalin A serve as controls.

v. CTL Assays: CTL responses to T-cell epitopes are quantitated using the appropriate murine cell lines (P815 or EL-4) transfected with plasmids (Wu and Kipps, J. Immunol. 159:6037-6043 (1997); Denis et al., Infect. Immun 66:1527-1533 (1998)) encoding the epitopes to serve as targets for assays of BALB/c or C57BL/6 immunized mice. Effector cells are obtained from spleens of non-immunized and immunized mice. Part of the vector cell population was used immediately in a Cytotox 96 non-radioactive CTL assay (Promega). The remaining cells are re-stimulated with the appropriate antigen for five to seven days prior to use in the CTL assay. The CTL assay measures lactate dehydrogenase released due to lysis of target cells. The values obtained are used to calculate the percentage of target cells that lyse relative to the quantity of effector cells added. A ⁵¹ Cr release assay can also be used (see below).

CTL responses to *Eimeria* sporozoite and merozoite antigens are quantitated using syngeneic and allogeneic primary chicken kidney target cells are infected with a Simbis virus vector expressing the relevant sporozoite or merozoite antigen. The target cells are labeled with 200-300 microCi of Na₂ ⁵¹CrO₄ for 90 min, washed three times and suspended in assay medium at a final concentration of 2 x 10⁵ cells/ml (99, 100). Effector cells are obtained from spleens of *Eimeria*-infected unimmunized chickens as well as from chickens immunized with attenuated *Salmonella* delivering a DNA vaccine. The CTL assay measures ⁵¹Cr released due to lysis of target cells and values obtained were used to calculate the percentage of target cells lysed relative to quantity of effector cells added. Mark Jenkins provided *Eimeria* oocysts for infections.

g. Statistical analysis. The results were analyzed using the appropriate statistical test from the SAS program to evaluate their relative significance.

**Example 1**

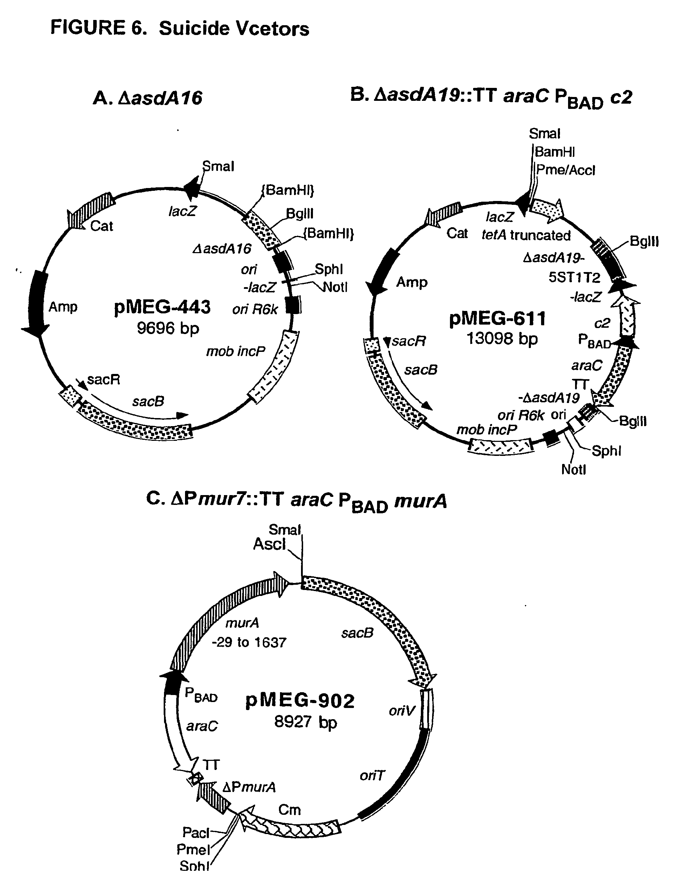

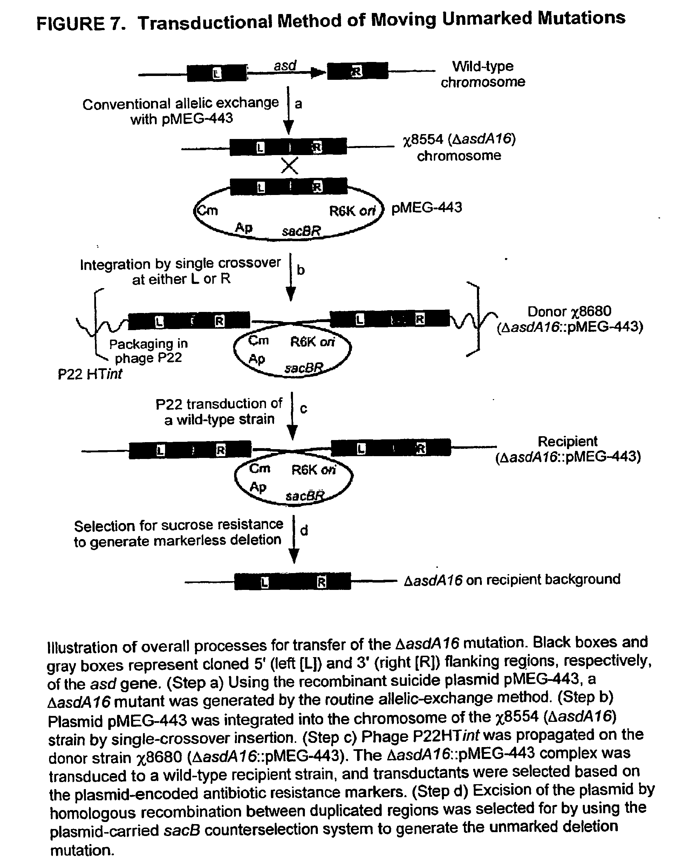

Example 1 demonstrates a method to construct bacterial strains with markerless deletion mutations. In vaccine strains for animals and humans, gene information specifying resistance to antibiotics is not preferred. Accordingly, we devised strategies for making recombinant vaccines that use a balanced-lethal host-vector system that did not rely on use of antibiotic resistance determinants (Nakayama et al., Bio/Technol. 6:155-160 (1988)). Further, we devised strategies using suicide vectors to introduce markerless deletion mutations into the chromosome of bacterial vaccine strains. This has been accomplished by first generating a deletion mutation for a specific gene or genes using PCR methodology. A fragment of DNA possessing the defined deletion is then cloned into a suicide vector. These suicide vectors generally possess genes for antibiotic resistance, such as for tetracycline, chloramphenicol and/or ampicillin, a gene that confers sensitivity to the sugar sucrose to cells that possess the suicide vector, a sequence enabling mobilization of plasmid transfer to a recipient cell due to the presence of a chromosomally integrated IncP plasmid in the donor bacterium, and the R6K *ori* that enables replication only in bacterial host cells possessing the *pir* gene inserted into the chromosome (Miller and Mekalanos, J. Bacteriol 70:2575-2583 (1988)). The suicide vector was generally maintained in the suicide vector donor strain MGN-617 that possesses the lambda *pir* and IncP conjugative plasmid in the chromosome and a Delta*asd* mutation that enables selection against it on agar medium lacking DAP. Mating of MGN-617, which possesses the suicide vector, with a suitable recipient was conducted on L agar containing DAP and an antibiotic to which the suicide vector confers resistance. Suicide vectors that integrated into the chromosome by a homologous reciprocal crossover event between sequences flanking the deleted gene on the suicide vector and homologous sequences on the bacterial chromosome survived. The mating mixture was then transferred to L agar containing the same antibiotic but lacking DAP which lead to the death of the donor cells. Antibiotic-resistant isolates were selected and grown as small cultures that were appropriately diluted and then plated on Luria agar with and without 5% sucrose. Sucrose-resistant isolates were screened for a phenotype associated with the presence of the defined markerless deletion mutation, such as for a requirement for DAP. In cases in which a discernible phenotype is unavailable, DNA from sucrose-resistant isolates was evaluated by PCR to identify isolates that possess the deletion of the gene in question. A wild-type strain served as a control. Table 1 lists many bacterial strains with markerless defined deletion mutations. Table 2 lists the suicide vectors for their introduction into these strains or into strains not yet possessing the defined deletion mutation. Figure 4 presents the defined deletion mutations in strains for the delivery of DNA vaccine vectors or of protective antigens by regulated lysis of the bacterial delivery strains. These include the Delta*asdA16* mutation (Kang et al., Infect. Immun. 70:1739-1749 (2002)), the Delta*asdA19*:: *araC* P_{BAD} c2 mutation (PCT/LTS01/13915), and the DeltaP_{murA7}:: *araC* P_{BAD} *murA* mutation (Figure 4). Figure 6 shows the suicide vectors for introducing these three mutations (with insertions). The transfer of unmarked defined deletion mutations from one strain to another was accomplished by a transductional method described by Kang et al. (J. Bacteriol. 184:307-312 (2002)). In this method, the suicide vector used to generate the defined unmarked deletion mutation was introduced into a strain with the same defined deletion mutation either by conjugation or electroporation into a strain with the same defined deletion mutation and an antibiotic-resistant isolate was selected. This isolate now possessed the defined deletion mutation both in its chromosome and in the suicide vector integrated into a chromosome sequence adjacent to the defined deletion mutation. Transducing phage P22 can be propagated on this isolate and the phage lysate used to transduce by selection for antibiotic resistance the integrated suicide vector and linked defined deletion mutation into other bacterial strains. Table 4 lists the P22 transducing lysates that we have and could be used to move markerless deletion mutations from one strain to another. To select for the loss of the suicide vector, we plated the strains on agar medium with 5% sucrose. The sucrose-resistant isolates have lost the suicide vector and associated antibiotic-resistance gene, but would now possess the defined unmarked deletion mutation in its chromosome. This procedure is diagramed in Figure 7.

**TABLE 4. P22 lysates**

| **P22 Lysate/Strain** | Deletion | Antibiotic Marker |
|---|---|---|
| χ8290::pMEG-611 | *Δasd419*::TT*araC*P_{BAD}*c2* | Cm |
| χ8448::pYA3484 | *ΔaraBAD1923* | Cm |
| χ8477::pYA3485 | *ΔaraE25* | Cm |
| χ8554::pMEG-443 | *ΔasdA16* | Cm |
| χ8600::pYA3547 | *ΔfliC825* | Cm |
| χ8601::pYA3548 | *ΔfljB217* | Tc |
| χ8573::pYA3529 | *ΔmsbB48* | Cm |
| χ8829::pYA3628 | *Δgmd-11* | Cm |
| χ8844::pYA3652 | Δ*endA2311* | Cm |
| χ8882::pYA3679 | *ΔrelA1123* | Cm |
| χ8925:pYA3719 | ΔP_{sifA196}::TT *araC* P_{BAD} *sifA* | Cm |
| χ8926::pYA3716 | *ΔsifA26* | Cm |

Example 2 shows the generation of suicide vectors for introduction of defined deletion and deletion-insertion mutations to contribute desirable features to bacterial strains to display regulated lysis for delivery of DNA vaccine vectors or attenuated viral vaccines or for release of antigens.

In order to develop a host-vector system with regulated delayed lysis to use in the immunization of humans, in those cases in which the DNA sequence for *S*. *typhi* and/or *S*. *paratyphi* A gene in question and its flanking sequences are different than in S. *typhimurium,* we construct suicide vectors to introduce defined deletion and deletion-insertion mutations into the chromosomes of *S*. *typhi* and *S*. *paratyphi* A. This is the case for the *asd* gene and its flanking sequences. We designed suicide vectors for introducing Delta*asdA33* and Delta*asdA183::*TT *araC* P_{BAD} c2 mutations into the chromosomes of S. *typhi* and *S*. *paratyphi* A (see Figures 4 j, 4 k and 8).

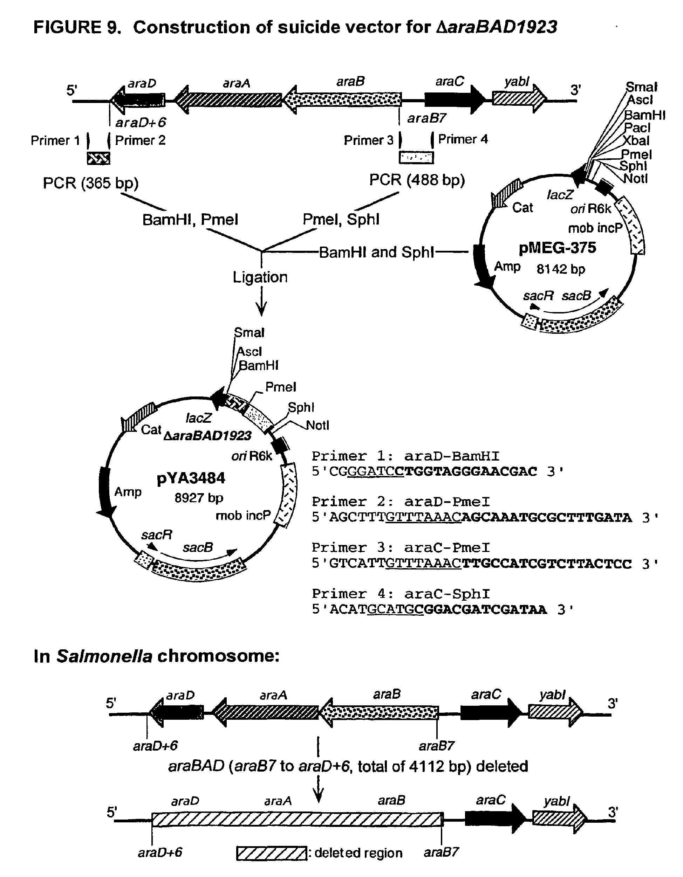

Figure 9 displays the construction of the defined Delta*araBAD1923* mutation (Figure 4 b) and the construction of the suicide vector pYA3484 (Table 2) for introducing this defined deletion mutation into the chromosome of a bacterial strain such as S. *typhimurium* UK-1 Chi3761 to generate strain Chi8448 (Table 1). Thereafter, the co-transductional method can be used to introduce the Delta*araBAD1923* mutation into any other bacterial strain desired. Inclusion of the Delta*araBAD1923* mutation in strains designed for regulated lysis delays the onset of lysis.

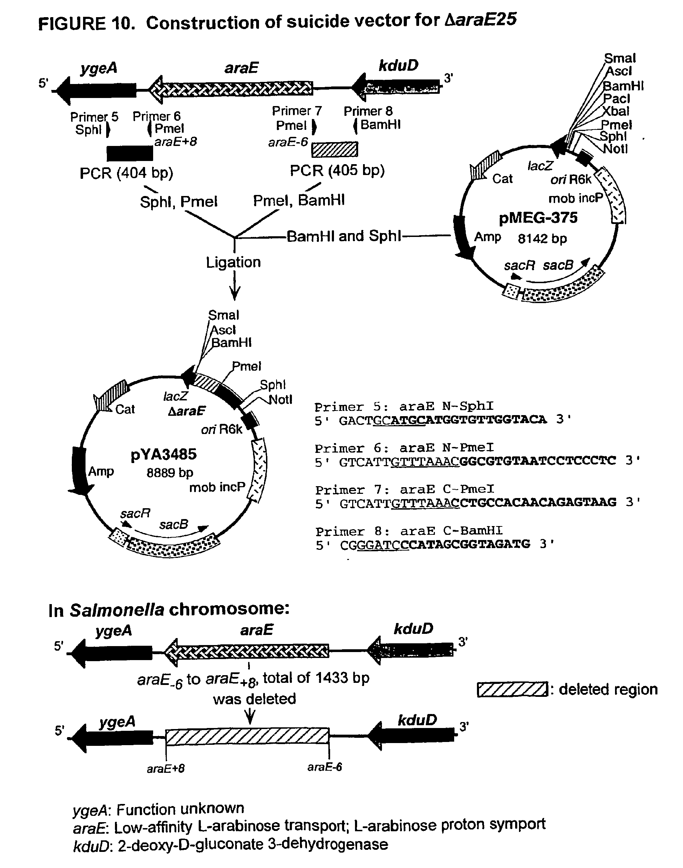

Figure 10 displays the construction of the defined Delta*araE25* mutation (Figure 4 g) and the construction of the suicide vector pYA3485 (Table 2) for introducing this defined deletion mutation into the chromosome of a bacterial strain such as S. *typhimurium* UK-1 Chi3761 to generate strain Chi8477 (Table 1). Thereafter, the co-transductional method described above can be used to introduce the Delta*araE25* mutation into any other bacterial strain desired. Inclusion of the Delta*araE25* mutation in strains designed for regulated lysis, preferably when the strain already possessed the Delta*araBAD1923* mutation, further delays onset of lysis since the arabinose taken up into cells during permissive growth does not leak out of cells via the AraE transport protein and is also not degraded due to the Delta*araBAD1923* mutation.

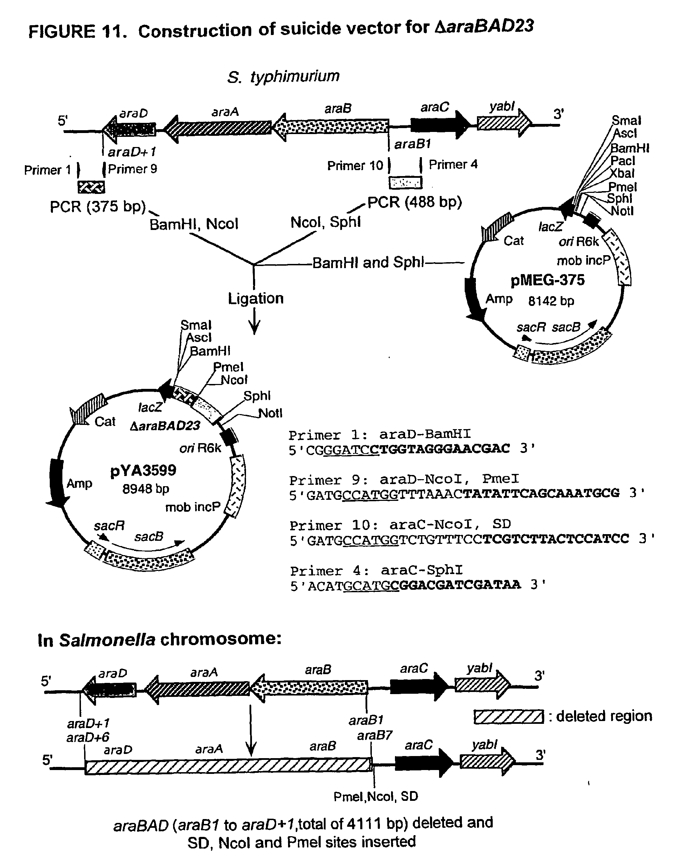

The Delta*araBAD1923* mutation (Figures 4 b and 9) leaves the start codon and codons for the first seven amino acids of the *araB* gene. Since deletion of the *araBAD* structural genes can leave the *araC* gene and the P_{BAD} promoter intact, we have developed a strategy to use this remaining *S*. *typhimurium araC* P_{BAD} promoter to drive the arabinose-dependant expression of regulatory genes specifying various repressors at this chromosome location. To accomplish this, we used a PCR method with primers that would engender an *Nco*I cloning site downstream from the P_{BAD} promoter. Since the *Nco*I restriction site includes an ATG sequence, this site facilitates cloning of entire gene sequences including their ATG start codon. This construction is diagramed in Figure s 4 and 11. The construction generated the mutation we have termed Delta*araBAD23* that can also be stated as *araC23* P_{BAD}. The suicide vector pYA3599 (Table 2) to introduce the Delta*araBAD23* mutation is diagramed in Figure 11. The suicide vector pYA3599 can be used to introduce the Delta*araBAD23* mutation into *S. typhimurium* strains such as Chi3761 to generate a strain such as Chi8767 (Table 1).

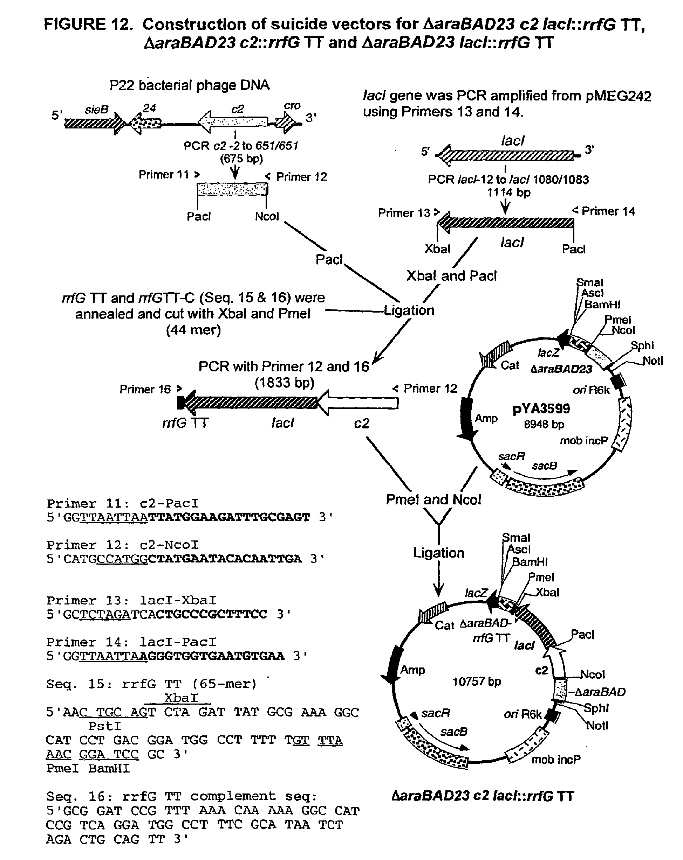

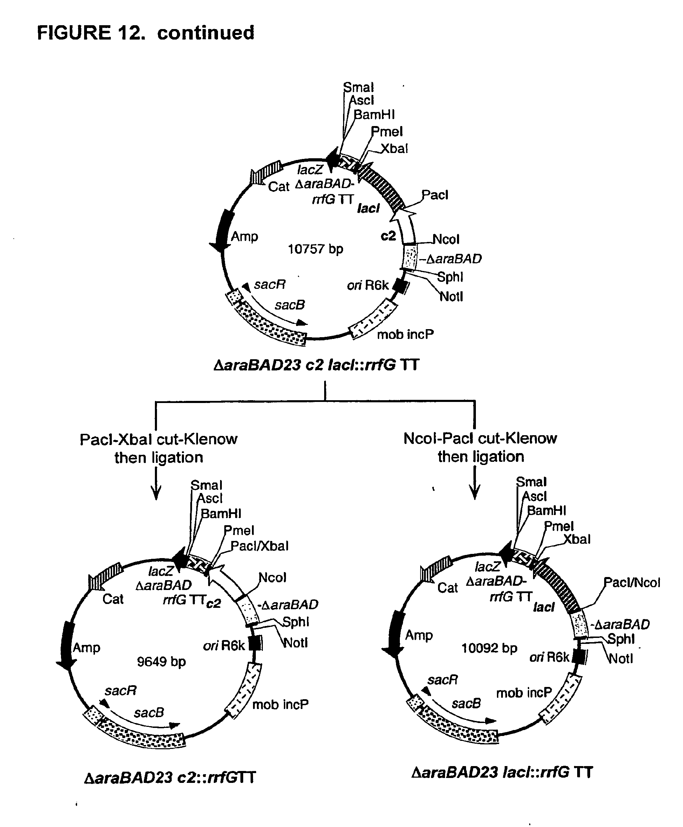

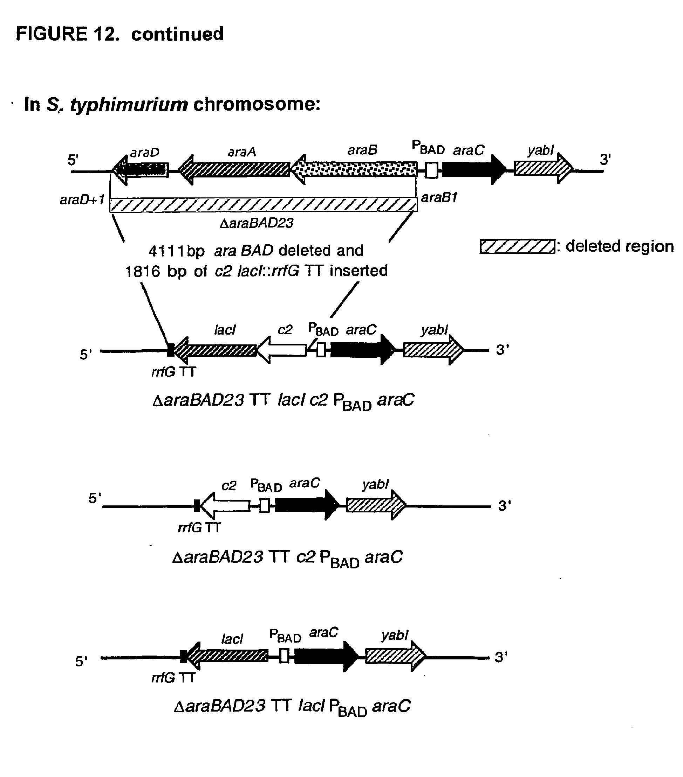

As depicted in Figure 5, both pYA3650 and pYA3651 possess the phage P22 P_{R} to cause synthesis of anti-sense mRNA for the *asd* and *murA* genes but only when the phage P22 repressor protein C2 is absent. This repressor protein is synthesized under the control of the *araC* P_{BAD} activator-promoter inserted within the chromosomal Delta*asdA19* mutation (see Figure 4 i). However, the amount of C2 repressor specified by a single chromosomal c2 gene might be insufficient to repress completely a plasmid localized P_{R} such that low-level transcription leading to anti-sense mRNA for the *asd* and *murA* genes might occur. For this reason, we insert an additional copy of the phage P22 *c2* gene into the chromosome using the *Nco*I site within the Delta*araBAD23* mutation so that the phage P22 c2 gene is controlled by the *S*. *typhimurium araC* P_{BAD} activator-promoter (Figure 11). Since we need to have synthesis of the LacI repressor of the P_{trc} promoter also under the control of an *araC* P_{BAD} activator-promoter, we designed the construction as depicted in Figure 12 to insert an operon specifying the P22 C2 repressor and the LacI repressor after which either both the repressor genes or either individually can be inserted into the Delta*araBAD23* mutation. To avoid having P_{BAD} driven transcription beyond the inserted c*2* and/or *lacI* genes that might attenuate or alter the physiology of the bacterial strain, we included the *rrfG* transcription terminator after the inserted c*2* and/or *lacI* genes to preclude such transcription. The procedures and the suicide vectors for accomplishing these objectives are presented in Figure 12. In these cases, the new Delta*araBAD23 c2 lacI*::TT (Figure 4 e) or Delta*araBAD23 c2*::TT (Figure 4 d) or Delta*araBAD23 lacI*::TT (Figure 4 f) constructions would be used to replace the existing Delta*araBAD1923* mutation (Figure 4 b) currently in strains such as Chi8854. This can be readily accomplished by use of P22 mediated-transduction and the constructed suicide vectors. Additional constructions have been designed to expand our options for potentially increasing the amount of LacI repressor that would decrease by half at each cell division following vaccination of an individual, and thus, the time needed to de-repress the LacI promoters to commence transcription of a gene encoding, for example, a protective antigen.

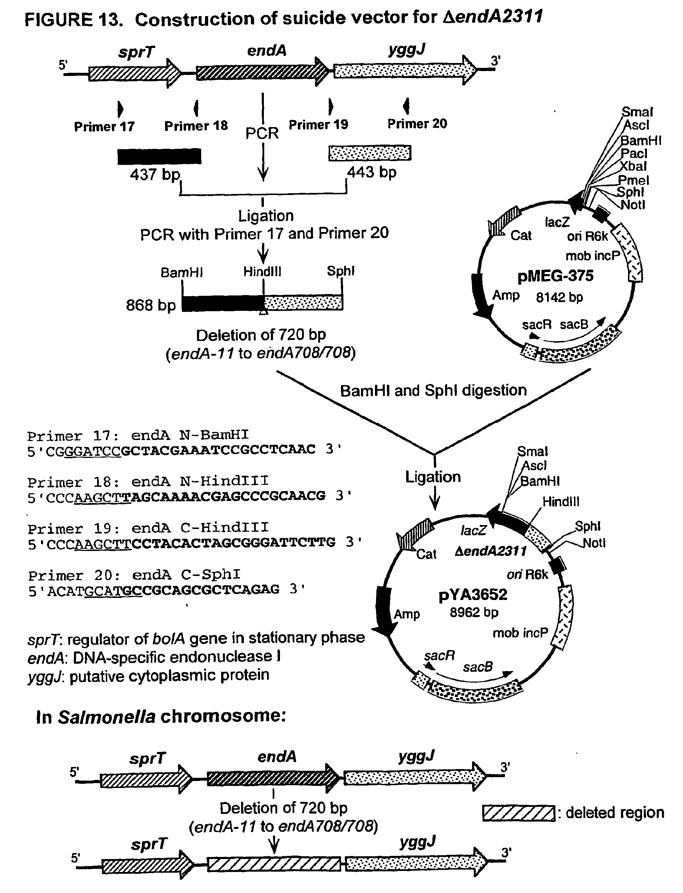

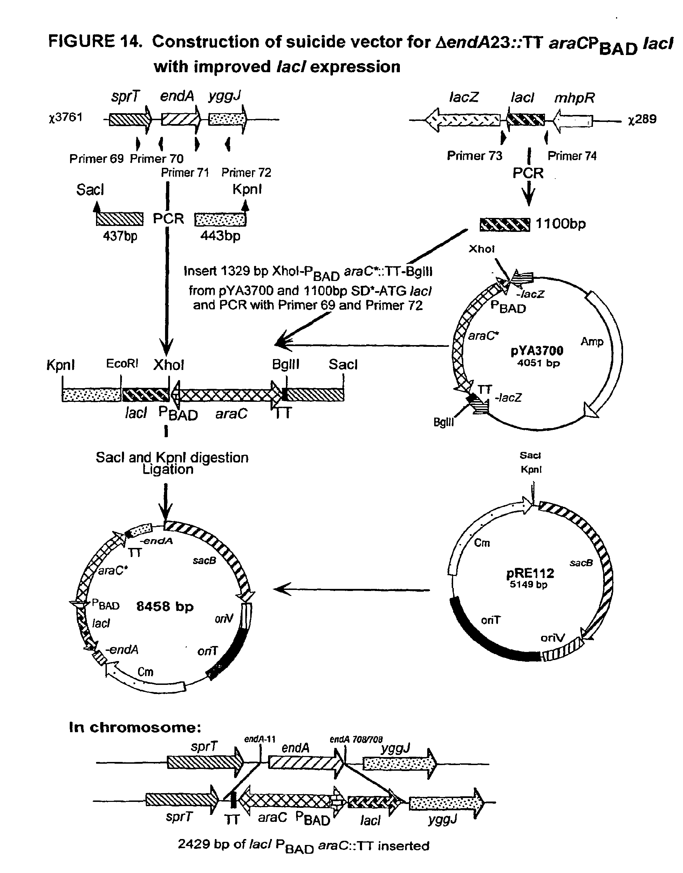

When the host strain possesses a mutation that inactivates the periplasmic endonuclease I enzyme, recovery of circular plasmid DNA is improved and the efficiency of plasmid transfer to a host strain by either transformation or electroporation is enhanced. It therefore follows that the integrity of DNA vaccine vectors released from bacteria undergoing regulated lysis *in vivo* would be superior from strains lacking endonuclease I. Thus, we designed and constructed the suicide vector pYA3652 for the introduction of the Delta*endA2311* mutation (Figure 4 m) into the *Salmonella* chromosome. Figure 13 schematically depicts the construction. The DNA vaccine vector delivery host strain Chi8854 possesses the Delta*endA2311* mutation. The transductional method of transfer on unmarked deletion mutations was also used with the suicide vector pYA3652 to move the Delta*endA2311* mutation from Chi 8844 (Table 1) into Chi8807 (Table 1) to yield Chi8854. The proposed suicide vector for introducing a TT *araC* P_{BAD} *lacI* insertion into the Delta*endA2311* chromosomal mutation is depicted in Figure 14. The native *lacI* gene in the *E. coli* K-12 chromosome has a GTG start codon and an inferior AGGG Shine-Dalgarno (SD) sequence, both of which decrease mRNA translation efficiency. The resulting Delta*endA23*::TT *araC P*_{BAD}*lacI* deletion-insertion mutation with improved expression of *lacI* is diagrammed in Figure 4 n. This deletion-insertion mutation, therefore, would provide a significant means to increase the concentration of LacI in the vaccine strain prior to immunization of an individual. This is an improved means to enhance LacI repressor concentration and obviates the usual need to express LacI from a multi-copy plasmid vector.

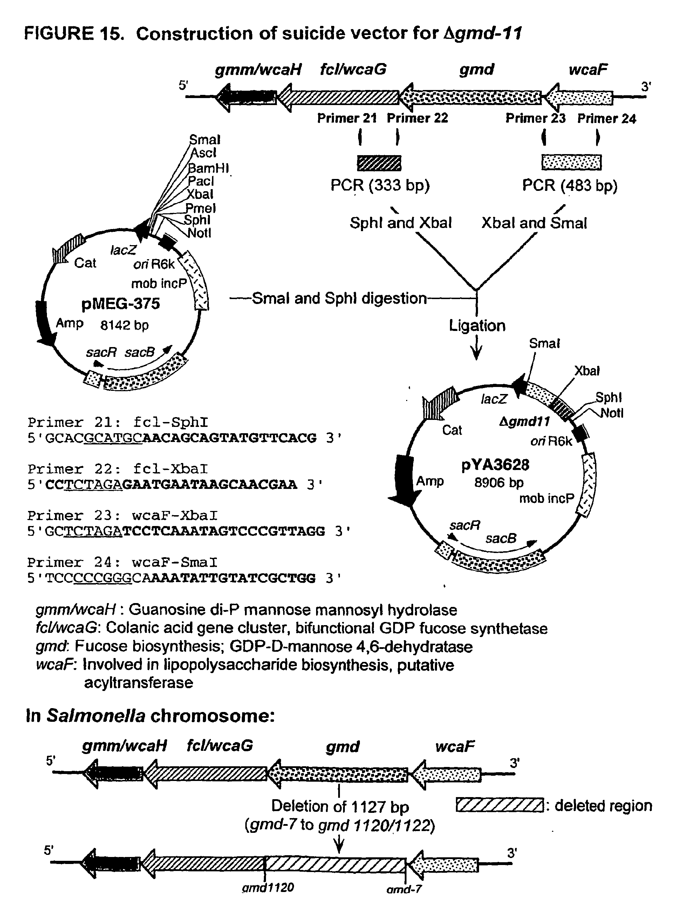

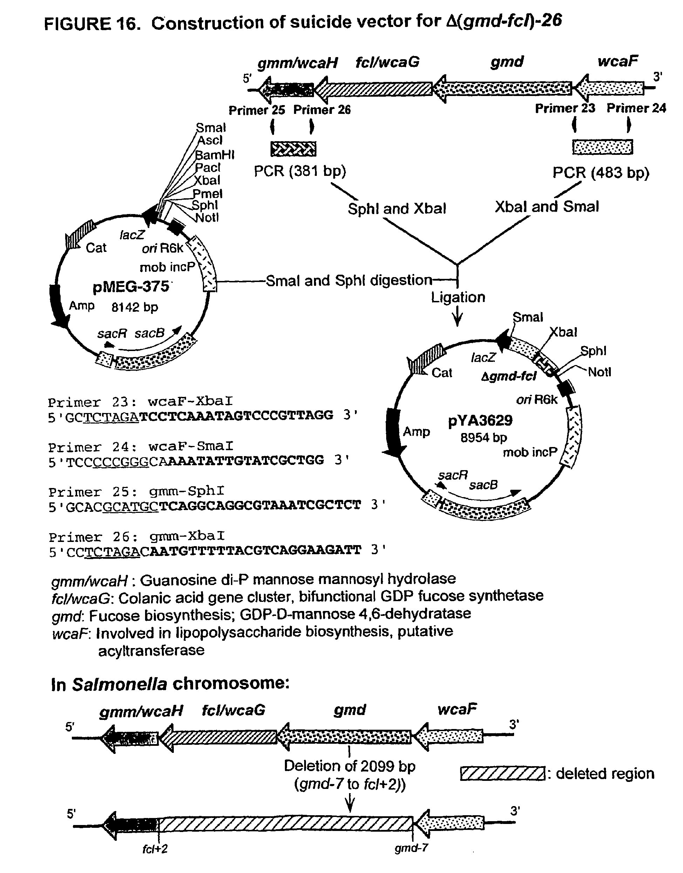

Bacteria experiencing perturbations in the synthesis of their cell walls, due to either inhibition of cell wall synthesis by antibiotics or by metabolic limitations as a result of mutational alterations, frequently respond to this type of stress by synthesizing the extracellular polysaccharide colanic acid. This behavior was observed in the design of *Escherichia coli* K-12 bacterial strains to exhibit biological containment for safe conduct of recombinant DNA research. Accordingly, we introduced mutations, present in Chi 1776, to preclude colanic acid synthesis that could enable bacterial cell to survive, while undergoing death by lysis due to the presence of *asdA* and *dapD* mutations, which block DAP biosynthesis that result in DAP-less death by cell lysis. We, therefore, have designed and generated deletion mutations that would block the conversion of GDP-mannose to GDP-fucose. GDP-fucose is necessary for the synthesis of colanic acid since fucose represents one-third of the sugar mass of colanic acid. Figure 15 diagrams the construction of the Delta*gmd-11* mutation (Figure 4 s) and the suicide vector pYA3628 for its introduction into the bacterial chromosome. Alternatively, the Delta(*gmd-fcl*)*-26* mutation, blocks colanic acid synthesis (Figure 4 t), since the mutation deletes two genes specifying two enzymes for the sequential conversion of GDP-mannose to GDP-fucose. Figure 16 diagrams the construction of the Delta(*gmd*-*fcl)*-*26* mutation and the suicide vector pYA3629 for its introduction into the bacterial chromosome.

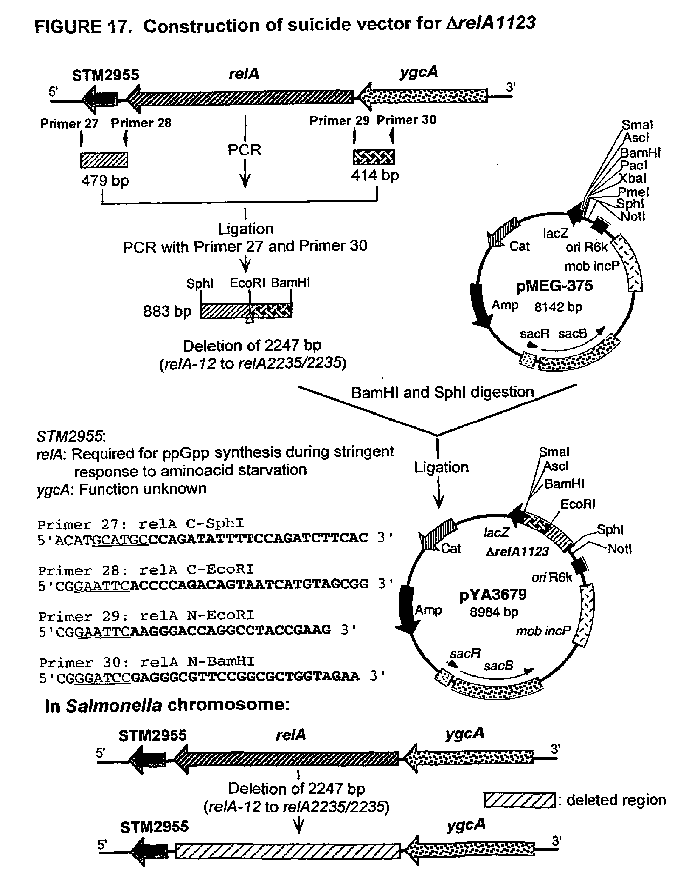

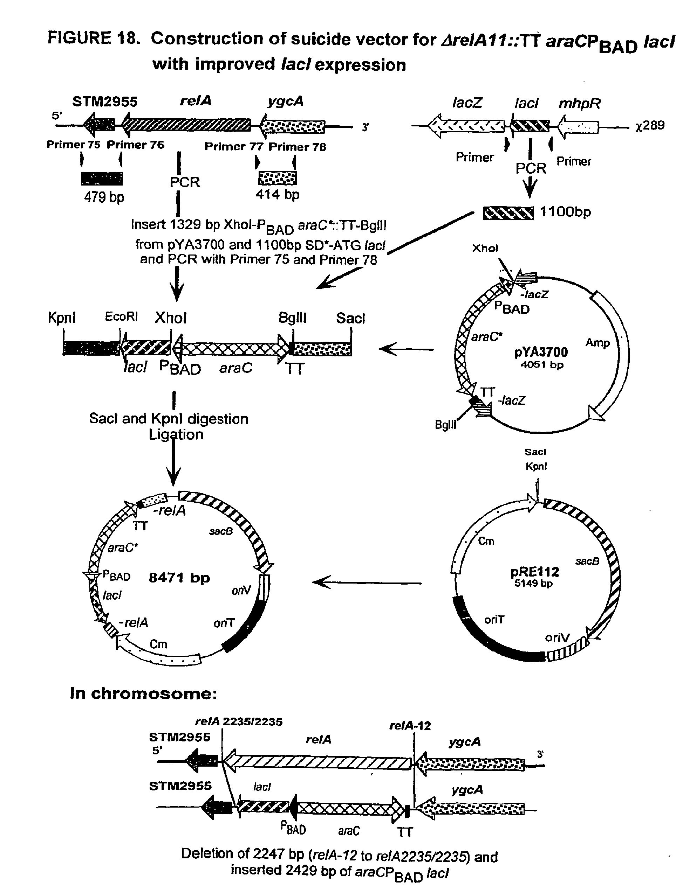

Bacterial strains that undergo lysis as a result of their inability to synthesize the rigid layer of the bacterial cell wall would cease to lyse if protein synthesis is arrested for any reason. To uncouple this interdependence for continued protein synthesis for lysis to occur, the *relA* gene was inactivated by mutation. Although protein synthesis within bacteria *in vivo* is not likely to impair DNA vaccine vector delivery by lysis, the bacterial strain might encounter some environments, following excretion in feces, for example, in which protein synthesis might be inhibited. In this case, a DNA vaccine or antigen delivery host-vector system might survive. Thus, inclusion of a Delta*relA1123* mutation would uncouple the dependence of lysis on protein synthesis and ensure the ultimate lysis and death of the DNA vaccine vector delivery host strain. Figure 17 presents the construction of the *DeltarelA1123* mutation (Figure 4 x) and the suicide vector pYA3679 for its introduction into the chromosome of DNA vaccine delivery host strains. The proposed suicide vector for introducing a TT *araC* P_{BAD} *lacI* insertion into the Delta*relA1123* chromosomal mutation is depicted in Figure 18. This deletion-insertion mutation, having an improved AGGA SD sequence and the ATG codon, instead of the GTG codon, provides an improved means to increase the concentration of LacI in the vaccine strain prior to immunization of an individual. Figure 4 y depicts the Delta*relA11*::TT *araC P*_{*BAD*}*lacI* deletion-insertion mutation with improved *lacI* expression.

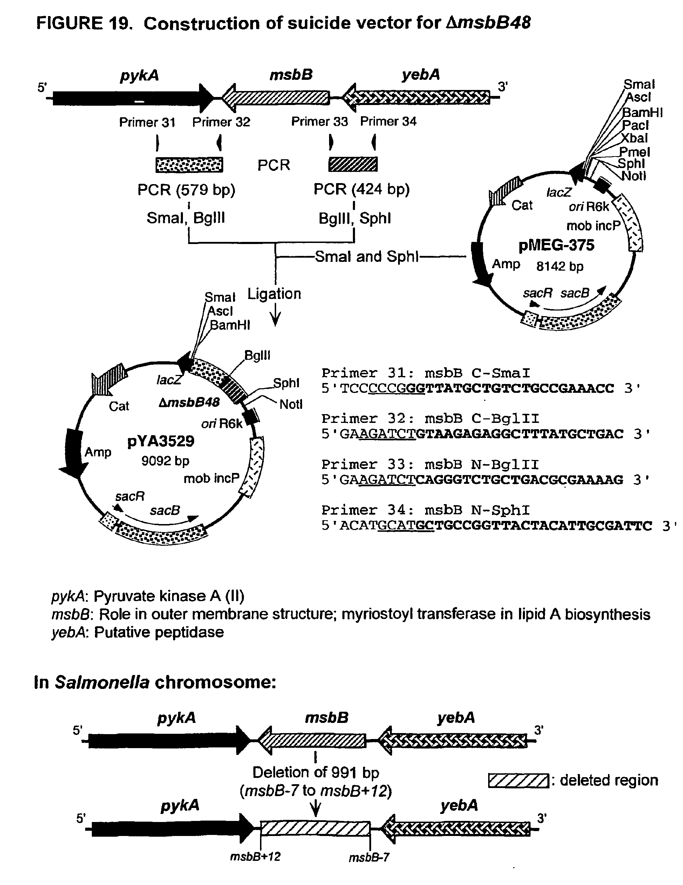

The lysis of Gram-negative bacteria *in vivo* leads to the release of lipopolysaccharide (LPS) that contains endotoxin due to the presence of lipid A. Although lipid A serves as an adjuvant to stimulate the innate immune system to produce interferon gamma, which enhances the induction of a desired cellular immune response, this inflammatory response can also induce fever and have an adverse consequence on an immunized animal or human host. There are numerous mutations that can alter the myristillation of lipid A with alterations of attached fatty acids to alter the degree of toxicity (Raetz and Whitfield, Annu. Rev. Biochem. 71:635-700 (2002)). We chose to delete the *msbB* gene to decrease LPS toxicity without altering the virulence of the *Salmonella* strain possessing this mutation. Figure 19 diagrams the construction of the Delta*msbB48* mutation (Figure 4 u) and the suicide vector pYA3529 for its introduction into the bacterial chromosome.

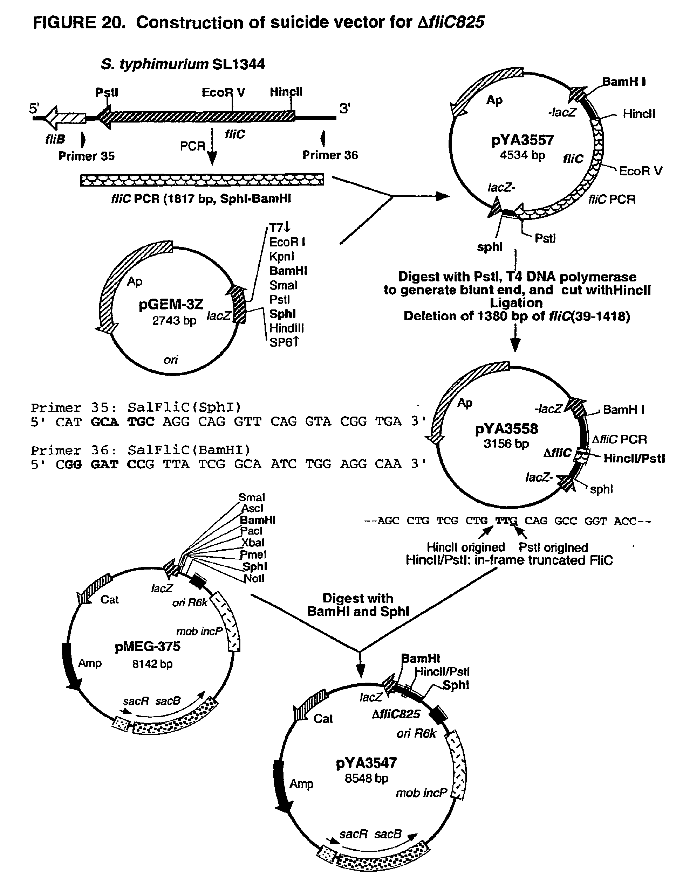

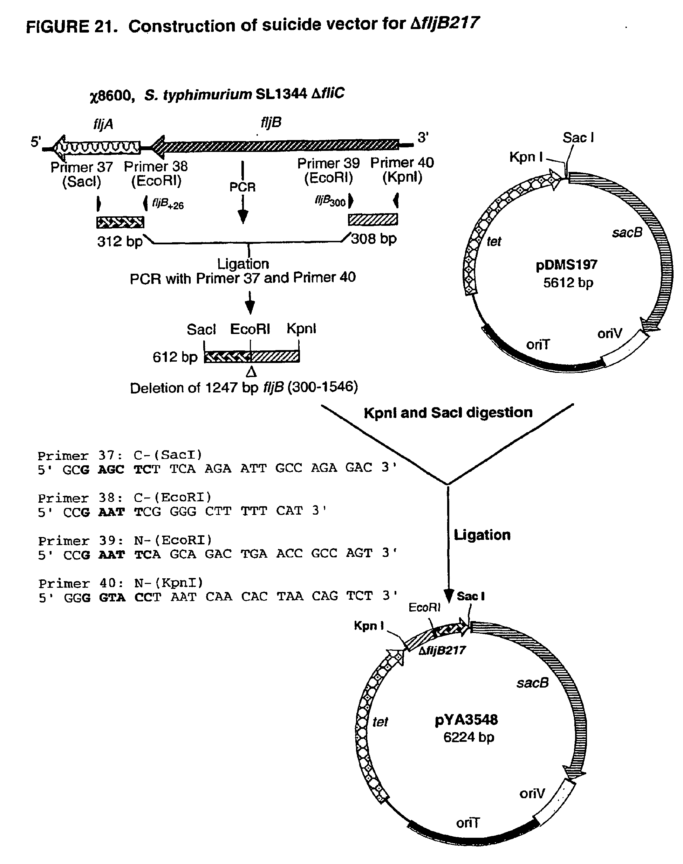

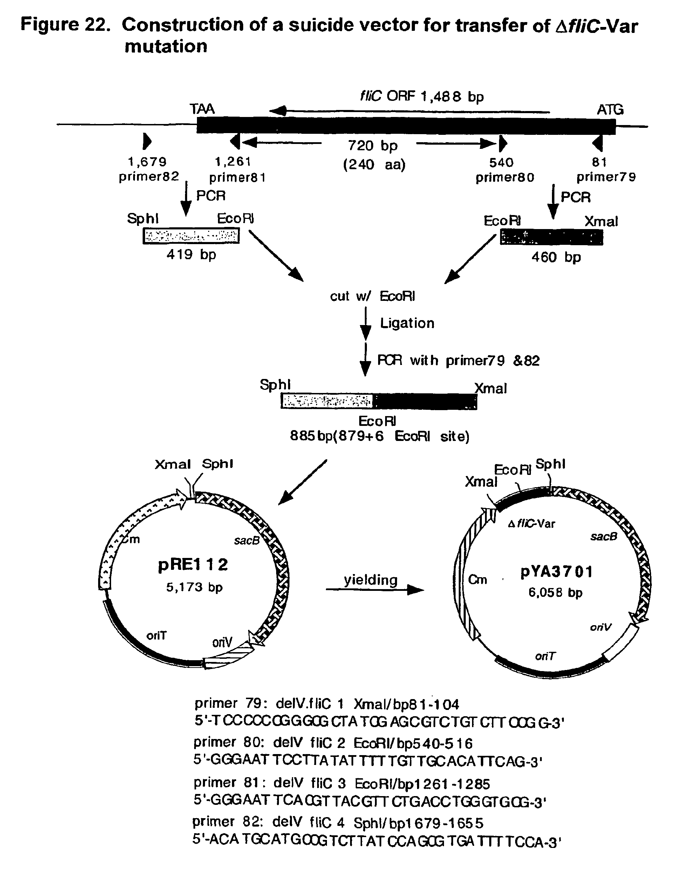

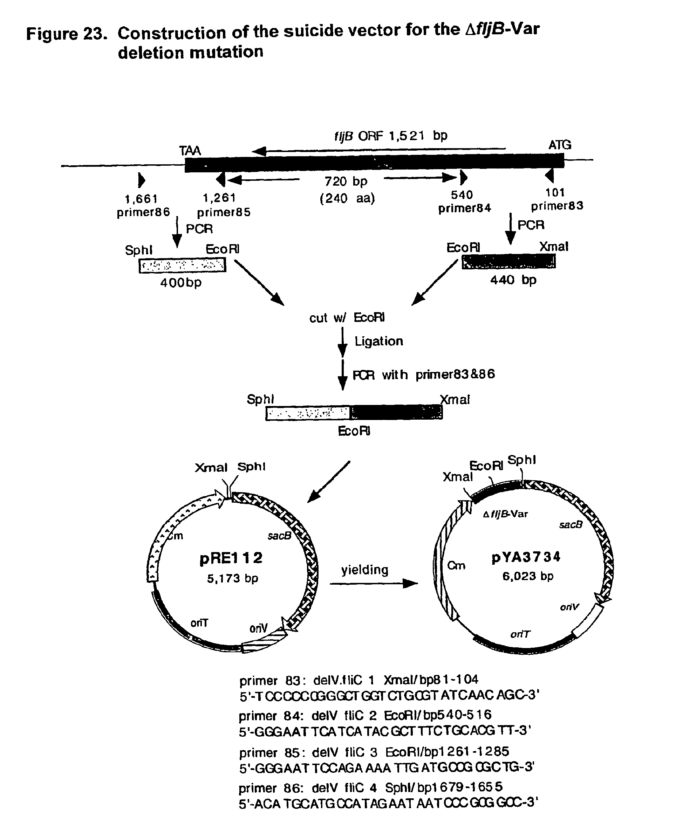

A goal in the delivery of a DNA vaccine vector by a bacterial strain exhibiting regulated lysis would be to use a bacterial strain that would efficiently deliver the DNA vaccine vector to stimulate a strong desired immune response to the antigen specified within the eukaryotic expression cassette in the DNA vaccine vector and to diminish the immune responses induced to the bacterial carrier strain. Since the flagellar antigens of *Salmonella* induce a dominant T cell immune response (Cookson and Bevan, J. Immunol. 158:4310-4319 (1997)) and their absence has no influence on *Salmonella* invasiveness to lymphoid tissues or virulence (Lockman and Curtiss, Infect. Immun. 60:491-496 (1992)), we generated the Delta*fliC825* (Figure 4 o) and the Delta*fljB217* (Figure 4 q) mutations and the pYA3547 and pYA3548 suicide vectors for introducing these mutations into the bacterial chromosome, respectively. The constructions for introducing the Delta*fliC825* mutation are diagrammed in Figure 20 and for introducing the Delta*fljB217* mutation in Figure 21. Because bacteria display phase variation in regard to expressing different flagellar antigens in different cells in the bacterial population, it is necessary to delete both genes encoding the two phase flagellar antigens, specified by the *fliC* and *fljB* genes in *S. typhimurium.* Alternatively, since the constant N- and C-terminal regions of the flagellar protein constitute a PAMP that is recognized by TLR5 to stimulate and recruit an innate immune response, we designed new deletion mutations that removed the variable domains of the FliC and FljB proteins but retained the ability for TLR5 recognition (Hayashi et al., Nature 410:1099-1103 (2001); Donnelly and Steiner, J. Biol. Chem. 277:40456-40461 (2000)). Figure 22 and 23 diagram the suicide vectors to introduce these Delta*fliC*-Var (pYA3701) and Delta*fljB*-Var mutations, respectively. Figure 4 diagrams the chromosomal mutations.

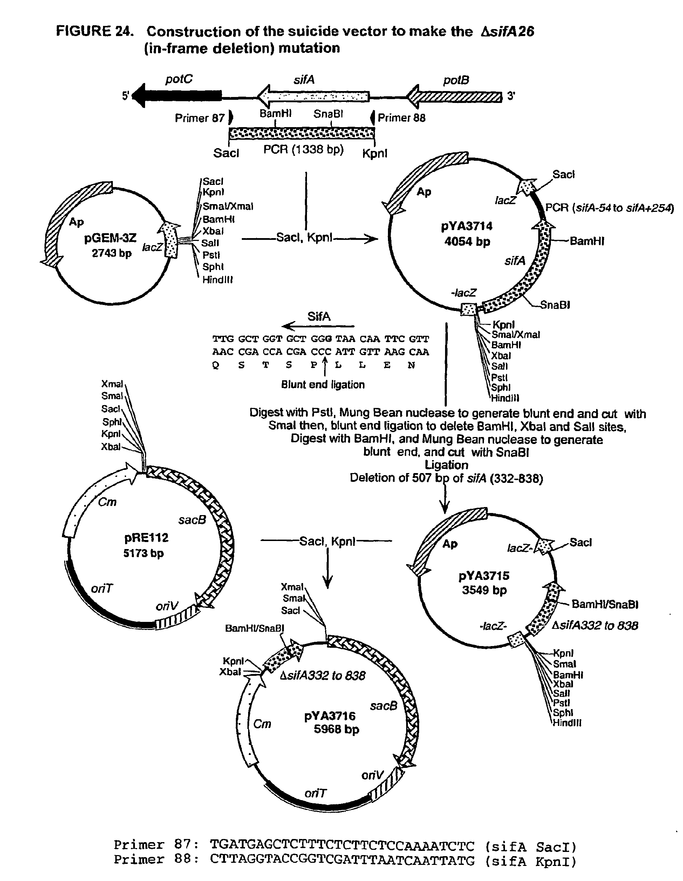

One means to enable *Salmonella* to escape from the endosome is to delete the *sifA* gene (Stein *et al.,* Mol. Microbiol. 20:151-164 (1996); Brumell *et al.,* Traffic 2002:3:407-415 (2002)). Figure 24 diagrams the suicide vector pYA3716 to introduce the Deltasif*A26* mutation (with an internal in-frame deletion of the *sifA* gene) into the chromosome to generate Chi8926. To minimize the adverse effects of a deletion of the *sifA* gene, we made another construction to place the chromosomal *sifA* gene under the control of *araC* P_{BAD}, such that the SifA protein would be in abundance early in infection and be diluted out as a consequence of cell division, to result in endosome escape after substantial colonization of lymphoid tissues has occurred. Figure 25 diagrams the suicide vector pYA3719 to introduce this chromosomal DeltaP_{sifA196}::TT *araC* P_{BAD} *sifA* deletion-insertion mutation. Figures 4 z and 4 aa depict both the *DeltasifA26* and DeltaP_{sifA196}::TT *araC* P_{BAD} *sifA* constructions. The constructs are introduced into Chi8888 using the transductional method described in Example 1.

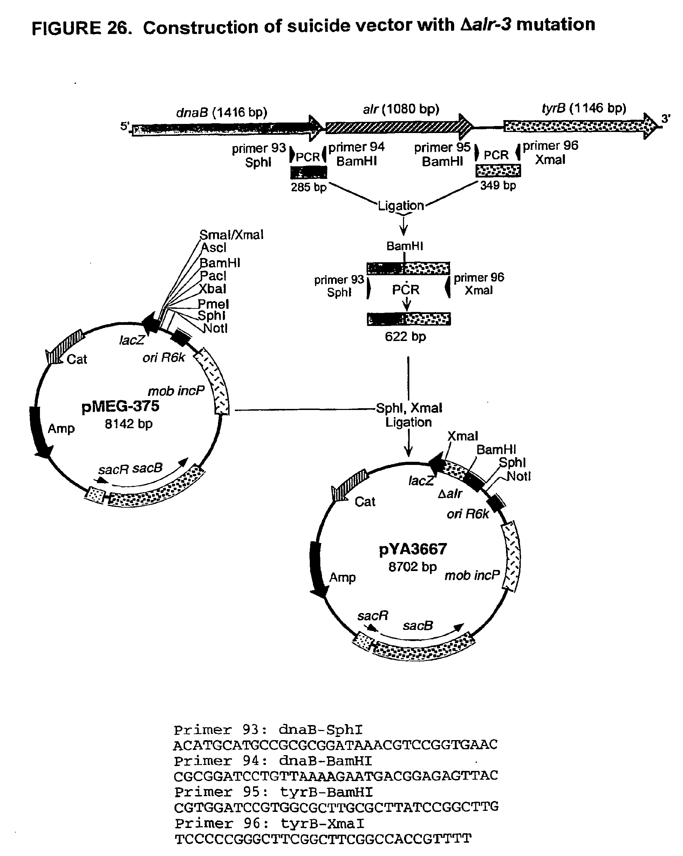

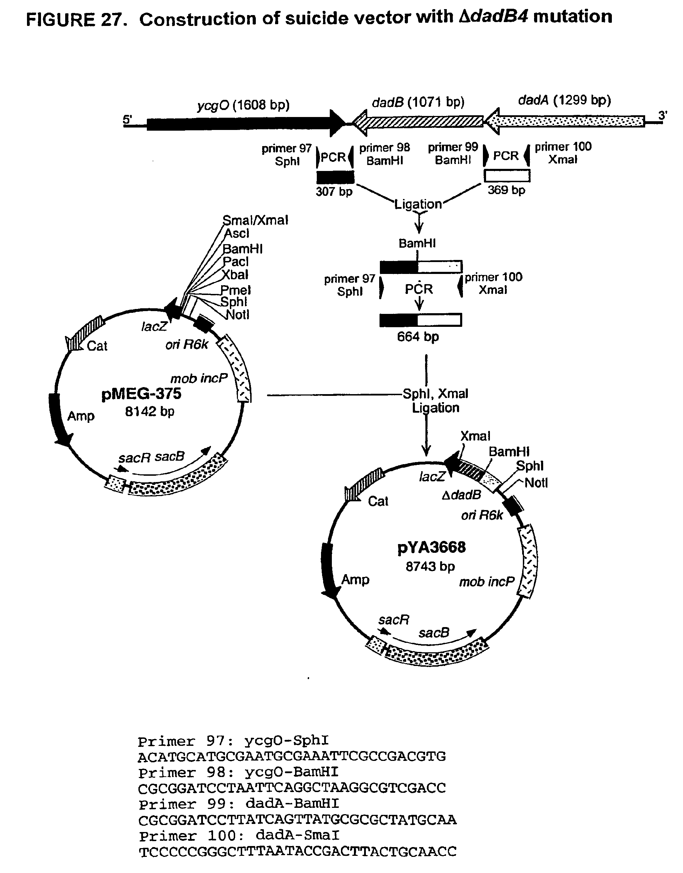

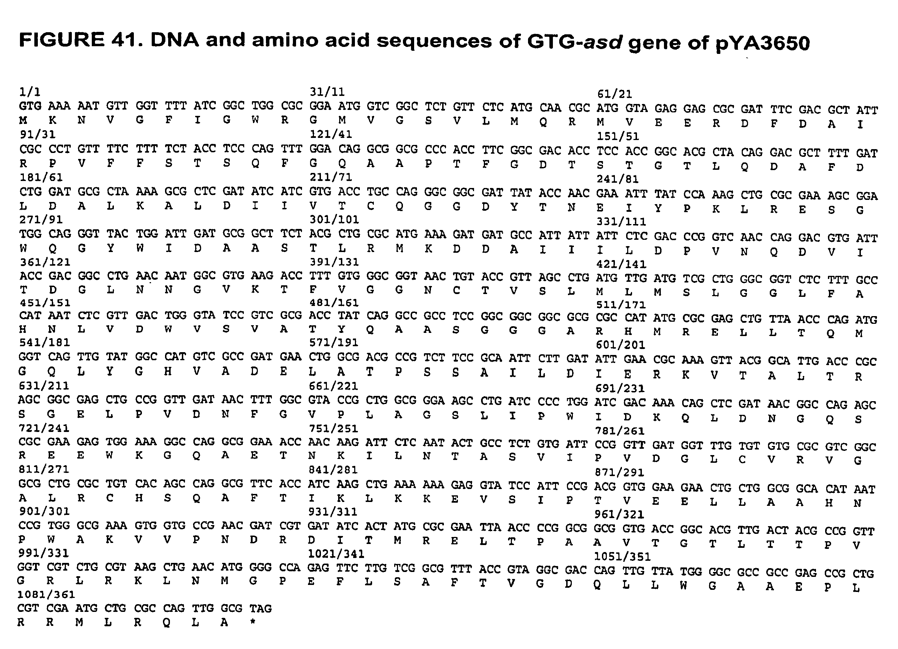

D-alanine is an essential constituent of the rigid layer of the cell wall in all bacteria and is incorporated into the peptidoglycan as D-alanyl-D-alanine. After covalent linkage to the L-alanine-D-glutamate-DAP tripeptide that is attached to the muramic acid-N-acetyl-glucosamine carbohydrate backbone of the murein layer, the terminal D-alanine is cleaved off to create the crosslink for the next muramic acid-N-acetyl-glucosamine chain. The synthesis of D-alanine in gram-negative bacteria is carried out by two genes, *alr* encoding alanine racemase, which interconverts D-alanine and L-alanine, and *dadB* which encodes a catabolic enzyme that enables metabolism of D-alanine as a carbon source but which can synthesize D-alanine when acting in the reverse reaction. We constructed the suicide vector pYA 3667 (Figure 26) to introduce the Delta*alr-3* mutation (Figure 4 a) into the chromosome to yield Chi8898 (Table 1). We also constructed another suicide vector pYA3688 (Figure 27) to introduce the Delta*dadB4* mutation (Figure 41) into the chromosome to yield Chi8897 (Table 1). The P22 transduction method has been used to make the double mutant strain Chi8899 (Table 1) that has both the Deltaalr-3 and Delta*dadB4* mutations. Chi8897 and Chi8898 can grow with or without D-alanine in the growth medium, but Chi8899 with both deletion mutations has a requirement for D-alanine. In the absence of D-alanine, Chi8899 undergoes cell wall-less death and lyses. It is evident from these observations that the *alr* or *dadB* gene could be used in lieu of or in addition to the *asd* and/or *murA* genes in the various vectors for regulated delayed lysis.

Alternatively, the *Deltaalr*-3 and *DeltadadB4* mutations can be introduced into the chromosome of a host-vector strain to enable use of an Alr⁺ or DadB⁺ plasmid to specify synthesis of an antigen that can be delivered to an immunized host. Various uses of this system are described in subsequent Examples to facilitate immunization of individuals with multiple antigens to induce immunity to a pathogen, to accomplish induction of infertility, or to develop a therapeutic vaccine against a particular cancer. The delivery of the different antigens by such a combination host-vector strain can be designed to stimulate specific types of mucosal, systemic and/or cellular immunities.

**Example 3**

Example 3 demonstrates the virulence and invasive properties of *S*. *typhimurium* strains with single mutations that are or can be included in strains exhibiting regulated lysis for DNA vaccine delivery and/or antigen release.

Table 5 provides data from a number of determinations of the relative virulence of *S. typhimurium* strains. Strains are grown in LB broth to an OD₆₀₀ of ~0.8, concentrated in BSG with 20 microl samples administered orally to mice that had been fasted for food and water for about 4 h. Food and water were returned 30 min after infection. The LD₅₀ for the wild-type *S*. *typhimurium* UK-1 strain Chi3761 is about 5 × 10⁴ based on multiple experiments. Strains such as Chi8276 and Chi8290 that possess Delta*asd* mutations are total avirulent independent of dose, a result supported in the literature. The DeltaP_{murA7}:: *araC* P_{BAD} *murA* mutant strain Chi8645 caused some lethal infections in one experiment but no deaths in another (Table 5). Chi8897 with the Delta*dadB4* mutation and Chi8898 with the Deltaalr-3 mutation have the same virulence and LD₅₀ as the wild-type parent Chi3761. Chi8899, on the other hand, is totally avirulent when used to orally inoculate mice with or without supplementing D-alanine or D-alanyl-D-alanine in the drinking water (Table 5). S. *typhimurium* strains with the Deltaara*BAD1923,* Delta*araE25,* Delta*endA2311,* Delta*gmd-11*, Delta*(gmd*-*f*c*l)-26,* Delta*relA4,* Delta*relA1123,* Delta*msbB48* and Delta*fliC825* Delta*fljB217* mutations all display virulence that is essentially the same, considering statistical noise, as that exhibited by the wild-type parents of these strains, Chi3761 and Chi3339 (Table 1). In regard to mutations that delete genes for arabinose utilization, numerous strains of *Salmonella* including *S*. *typhi* are unable to ferment or utilize arabinose with no impact on virulence.

**Table 5. Evaluation of mutant strains of bacteria for virulence after oral infection of 8-week old female BALB/c mice.**

| Strain | Genotype | CFU/dose | Survival/total |
|---|---|---|---|
| χ3761 | UK-1 wild-type | 1 x 10⁷ | 0/2 |
| | | 1 x 10⁶ | 1/5 |
| | | 1 x 10⁵ | 1/5 |
| χ3761 | wild-type | 1.5 x 10⁶ | 0/4 |
| | | 1.5 x 10⁵ | 1/4 |
| | | 1.5 x 10⁴ | 3/4 |
| | | 1.0 x 10³ | 4/4 |
| χ3761 | wild-type | 9 x 10⁵ | 0/4 |
| | | 9 x 10⁴ | 2/4 |
| χ8276 | *ΔasdA16* | 6.0 x 10⁸ | 5/5 |
| | | 9.8 x 10⁸ | 4/4 |
| χ8290 | *ΔasdA19*::TT *araC* P_{BAD} *c2* | 1.0 x 10⁹ | 4/4 |
| χ8448 | *ΔaraBAD1923* | 1.0 x 10⁸ | 0/4 |
| | | 1.0 x 10⁷ | 1/4 |
| | | 1.0 x 10⁶ | 3/4 |
| | | 1.0 x 10⁵ | 4/4 |
| χ8477 | *ΔaraE25* | 1.1 x 10⁸ | 0/4 |
| | | 1.1 x 10⁷ | 1/4 |
| | | 1.1 x 10⁶ | 1/4 |
| | | 1.1 × 10⁵ | 2/4 |
| χ8645 | *ΔP*_{murA7}::*araC*P_{BAD}*murA* | 1.2 x 10⁸ | 5/5 |
| | | 1.2 x 10⁷ | 4/5 |
| | | 1.2 x 10⁶ | 4/5 |
| χ8645 | ΔP_{murA7}::*araC* P_{BAD}*murA* | 1.1 x 10⁹ | 5/5 |
| χ8829 | *Δgmd-11* | 6.9 × 10⁵ | 0/4 |
| | | 6.9 × 10⁴ | 2/4 |
| | | 6.9 × 10³ | 4/4 |
| χ8829 | *Δgmd-11* | 8.0 × 10⁵ | 0/4 |
| | | 8.0 × 10⁴ | 3/4 |
| | | 8.0 x 10³ | 4/4 |
| χ8844 | *ΔendA2311* | 8.6 x 10⁶ | 0/4 |
| | | 8.6 x 10⁵ | 2/4 |
| | | 8.6 x 10⁴ | 2/2 |
| χ8844 | *ΔendA2311* | 3.0 x 10⁵ | 0/2 |
| | | 3.0 x 10⁴ | 1/2 |
| χ8831 | *Δ(gmd-fcl)-26* | 5.9 x 10⁵ | 1/4 |
| | | 5.9 x 10⁴ | 4/4 |
| | | 5.9 x 10³ | 4/4 |
| | | 5.9 x 10² | 4/4 |
| χ8831 | *Δ(gmd-fcl)-26* | 8.6 x 10⁶ | 0/4 |
| | | 8.6 x 10⁵ | 0/4 |
| | | 8.6 x 10⁴ | 0/4 |
| | | 8.6 x 10³ | 1/4 |
| χ8619 | *ΔrelA4* | 1.6 x 10⁹ | 0/5 |
| | | 1.6 x 10⁷ | 0/5 |
| | | 1.6 x 10⁶ | 3/5 |
| | | 1.6 x 10⁵ | 5/5 |
| | | 1.6 x 10⁴ | 5/5 |
| χ8882 | *ΔrelA1123* | 8.0 x 10⁷ | 0/4 |
| | | 8.0 x 10⁶ | 1/5 |
| | | 8.0 x 10⁵ | 1/4 |
| | | 8.0 x 10⁴ | 3/4 |
| | | 8.0 x 10³ | 4/4 |
| χ8573 | *ΔmsbB48* | 7.6 x 10⁵ | 2/5 |
| | | 7.6 x 10⁴ | 5/5 |
| | | 7.6 x 10³ | 5/5 |
| χ8899 | *Δalr-3 ΔdadB4* | 1.1 x 10⁹ | 5/5 |
| χ8899 | *Δalr-3 ΔdadB4* | 1.1 x 10⁹ | 5/5 |
| | PO CFU/Dose | 1.1 x 10⁸ | 5/5 |
| | + 5mg/ml DL-Alanine | | |
| *χ*8899 | *Δalr-3 ΔdadB4* | 1.1 x 10⁹ | 5/5 |
| | PO CFU/Dose | 1.1 x 10⁸ | 5/5 |
| | + 5mg/ml DL-Alanyl | | |
| | DL-Alanine | | |
| χ3339 | SL1344 wild-type | 1.0x10⁶ | 0/4 |
| χ8602 | *ΔfliC825 ΔfljB217* | 2.9 x 10⁶ | 0/4 |
| SL1344 | | 2.9 x 10⁵ | 1/4 |
| | | 2.9 x 10⁴ | 4/4 |

**Example 4**

Example 4 shows construction of the DNA vaccine vector delivery strain Chi8854. The *S. typhimurium* UK-1 strain Chi8645 with the DeltaP_{murA7}:: *araC* P_{BAD} *murA* deletion-insertion mutation, which was derived from the wild-type strain Chi3761, was the starting strain. This strain is arabinose-dependent, so we added 0.1 % arabinose to LB and L broth or agar media because they both contain yeast extract which contains some arabinose. The suicide vector pYA3485 (Figure 10) that possessed the Delta*araE25* mutation was introduced into the suicide donor strain MGN-617 (Table 1) by electroporation. The MGN-617 (pYA3485) strain was mated with Chi8645 and chloramphenicol-resistant transconjugants selected on L agar medium containing 0.1 % arabinose without DAP. Several isolates were purified by re-streaking on chloramphenicol-containing plates. Small cultures were grown up in L broth without chloramphenicol and when they reached a density of 10⁸ CFU or more were diluted and plated on L agar medium (with 0.1% arabinose) with and without 5% sucrose. When sucrose-resistant isolates were 100 times or more less prevalent than the titer of bacteria plated on media without sucrose, there is an excellent chance that allele replacement has occurred. We then screened for poor fermentation on MacConkey agar with 0.5% arabinose since *araE* mutants are defective in arabinose uptake. Such isolates were obtained and shown to give a good fermentation reaction for arabinose when plated on MacConkey agar with 1% arabinose. The presence of the Delta*araE25* mutation was verified by PCR and the strain shown to possess wild-type levels of LPS both by agglutination with Group B antisera and by gel electrophoresis and silver staining. We stocked one isolate as Chi8653.

We next introduced the Delta*araBAD1923* mutation by mating Chi8653 with MGN-617 (pYA3484). The suicide vector pYA3484 (Figure 9) has the Delta*araBAD1933* mutant allele and for ease of detection, we introduced this mutation after the Delta*araE25* mutation has been introduced. The methods were as described above except that we screened sucrose-resistant isolates for inability to ferment 1 % arabinose or to grow on minimal agar with arabinose as sole carbon source. An isolate whose mutations were confirmed by PCR and which had wild-type LPS was stocked as Chi8655.

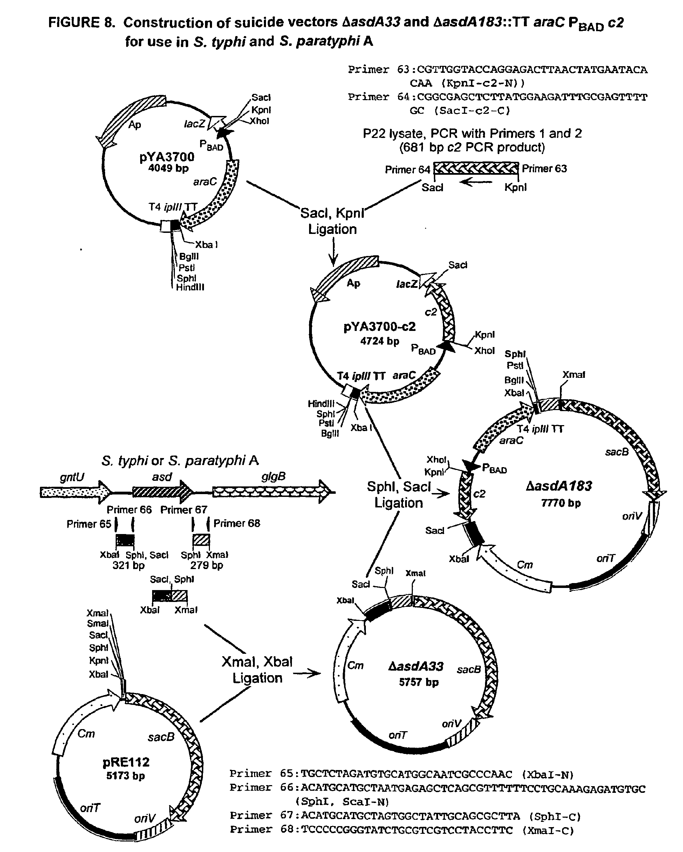

We next introduced the Delta*asdA19*::TT *araC* P_{BAD} *c2* deletion-insertion mutation into Chi8655. DAP was present during all steps. To do this, the pMEG-611 suicide vector (Figure 6) was introduced by conjugation from MGN-617 into Chi8290 (Table 1) which has the Delta*asdA19*::TT *araC* P_{BAD} *c2* mutation-insertion. Chloramphenicol-resistant isolates were selected which resulted from integration of the suicide vector into the chromosome adjacent to the defined deletion-insertion mutation, Phage P22 was propagated on a mixture of these integrants and the lysate used to transduce Chi8655 to chloramphenicol resistance. We then selected sucrose-resistant isolates and 100% had lost the suicide vector and possessed the Delta*asdA19*::TT *araC* P_{BAD} *c2* mutation-insertion. All properties for arabinose-dependent growth, DAP requirement and inability to ferment arabinose were confirmed. An isolate with wild-type LPS was stocked as Chi8807. As stated above, the regions flanking the *asd* gene differ in *S. typhi* and *S. paratyphi* A as compared to *S*. *typhimurium.* We construct the Deltaasd and Delta*asd*::TT *araC* P_{BAD} c2 mutations (Figure 8) to make *S. typhi* and *S. paratyphi* A strains with the regulated delayed lysis phenotype for the immunization of humans.

We next introduced the Delta*endA2311* mutation. The suicide vector pYA3652 (Figure 13) with the Delta*endA2311* mutation was transferred into the suicide vector donor strain MGN-617. MGN-617 (pYA3652) was mated with Chi8844 (Table 1), which also has the Delta*endA2311* mutation. Chloramphenicol-resistant isolates were selected and a mixture was used for propagation of phage P22. The P22 lysate was used to transduce Chi8807 to chloramphenicol resistance and these were subjected to 5% sucrose to select for excision of the pYA3652 suicide vector. The presence of the *endA2311* mutation was confirmed by PCR as was the presence of all other mutations. The resulting strain was stocked as Chi8854. Chi8854 is DAP requiring, arabinose dependant for growth and is unable to ferment or utilize arabinose as a carbon source.

**Example 5**

Example 5 shows modifications of Chi8854 to provide features to further ensure complete lysis of the host-vector system. Either the Delta*gmd*-*11* or Delta*(gmd-fcl)-26* mutation was introduced using Chi8829 and pYA3628 (Figure 15) or Chi8831 and pYA3629 (Figure 16). The presence of such mutations provides additional safety by decreasing the possibility that the vaccine strain could make colanic acid to survive as exopolysaccharide-encased spheroplasts during the stressful process of undergoing cell wall-less death. We introduced the Delta*(gmd-fcl)-26* mutation that yielded Chi8885.

We next introduced the Delta*relA1123* mutation present in Chi8882 into Chi8885 using the suicide vector pYA3679 (Figure 17). The presence of the *relA* mutation uncouples the effect of inhibition of protein synthesis and lysis. Inhibition of protein synthesis occurs because of depletion of the amino acids threonine, isoleucine, methionine and lysine (whose syntheses are dependent on the Asd enzyme). Lysis occurs because of depletion of the essential constituents DAP and muramic acid required for cell wall synthesis. The introduction of Delta*relA1123* mutation into Chi8885 yielded strain Chi8888.

**Example 6**

Example 6 shows further modifications of strain Chi8854 or strain Chi8888 to enhance efficacy and safety as a DNA vaccine vector delivery host.

A diversity of genotypic alterations of Chi8854 and/or Chi8888 is possible using available strains with defined deletion mutations as listed in Table 1 and suicide vectors as listed in Table 2. These constructions make use of the transductional method of strain construction with markerless deletion mutations as described in Examples 1 and 2. The Delta*msbB48* mutation changes the myristillation of fatty acids on lipid A of LPS and decreases the toxicity of the LPS endotoxin. This may be important if the release of endotoxin due to cell lysis *in vivo* causes adverse symptoms. The presence of the Delta*msbB48* mutation is without effect on the virulence of *S. typhimurium* (see Table 5). Using the suicide vector pYA3529 (Figure 19), the Delta*msbB48* mutation was introduced into Chi8854 which yielded Chi8884 (Table 1). This mutation can easily be introduced into Chi8888 or one of its derivatives.

If additional sustained growth *in vivo* prior to onset of lysis is beneficial, the Delta*araBAD1923* mutation in Chi8888 can be replaced with the Delta*araBAD23 c2::rrfG* TT mutation using the suicide vector diagrammed in Figure 12. This would provide a second *araC* P_{BAD} regulated c2 gene in the chromosome (the other is in the Delta*asdA19*::TT *araC* P_{BAD} c2 mutation-insertion) and double the concentration of C2 repressor protein to repress transcription from the P22 P_{R} present on the plasmid vectors and thus delay, for about one cell doubling, synthesis of anti-sense mRNA to inhibit translation of *murA* and *asd* mRNA.

The Delta*fliC825* mutation in Chi8600 can be introduced into Chi8888 using the suicide vector pYA3547 (Figure 20). Following this step to eliminate the phase 1 flagellar antigen, a motile isolate is selected to express the phase 2 flagellar antigen FljB. The Delta*fljB217* mutation can then be introduced using Chi8601 and the suicide vector pYA3548 (Figure 21). Introducing the Delta*fliC825* and Delta*fljB217* mutations would eliminate two potent competing *Salmonella* T-cell antigens. If the immune responses to antigens expressed and delivered or specified by DNA vaccines delivered by the host-vector system are diminished when the strain has deletion mutations eliminating all the coding sequences for the FliC and FIjB flagellar antigens, the Delta*fliC*-Var and Delta*fljB*-Var mutations (Figures 22 and 23) are introduced into Chi8888 or its derivative instead of the Delta*fliC825* and Delta*fljB217* mutations. This permits the host-vector strain to express the N- and C-terminal portions of flagellar antigens to serve as the PAMPs, to interact with TLR5 to trigger an innate immune response. The constructed strain is unable to induce the serotype-specific antibody response against the variable flagellar antigen domains.

The use of additional plasmid vectors to specify synthesis or delivery of additional antigens that encode the genetic information for the regulated delayed lysis phenotype can be developed using the *alr*^{*+*} or *dadB*^{*+*} wild-type genes in a bacterial strain that have both the Deltaalr-3 and Delta*dadB4* mutations. Such a host-vector combination ensures the stable maintenance of the plasmid vector in the host-vector strain *in vivo.* The P22-mediated transduction system described in Example 1 is used to transfer the Delta*alr-3* mutation from Chi8898 (Table 1) through the suicide vector pYA3667 (Figure 26) and the Delta*dadB4* mutation from Chi8897 (Table 1) through the suicide vector pYA3688 (Figure 27) into Chi8888 or a derivative. The presence of both mutations confers a requirement for D-alanine in the growth media.

**Example 7**

Example 7 shows construction of strains derived from *S*. *typhi* and *S. paratyphi* A to display regulated lysis for the delivery of either DNA vaccine vectors or protective antigens to humans.

The construction of host-vector systems for vaccination of humans would preferably use host-adapted invasive strains of *Salmonella* such as either *S*. *paratyphi A* or *S. typhi.* We would use the *S. paratyphi* A strain Chi8387, which is derived from ATCC 9281 by curing of a low molecular weight ColE1-like plasmid that interferes with replication of cloning vectors with pBR *ori* or pUC *ori.* The S. *typhi* strains would either be Chi8438, an RpoS⁺ derivative of *S. typhi* Ty2, or the S. *typhi* ISP1820 strain Chi3744 which also has the RpoS⁺ phenotype. We have previously shown that the RpoS⁺ phenotype is important for initial colonization of the GALT and is therefore necessary for high immunogenicity. These wild-type human-virulent *Salmonella* strains can be genetically modified in the same manner using the methods described in Example 1 and used for the construction of Chi8858 (Example 5) and further gene modifications as described in Example 6. One exception is the absence of deleting genes for arabinose breakdown in *S. typhi* since it lacks these genes but it may still be necessary to delete the *araE* gene.

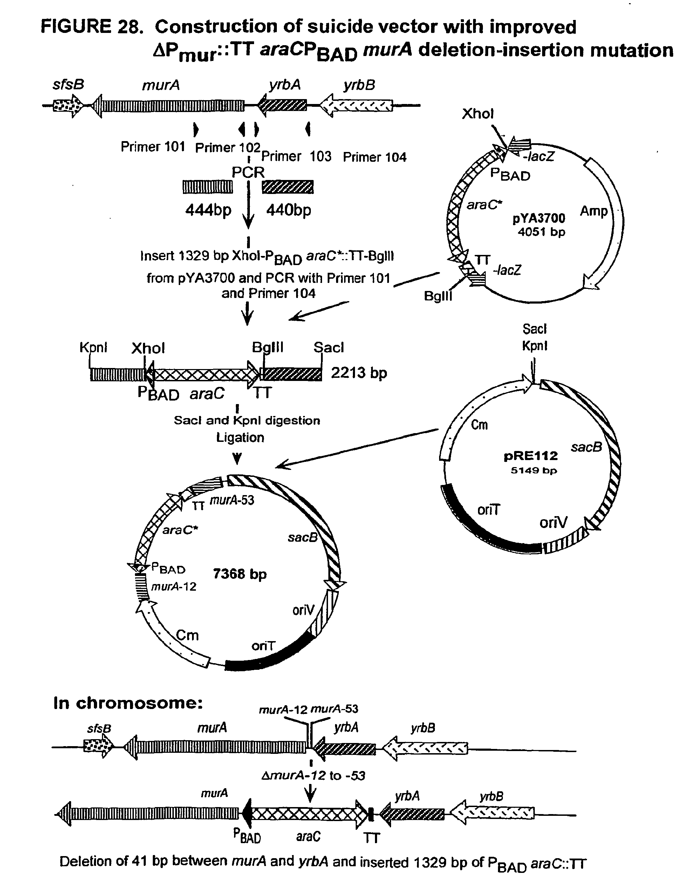

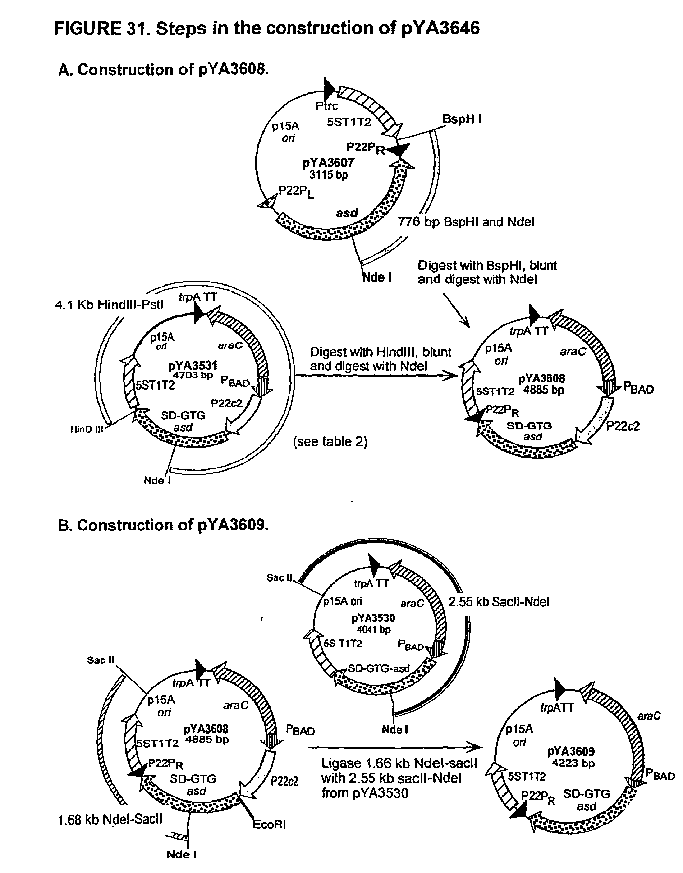

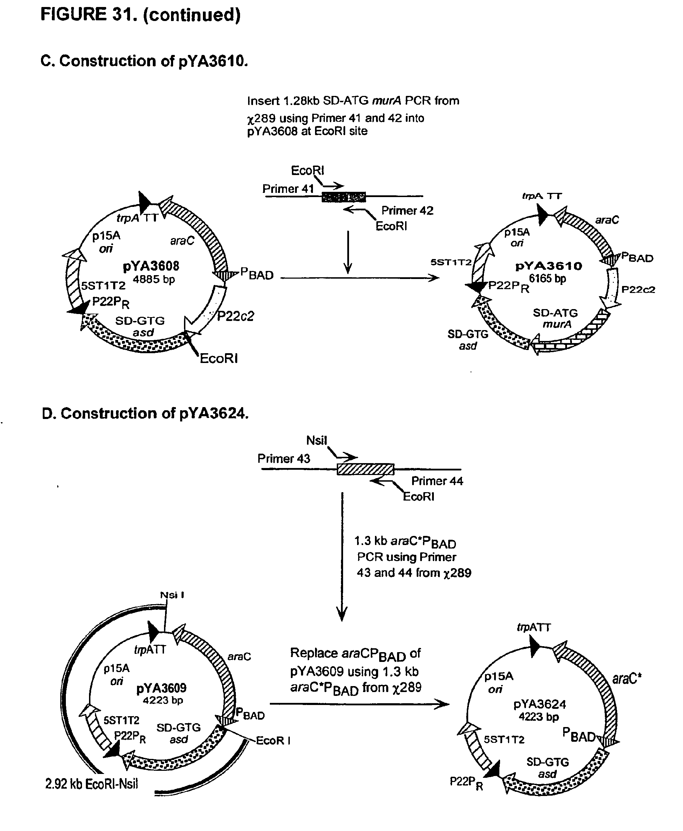

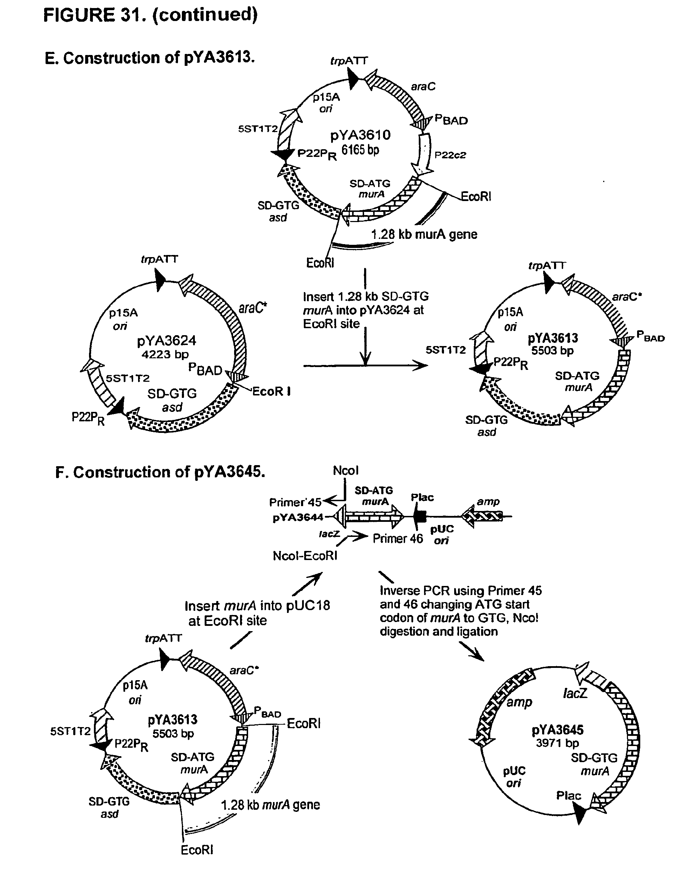

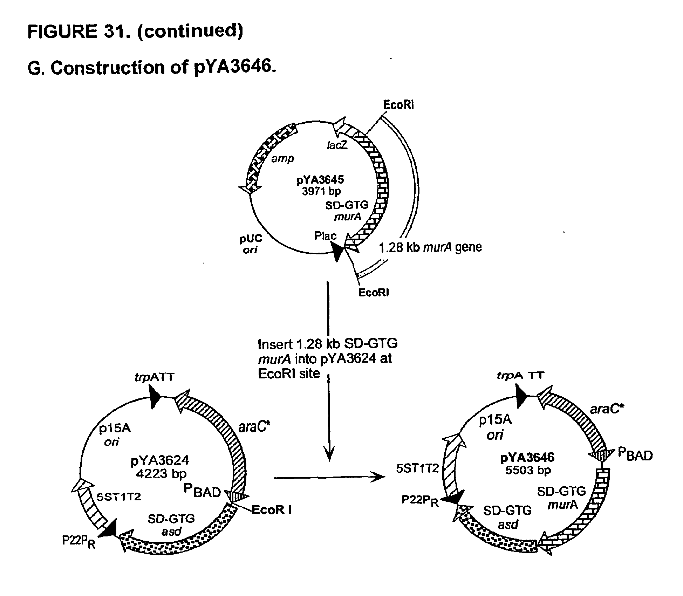

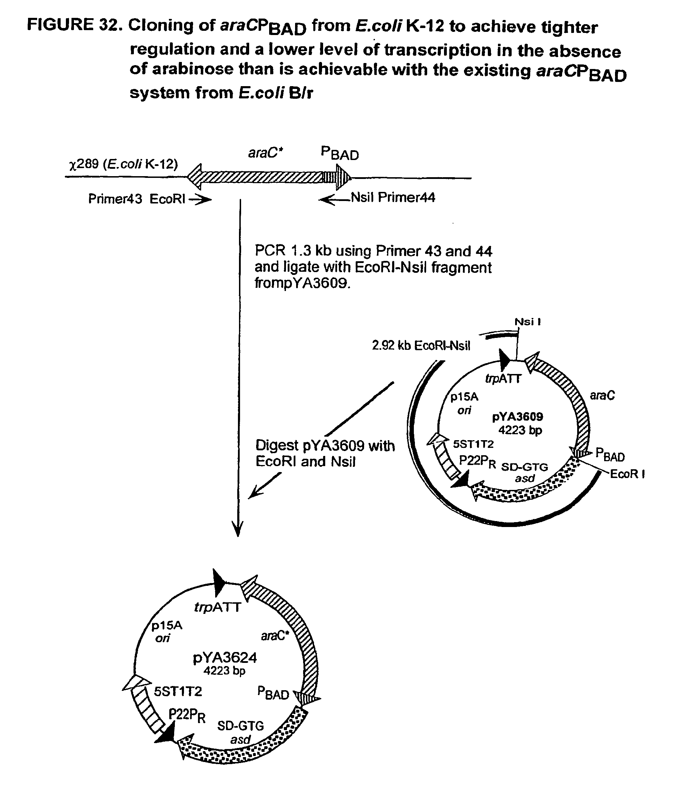

As already described in Examples 2 and 4, we designed Deltaasd and Deltaasd::TT *araC* P_{BAD} c2 deletion and deletion-insertion mutations (Figure 4 k) that have flanking nucleotide sequences adjacent to the *asd* gene that are unique to *S. typhi* and *S. paratyphi* A. In addition, we designed an improved DeltaP_{murA}::TT *araC* P_{BAD} *murA* deletion-insertion mutation. (The DeltaP_{murA7}::TTaraC_{BAD}murA deletion-insertion mutation used in Chi8888 deletes most of the *ybrA* gene that is adjacent and upstream of the *murA* gene. Even though the function of the *ybrA* gene is unknown, it would be prudent to not delete it or interfere with its unknown function.) Furthermore, the *E. coli* B/r *araC* P_{BAD} activator-promoter is somewhat leaky as noted in Example 16. We, therefore, identified a more stringently regulated *araC* P_{BAD} activator-promoter (see Example 11 and Figures 31D and 32) that could be used to substitute for the P_{murA} in the chromosome of constructed *S. typhi* and *S. paratyphi* A strains. Figure 28 depicts this new DeltaP_{murA}::TT *araC* P_{BAD} *murA* construction, in which only 40bp of sequence between the *ybrA* and *murA* genes are deleted.

**Example 8**

The deletion and deletion-insertion mutants (e.g. Chi8888 or its derivatives as identified and described in Examples 2-7) and the plasmid vectors described in the following Examples together can impart, a regulated delayed lysis phenotype which can be used to provide attenuation and biological containment properties to constructed derivatives of numerous bacterial pathogens, such as strains of *Yersinia,* (e.g. *Y. pestis),* strains of *Shigella,* (e.g. *S. flexneri),* strains of *E. coli,* strains of *Francisella,* (e.g *F. tularensis),* strains of *Listeria,* (e.g. *L. monocytogenes),* and other bacterial strains that are naturally invasive or genetically modified to be invasive in an immunized individual. Table 1 lists strains of bacteria that can be used for these constructions. Occasionally, the constructions need to be modified to accommodate differences in nucleotide sequences, which a person of ordinary skill in the art can readily perform. These same bacterial strains can be designed to serve as hosts for the delivery of antigens synthesized by the host-vector strain, for delivery of a genetic vaccine to specify synthesis of antigens by the immunized individual, or for delivery of attenuated viral genomes as an anti-virus vaccine.

**Example 9**

Example 9 shows the steps toward eventual construction of plasmids to enable regulated lysis of bacterial strains for DNA vaccine delivery and/or protective antigen release. As stated in the introduction, the plasmid vectors pYA3450 (Figure 1, Table 2), pYA3530 (Figure 2A, Table 2) and pYA3531 (Figure 2B, Table 2) when introduced into a *S. typhimurium* strain such as Chi8276 with the Delta*asdA16* mutation (Table 1) did not result in arabinose-dependent growth when the recombinant strains were inoculated into various media without arabinose and did not exhibit cell wall-less death and lysis. Furthermore, Chi8276 with these plasmids still retained virulence for orally inoculated BALB/c mice. There was, therefore, no attenuation and no biological containment in the Chi8276 (pYA3450) or Chi8276 (pYA3530) or Chi8276 (pYA3531) strains. We initiated studies in an attempt to succeed in achieving the elusive goal to create a stable, safe and efficacious DNA vaccine vector or antigen delivery vector that would exhibit regulated lysis *in vivo* after colonizing lymphoid tissues in immunized animal or human hosts.

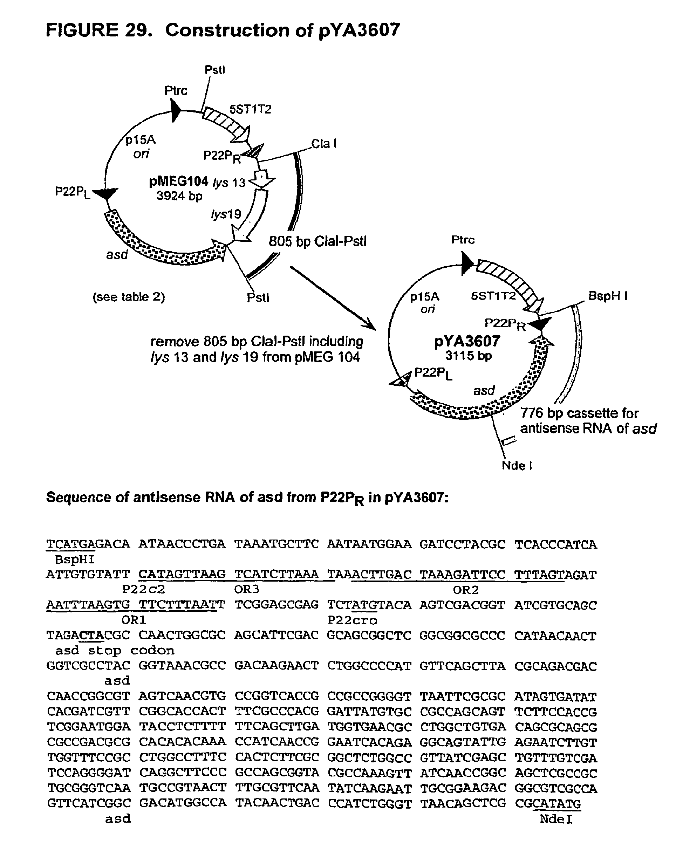

To construct pYA3607, isolated plasmid pMEG-104 (Figure 29, Table 2) was digested with *Cla*I and *Pst*I to remove an 805 bp sequence encoding the P22 phage lysis genes followed by blunt-end religation of the plasmid to yield pYA3607 (Figure 29, Table 2). pYA3607 was later used as a source of the DNA fragment with the P22 P_{R} at the C-terminal end of the *asd* gene all on a 776 bp DNA fragment to enable synthesis of anti-sense *asd* mRNA. The sequence of the DNA encoding this anti-sense RNA is also given in Figure 29.

**Example 10**

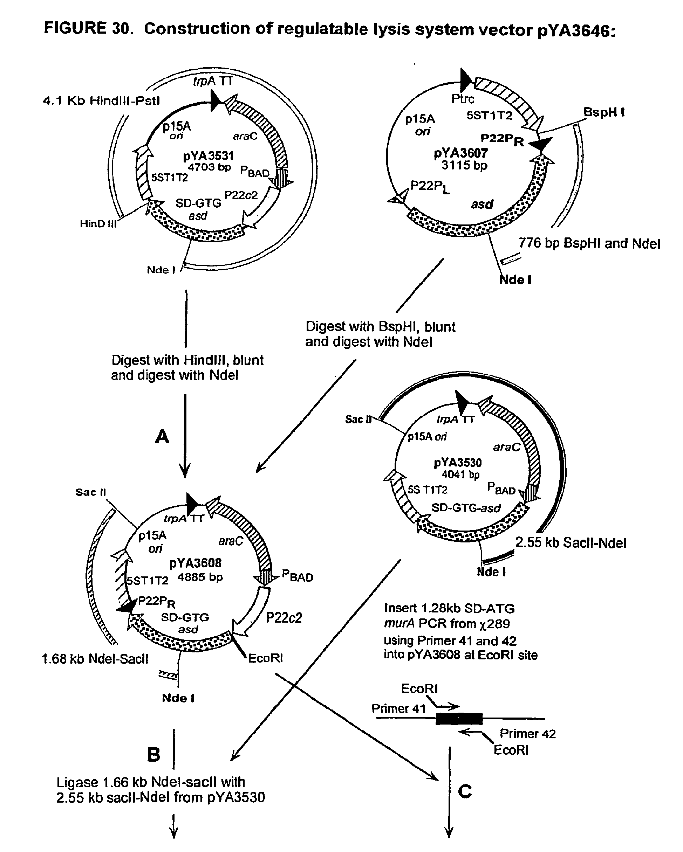

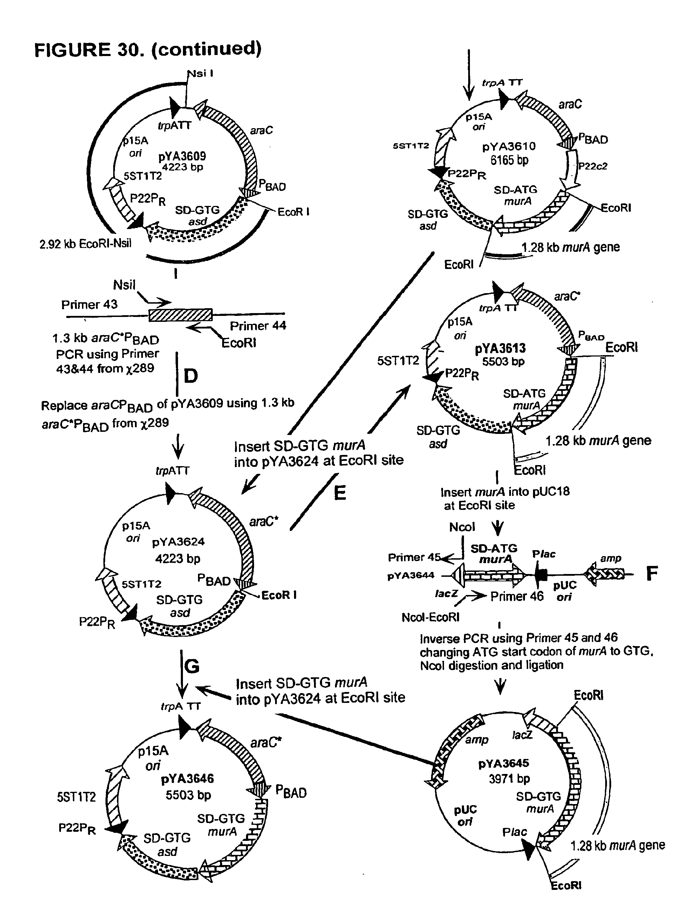

Example 10 shows construction of pYA3646, which allows the *S*. *typhimurium* cells to exhibit arabinose-dependent growth and to lyse after several cell divisions in the absence of arabinose. Figure 30 diagrams a flow chart of the construction steps leading to pYA3646 that conferred the desired phenotype on *S. typhimurium.* The individual steps to achieve this final construction of pYA3646 are diagramed in Figure 31. In Figure 31A, the 776 bp *Bsp*HI (blunt-ended) to *Nde*I fragment from pYA3 607 (Figure 29) was inserted into pYA3531 (Figure 2B) which had been digested with *Hin*dIII, blunt-ended, and then digested with *Nde*I to enable ligation of the DNA fragment containing the P22 P_{R} and the C-terminal portion of the *asd* gene. This yielded plasmid pYA3608 (Figure 31A). This construction added the P22 P_{R} with an appropriate juxtaposition to cause synthesis of anti-sense *asd* mRNA.

To eliminate the P22 *c2* gene fragment in pYA3608 (Figure 31A), pYA3608 was digested with *Sac*II and *Nde*I and ligating the 1.68 kb fragment generated with a larger 2.55 kb fragment of DNA from pYA3530 (Figure 2A) to yield pYA3609 (Figure 31B).

**Example 11**

Example 11 shows construction of a system with regulation of two genes encoding enzymes for essential constituents of the rigid layer of the bacterial cell wall. We had already determined that the DeltaP_{murA7}::*araC* P_{BAD} *murA* mutation (Figure 4 v) in the chromosome caused *S*. *typhimurium* to exhibit arabinose-dependant growth. We introduced a copy of the *murA* gene into the plasmid such that its expression and that of the *asd* gene would be under the control of the *araC* P_{BAD} activator-promoter. We therefore used PCR to amplify a 1.28 kb DNA fragment with the *murA* gene open reading frame and its Shine-Dalgarno sequence and added *Eco*RI sites to enable ligation into an *Eco*RI site at the beginning of the *SD-asd* sequence in pYA3608 (Figure 31C). This yielded pYA3610 (Figure 31C).

The *araC* P_{BAD} activator-promoter on the plasmids permits a low basal level of transcription in the absence of arabinose. (Guzman *et al.,* J. Bacteriol. 177:4121-4130 (1995)). This sequence is derived from *E. coli* B/r. We used computer search analyses to examine sequences of many *araC* P_{BAD} activator-promoters in various gram-negative bacteria and used PCR to amplify the sequence selected from *E. coli* K-12 strain Chi289 (Table 1). This is diagrammed in Figures 31D and 32. In this construction, pYA3609 was digested with *Nsi*I and *Eco*RI and a 2.92 kb DNA fragment was ligated to a PCR amplified 1.3 kb DNA fragment, which contains the *E. coli* K-12 *araC* P_{BAD} activator-promoter, which was also digested with *Nsi*I and *Eco*RI*.* This generated pYA3624.

Figure 31E shows the construction of a plasmid containing *araC* P_{BAD} activator-promoter from *E. coli* K-12 and a *murA* gene. In this manipulation, a 1.28 kb DNA fragment containing the *E. coli murA* gene with its SD sequence was excised from pYA3610 by digestion with *Eco*RI*.* This fragment was then ligated into *Eco*RI digested pYA3624 to yield pYA3613 (Figure 31E).

We further synthesized and evaluated various combinations of *asd* and *murA* genes with ATG, GTG and TTG start codons. Little or no difference was observed with either TTG or GTG start codons. Both enabled us to construct plasmids that conferred arabinose-dependant growth and strains that could undergo a suitable number of cell divisions in media without arabinose. Figure 31F shows the construction of the *murA* gene with a GTG start codon, resulting in pYA3645.

Figure 31 depicts a construction of a plasmid with several desirable attributes. In this construction, the 1.28 kb SD-GTG *murA* sequence is used to insert into the *Eco*RI site on pYA3624 (Figure 31D) to yield pYA3646. pYA3646 possess the regulated *E. coli* K-12 *araC* P_{BAD} activator-promoter, the SD-GTG *murA* genes, the SD-GTG *asd* gene, and the P22 P_{R}. pYA3646 serves as one means to construct a DNA vaccine vector and another vector for the expression of protective antigens to be delivered by or synthesized by, respectively, *Salmonella* or other suitable bacteria such as strains of *Yersinia. Shigella, Escherichia, Listeria* or other natural or constructed invasive species and released by regulated lysis *in vivo.* One skilled in the art would readily appreciate that pYA3646 (Figure 31 G) could be constructed using any number of standard molecular biology methods and in any order.

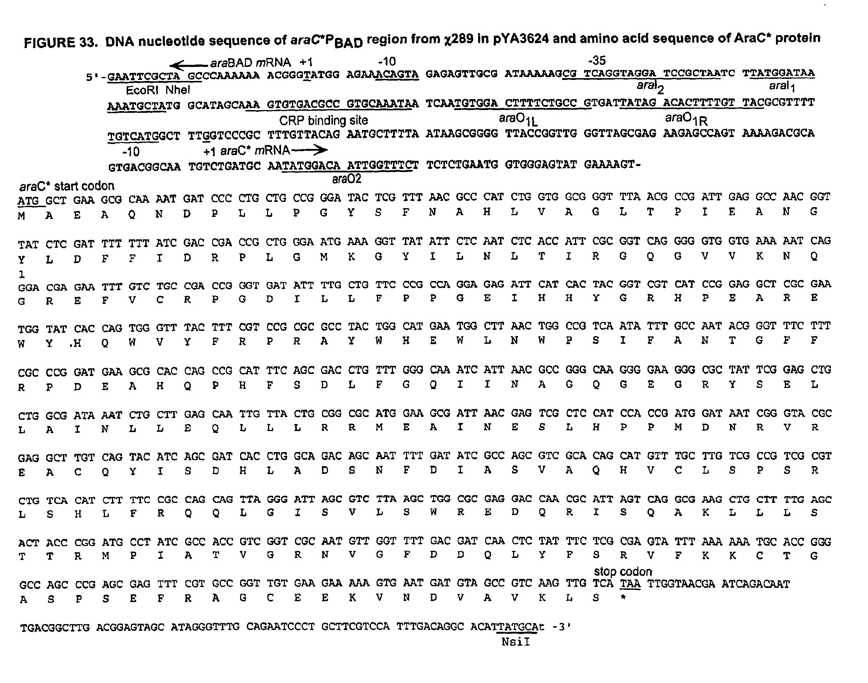

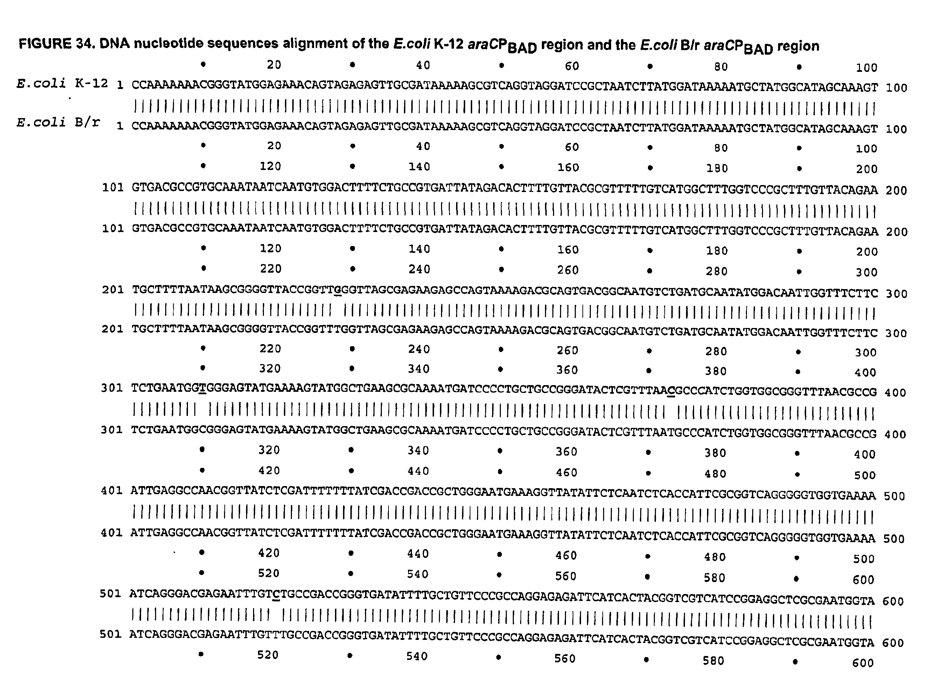

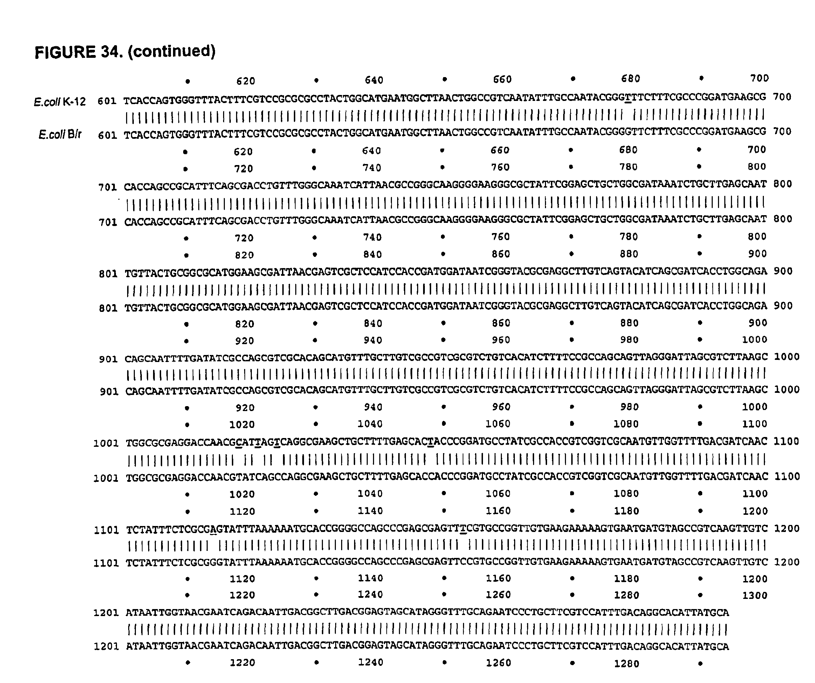

Figure 33 provides the nucleotide sequence of the *E*. *coli* K-12 *araC* P_{BAD} activator-promoter. The regulatory domains are identified for regulating both the P_{BAD} and *araC* promoters. The *araC* P_{BAD} activator-promoter is also subject to catabolite repression control such that in the presence of glucose *in vivo,* for example, there is insufficient cAMP to bind to the Crp protein to activate transcription from P_{BAD}. Thus *in vivo,* regulation of the *araC* P_{BAD} activator-promoter is stringent with a decreased likelihood of transcription of the *murA* and *asd* vector genes (as well as of genes under the control of *araC* P_{BAD} inserted into the chromosome such as in the Delta*asdA19::TT araC* P_{BAD} c2, DeltaP_{murA7}::araC P_{BAD} *murA* and Delta*araBAD23::c2*/*lacI* deletion-insertion mutations). Figure 33 also presents the amino acid sequence of the AraC gene product. Figure 34 presents the comparative nucleotide sequences for the *E. coli* B/r and *E. coli* K-12 *araC* P_{BAD} sequences.

**Example 12**

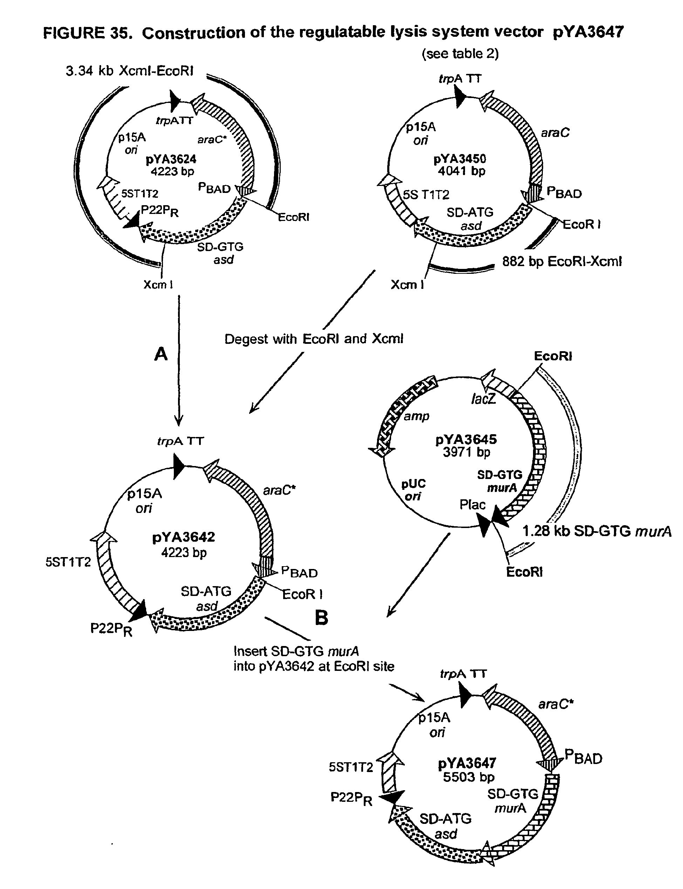

Example 12 shows construction of pYA3647. To enhance regulated lysis, we used a regulated *araC* P_{BAD} activator-promoter, the synthesis of anti-sense mRNA and alteration in the start codons for the *murA* and *asd* genes from ATG to GTG. Since the *asd* gene is distal from the P_{BAD} promoter (the *murA* gene is proximal), it is likely transcribed less well than the *murA* gene. The *asd* gene is also proximal to the P22 P_{R-} Vectors with an SD-ATG *asd* provides similar results in regulated lysis experiments as in animals. Figure 35 depicts the construction of pYA3647. The N-terminal SD-ATG *asd* gene fragment was cloned as a 882 bp *EcoR*I to *Xcm*I fragment from pYA3450 (Figure 1) and inserted in place of a similar fragment with the N-terminal SD-GTG *asd* sequence in pYA3624 (Figures 30 and 31D) cut with the same two restriction enzymes. The resulting plasmid pYA3642 possesses the improved *araC* P_{BAD} activator-promoter regulating expression of the SD-ATG *asd* gene. We next inserted the SD-GTG *murA* sequence by digesting pYA3645 (Figure 31 F) with *Eco*RI and cloning the fragment into *Eco*RI digested pYA3642. Recombinant clones were isolated in *E. coli* Chi6212 selecting for DAP-independent isolates. DNA sequencing was used to verify the correct orientation of the *murA* gene, and pYA3647 was stocked as having *araC* P_{BAD} SD-GTG *murA* SD-ATG *asd.*

**Example 13**

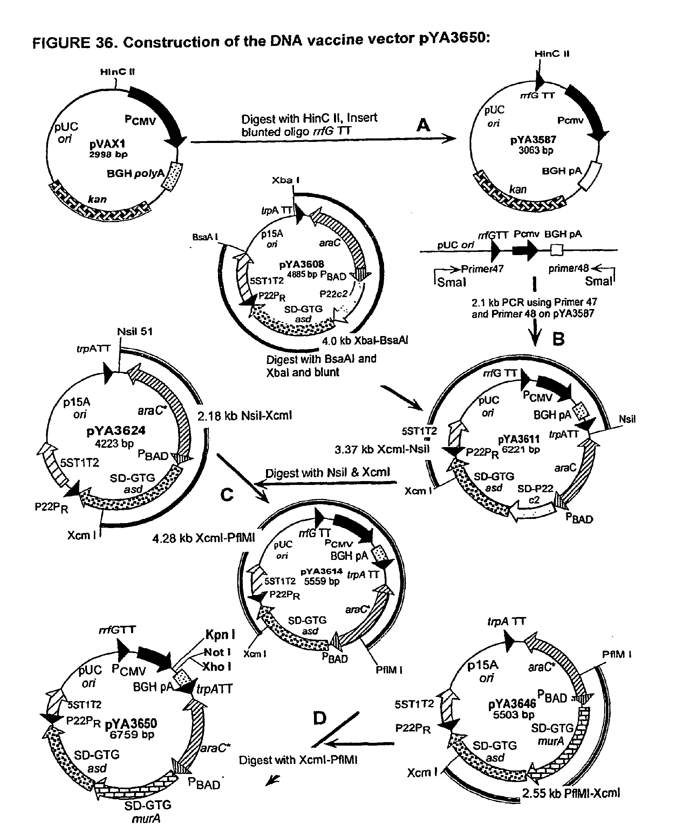

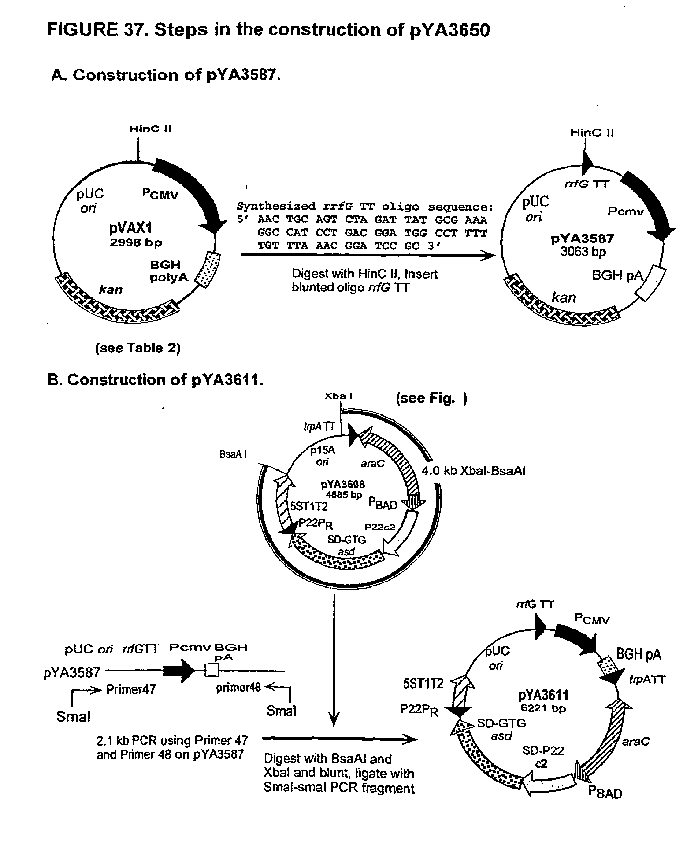

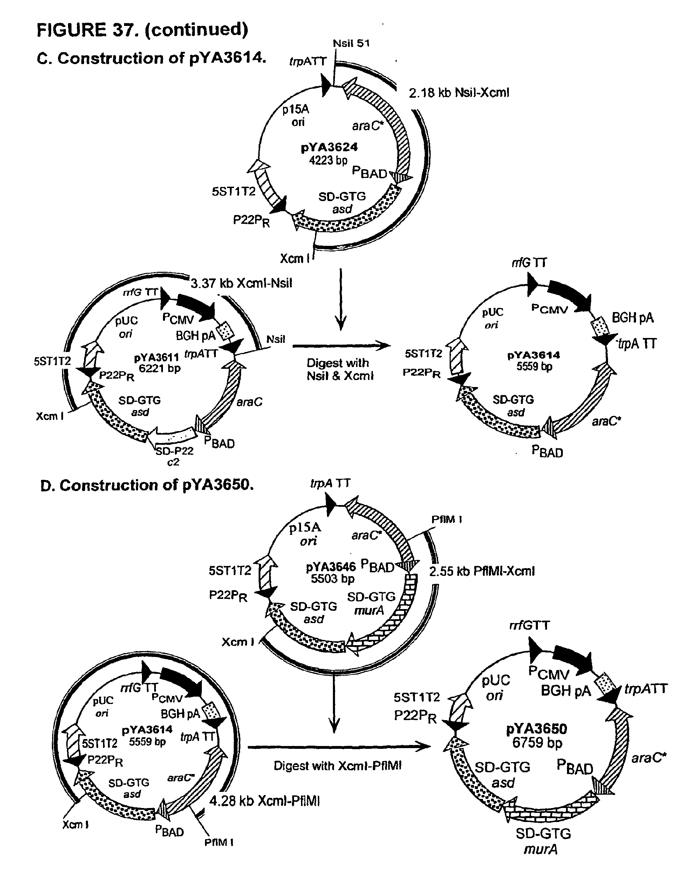

Example 13 shows construction of pYA3650. The DNA vaccine vector pYA3650 (Figure 5A) was constructed by a series of steps that are diagramed in Figure 36. Each of the steps are described based on the diagrams in Figure 37. As described earlier, it is desirable to use transcription termination sequences to preclude interference of gene expression in the prokaryotic controlled expression domain on gene information in the eukaryotic expression domain and to have neither of these expression domains influenced by activities associated with replication of the plasmid vector. For these reasons, transcription terminators are desired between each of the three domains of the final plasmid constructs. As diagrammed in Figure 37A, the commercially-available DNA vaccine vector pVAX1 was modified by insertion of a synthesized oligonucleotide sequence to represent the *E. coli* ribosomal gene *rrfG* transcriptional terminator (TT) at the *Hinc*II site 5' to the Cytomegalovirus (CMV) enhancer-promoter. The *rrfG* oligonucleotides were annealed and blunt-ended with T4 DNA polymerase for blunt-end ligation into the pVAX1 *Hinc*II site. The resulting plasmid was designated pYA3587.

To establish a balanced-lethal host-vector system, the drug-resistance marker present in pVAX1 was replaced with a regulatable *araC* P_{BAD} *asd* cassette (see Figure 37B). The 2.1 kb DNA fragment containing the eukaryotic DNA expression cassette was PCR-amplified from the pYA3587 (Figure 37A) DNA template with a pair of primers (Primer 47: 5'CGACCCGGGATCGATCTGTGCGGTATTTCACACCG 3'and Primer 48: 5'GCACCCGGGTCGACAGATCCTTGGCGGCGAGAAAG 3'). (See Table 3). The PCR product, digested with *Sma*I enzyme, was ligated with the 4.0 kb blunted *Xba*I*-Bsa*AI fragment from pYA3608 (Figure 30 and 31A), a plasmid possessing an SD-GTG *asd* and the *araC* P_{BAD} fragment from *Escherichia coli* B/r, to result in plasmid pYA3611.

In the absence of the inducer arabinose, AraC is capable of activating P_{BAD} transcription to about 1% of the induced level when using the *E. coli* B/r *araC* P_{BAD} sequences. The transcriptional level of *asd* under the regulation of the B/r P_{BAD} in pYA3611 without inducer resulted in sufficient Asd to permit construction of balanced-lethal systems with strains such as Chi8289 (Table 1) which could grow in the absence of either arabinose or DAP. A new *araC* P_{BAD} fragment from *E. coli* K-12 strain Chi289 that is present in pYA3624 (Figures 30 and 31D) was introduced into pYA3611 to decrease the leakiness of the *araC* P_{BAD} system in the absence of arabinose. Also, to further decrease expression of the *asd* gene by anti-sense mRNA under the regulation of P22 P_{R}, the P22 c2 gene encoding the repressor of P22 P_{R}, was removed from pYA3611. pYA3611 and pYA3624 (see Figure 37C) were each digested with *Xcm*I and *Nsi*I followed by ligation of the 3.37 kb *Xcm*I*-Nsi*I pYA3611 and 2.18 kb *Xcm*I*-Nsi*I pYA3624 fragments, generating plasmid pYA3614.

We introduced the *murA* sequence derived from *E. coli* K-12 strain Chi289 into pYA3614 to enhance the arabinose-dependent growth attributes of strains with the plasmid vector. As diagrammed in Figure 37D, pYA3614 and pYA3646 were digested with *Xcm*I and *Pfl*MI*,* and then the 4.28 kb *Xcm*I*-Pfl*MI fragment from pYA3614 was ligated with the 2.55 kb *Pfl*MI*-Xcm*I fragment from pYA3646 (Figures 30 and 31G), resulting in the DNA vaccine vector pYA3650 (Figures 5A and 37D).

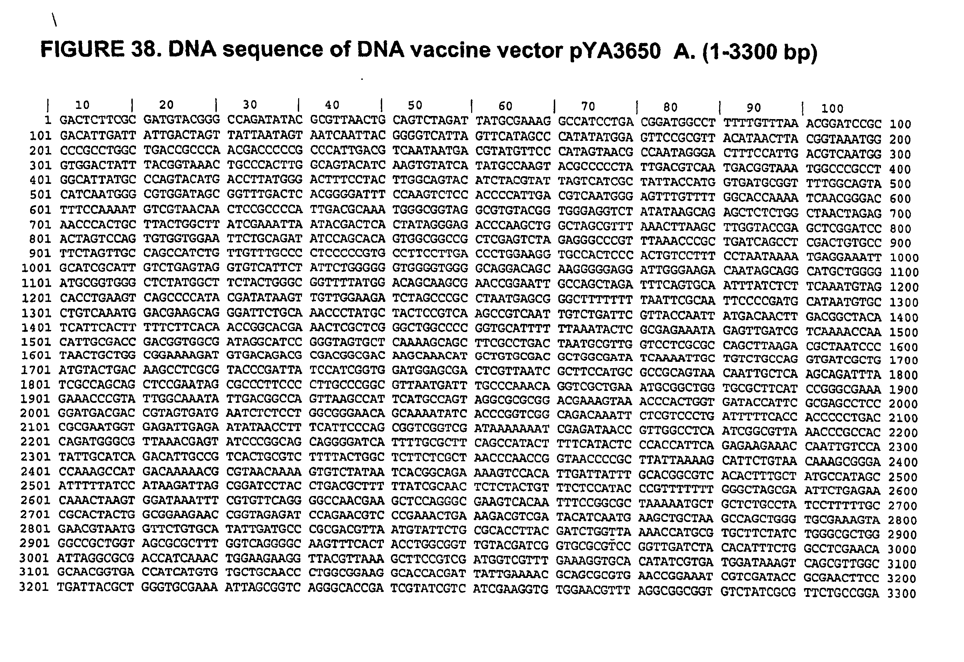

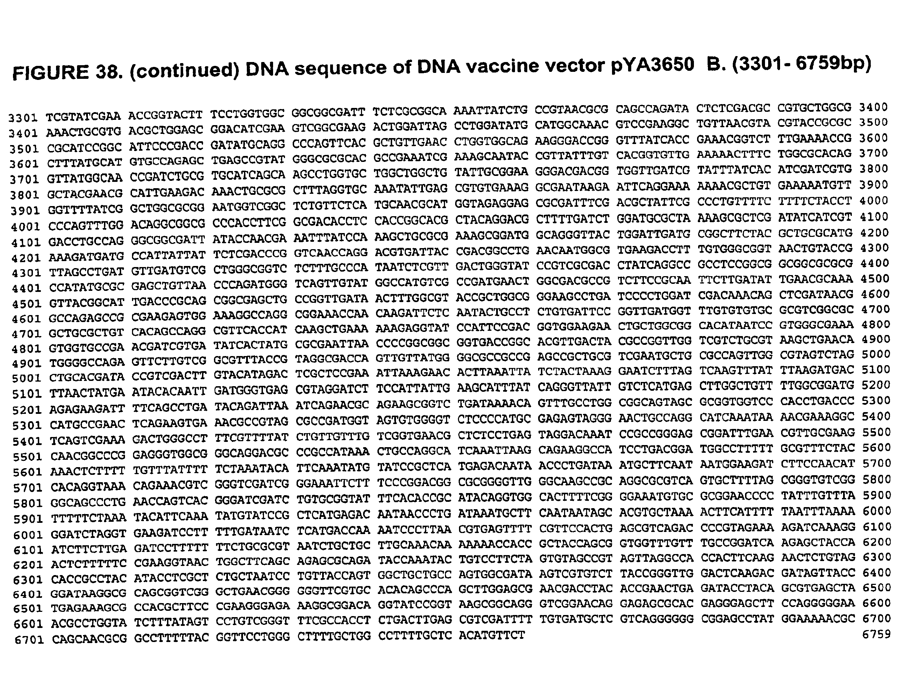

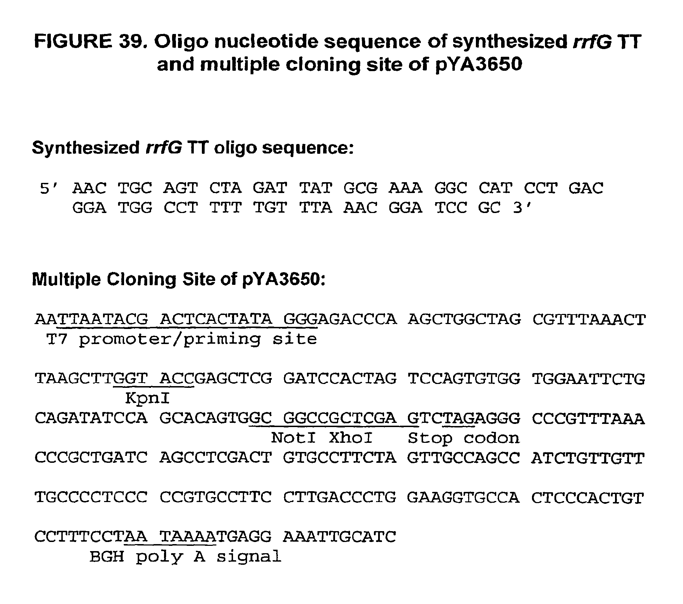

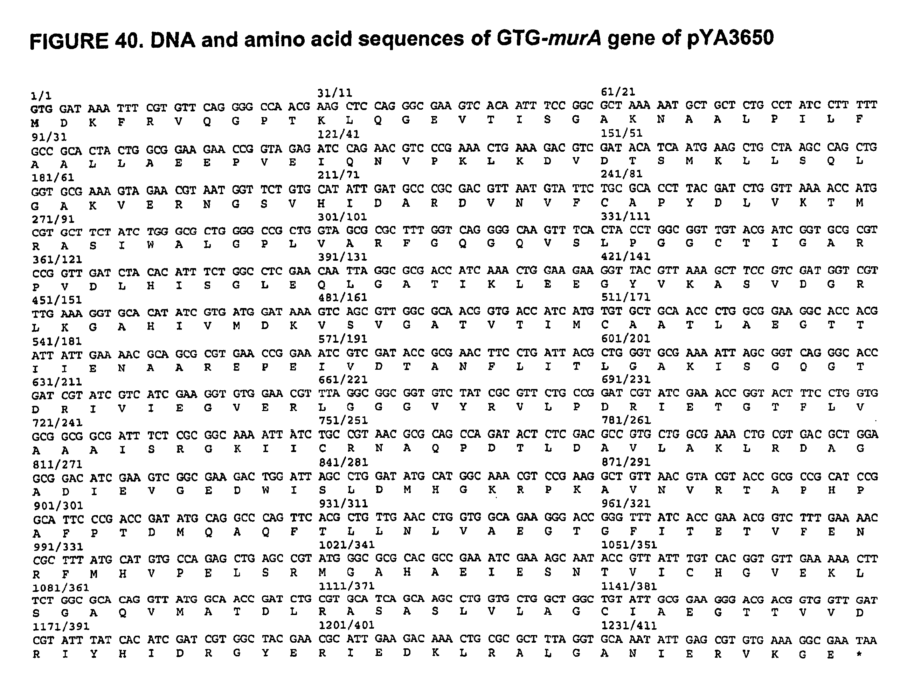

Figure 38 presents the nucleotide sequence of pYA3650. Figure 39 presents the sequence of the synthetic *rrfG* TT sequence and the sequence of the multiple cloning sites within the eukaryotic expression cassette. This sequence also contains the recognition sequence for T7 RNA polymerase that enables synthesis of an mRNA transcript in bacteria engineered to express T7 RNA polymerase. This leads to translation of the mRNA to synthesize the protein encoded within the eukaryotic expression cassette. This feature allows synthesis of the desired protein and provides a means to synthesize that protein in bacteria to use in assays for immune responses or as an antigen to induce antibodies against that protein. Figure 40 presents the nucleotide and amino acid sequences of the pYA3650 GTG*-murA* gene and gene product, respectively. Figure 41 presents the nucleotide and amino acid sequence of the pYA3650 GTG-*asd* gene and gene product, respectively.

**Example 14**

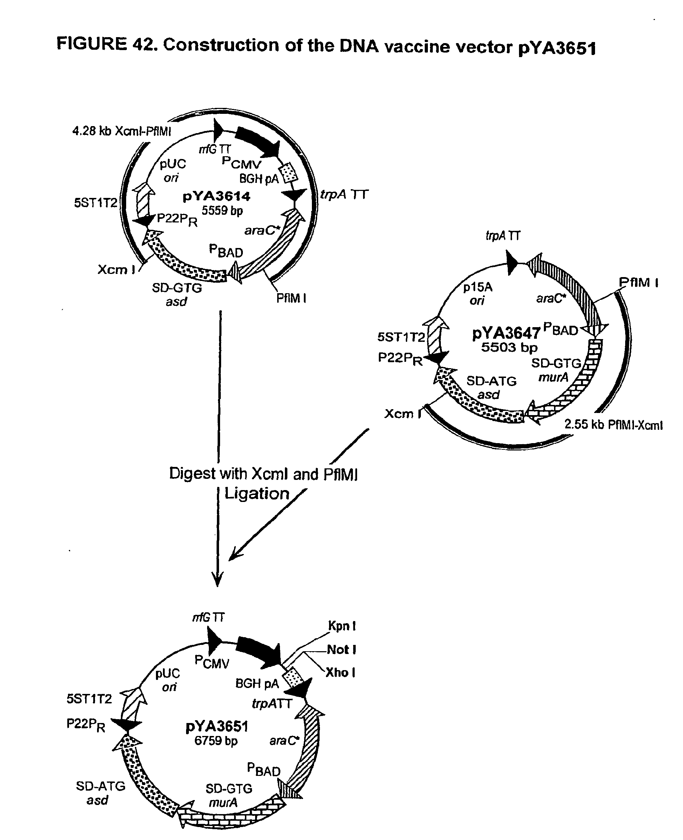

Example 14 shows construction of pYA3651. To generate a DNA vaccine vector with the SD-ATG *asd* sequence, pYA3614 (Figure 36 and 37C) and pYA3647 (Figure 35) were both digested as diagrammed in Figure 42 with the restriction enzymes *Xcm*I and *Pfl*MI and the 4.28 kb fragment from pYA3614 and the 2.55 kb DNA fragment from pYA3647 were ligated to each other. The recombinant plasmids were electroporated into Chi6212 (Table 1) and DAP-independent isolates selected. Plasmids from these isolates were analyzed for size, the high copy number associated with the pUC *ori* and for restriction cleavage sites unique to each desired fragment. The resulting plasmid was designated pYA3651 (Figures 5B and 42).

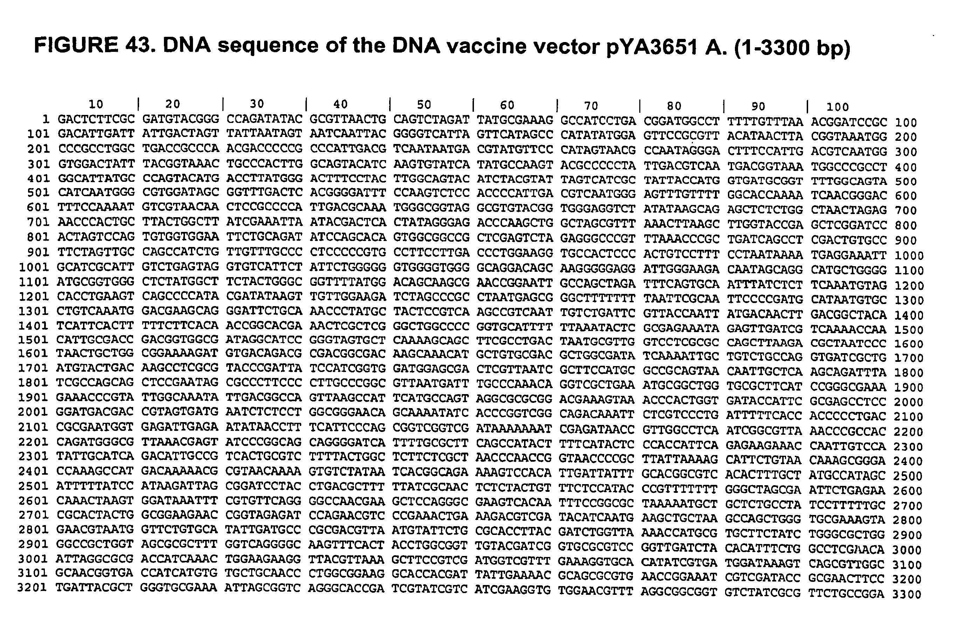

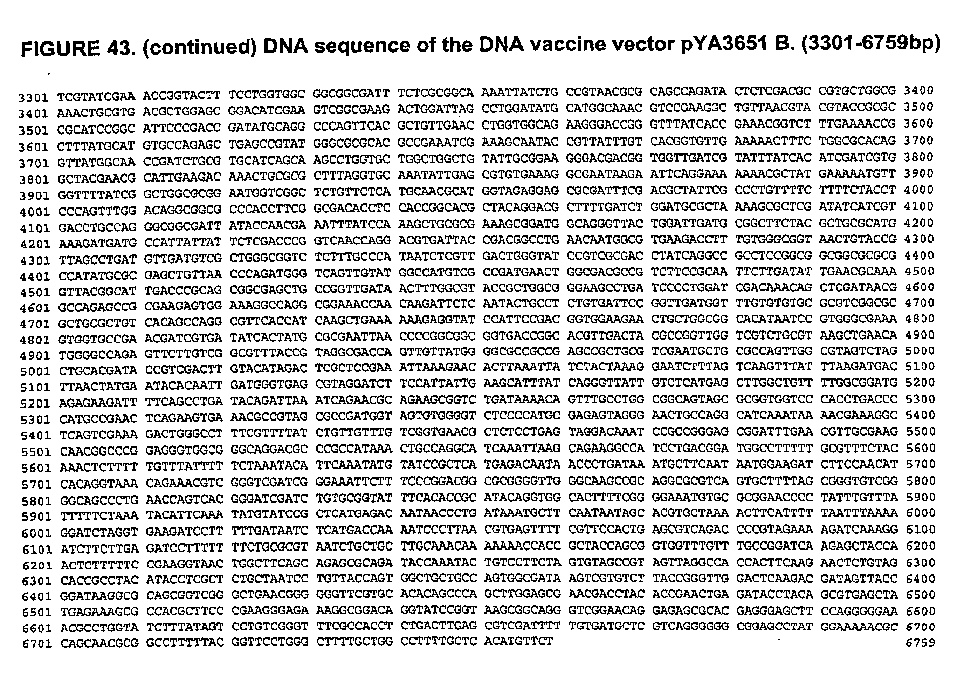

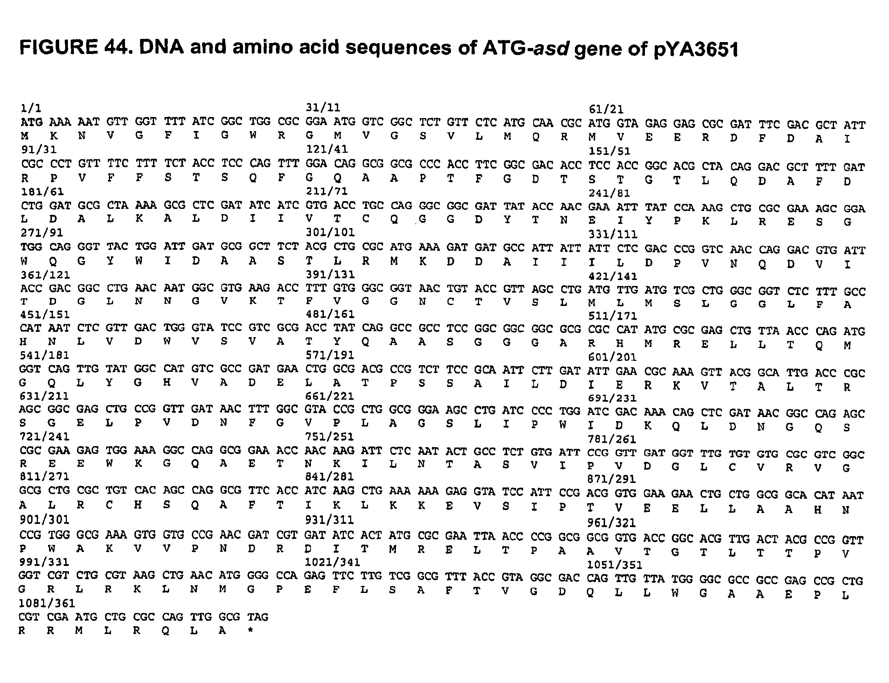

Figure 43 presents the nucleotide sequence of pYA3651. Figure 44 presents the nucleotide and amino acid sequences of the pYA3651 ATG-*asd* gene and gene product, respectively.

CpG sequences (Krieg, Annu. Rev. Immunol. 20:709-760 (2002)) can enhance the immunogenicity of DNA that are present in bacterial but not in mammalian DNA. These immune enhancing unmethylated CpG sequences act like adjuvants. The DNA vaccine vectors pYA3650 and pYA3651 have a higher number (15) of such sequences to stimulate immune responses in both mice and humans than commercially-available vectors such as pVAX1. Table 6 lists the 15 immune -enhancing CpG sequences in pYA3650 and pYA3651.

**Table 6. Presence of multiple immune enhancing CpG motifs in pYA3650 and pYA3651**

| Sequence* | Location | Description |
|---|---|---|
| 5' GTCGTT 3' | 3054-3059 | Direct pattern hits, exist in *murA* gene |
| 5' GTCGTT 3' | 220-225 | Antiparallel pattern hits, exist in P_{CMV} |
| 5' GTCGTT 3' | 6461-6466 | Antiparallel pattern hits, pUC *ori* |
| 5' GACGGT 3' | 2834-2839 | Direct pattern hits, exist in *murA* gene |
| 5' GACGGT 3' | 2735-2740 | Antiparallel pattern hits, exist in *mur*A gene |
| 5' GACGGT 3' | 3468-3473 | Antiparallel pattern hits, exist in *mur*A gene |
| 5' GACGGT 3' | 5715-5720 | Antiparallel pattern hits, between 5ST1T2 and *ori* |
| 5' GACGTC 3' | 237-242 | Direct pattern hits, exist in P_{CMV} |
| 5' GACGTC 3' | 290-295 | Direct pattern hits, exist in P_{CMV} |
| 5' GACGTC 3' | 373-378 | Direct pattern hits, exist in P_{CMV} |
| 5' GACGTC 3' | 559-564 | Direct pattern hits, exist in P_{CMV} |
| 5' GACGTC 3' | 2753-2758 | Direct pattern hits, exist in *araC* gene |
| 5' AACGTT 3' | 3264-3269 | Direct pattern hits, exist in *mur*A gene |
| 5' AACGTT 3' | 5489-5494 | Direct pattern hits, exist in 5ST1T2 |
| 5' AGCGCT 3' | 4083-4088 | Direct pattern hits, exist in *asd* gene |

| | | |
|---|---|---|
| * GTCGTT motif is optimal for stimulation of lymphocyte proliferation in several species, including cattle, sheep, goats, horses, pigs, dogs, cats and chickens. | | |

**Example 15**

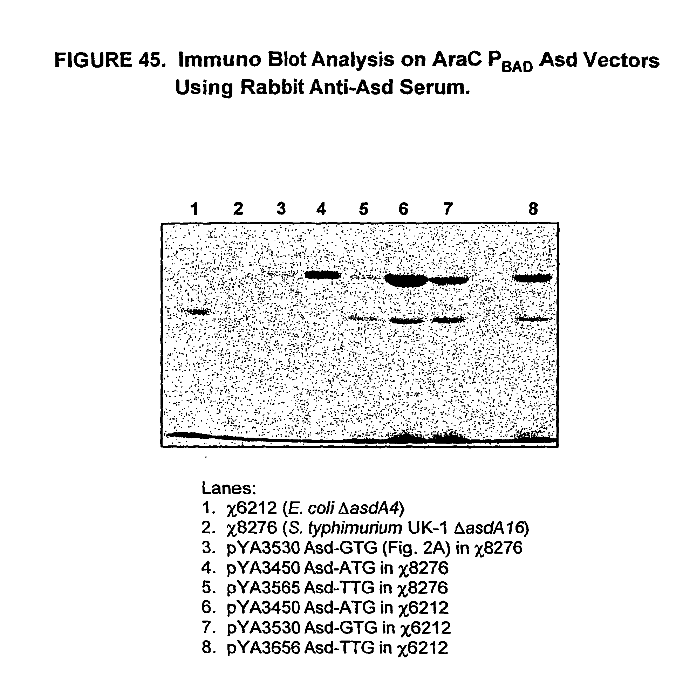

Example 15 shows the influence of ATG, GTG, and TTG start codons in the *asd* gene on synthesis of Asd protein in *S*. *typhimurium* and *E. coli* K-12. Figure 45 presents a western blot of electrophoretically separated proteins specified by the *asd* gene in pYA3450 (Figure 1) with the original ATG start sequence and GTG and TTG start codons generated by site directed mutagenesis to yield pYA3530 (Figure 2A) and pYA3565 (Table 2), respectively. Both the *S*. *typhimurium* host strain Chi8276 with the Delta*asdA16* mutation and the *E. coli* K-12 strain Chi6212 with the Delta*asdA4* mutation were used. The Asd protein was detected using rabbit anti-Asd antibodies prepared as described above. There was more Asd protein made in either *E*. *coli* or *S*. *typhimurium* when the plasmid *asd* gene had the ATG start codon (Figure 45). The amounts of protein synthesized when the start codon was GTG are only slightly more than when the *asd* gene had the TTG start codon. *E*. *coli* strains synthesized more Asd enzyme than *S*. *typhimurium* strains.

**Example 16**

Example 16 shows phenotypic properties of recombinant host-vector strains displaying arabinose-dependant growth and regulated cell lysis in the absence of arabinose. As a routine procedure, bacterial strains with or without plasmids were grown overnight at 37° C on a roller drum in LB broth supplemented with 0.1% arabinose and with 50 microg DAP/ml, if necessary. These cultures were diluted 1:1000 in BSG and plated on Minimal agar supplemented with lysine, threonine and methionine (to satisfy the nutritional requirements, other than for DAP, due to the *asd* mutation) and 0.4 % glycerol which were supplemented with (a) 50 microg DAP/ml plus 0.1 % arabinose, (b) 0.1 % arabinose with no DAP, (c) 50 microg DAP/ml with no arabinose, and (d) without DAP and arabinose but with 0.1 % glucose. Plates were incubated at 37°C for up to four days, with growth or no growth usually recorded after 48 h of incubation. Results of these tests are presented in Table 7.

**Table 7. Table 7. DAP-less and Muramic-less Death in Chi8854 with DNA Vaccine Vectors and Lysis Vectors***

| | **Medium** | | | |
|---|---|---|---|---|
| **Strain** | (a) Minimal agar (Lys, Met, Thr) 0.4% (v/v) glycerol 50ug/ml DAP 0.1% Arab | (b) Minimal agar (Lys, Met, Thr) 0.4% (v/v) glycerol 0.1% Arab without DAP | (c) Minimal agar (Lys, Met, Thr) 0.4% (v/v) glycerol 50ug/ml DAP without Arab | (d) Minimal agar (Lys, Met, Thr) 0.4% (v/v) glycerol 0.1% glucose without Arab without DAP |
| Chi8844 ΔendA | ++ | ++ | ++ | ++ |
| Chi8854 *DeltaaraE25* Delta*araBAD1923* Delta*endA*Delta*asdA 19*::TT *araC* P_{BAD} c2 5ST1T2 DeltaPmurA::*ara*CP_{BAD}*murA* | ++ | **-** | **-** | - |
| Plasmid in χ8854 pYA3680 p15A *- araC* P_{BAD} SD GTG-*asd* | ++ | ++ | - | - |
| pYA3624 p15A *- araC* P_{BAD} SD GTG*-asd* anti-*asd* | ++ | + | - | - |
| pYA3642 p15A *- araC* P_{BAD} SD ATG-*asd* anti*-asd* | ++ | + | - | - |

| | **Medium** | | | |
|---|---|---|---|---|
| Plasmid in Chi8854 | (a) Minimal agar (Lys, Met, Thr) 0.4% (v/v) glycerol 50ug/ml DAP 0.1% Arab | (b) Minimal agar (Lys, Met, Thr) 0.4% (v/v) glycerol 0.1% Arab without DAP | (c) Minimal agar (Lys, Met, Thr) 0.4% (v/v) glycerol 50ug/ml DAP without Arab | (d) Minimal agar (Lys, Met, Thr) 0.4% (v/v) glycerol 0.1% glucose without Arab without DAP |
| pYA3613 p15A - *araC* P_{BAD} SD-ATG *murA* SD GTG-*asd* anti-*asd* | ++ | + | +/- | - |
| pYA3646 p15A - *araC* P_{BAD} SD-GTG *murA* SD GTG-asd anti-asd | ++ | ++ | - | - |
| pYA3643 p15A - *araC* P_{BAD} SD-ATG *murA* SD ATG-*asd* anti-*asd* | ++ | + | + | - |
| pYA3647 p15A *- araC* P_{BAD} SD-GTG *murA* SD ATG-*asd* anti-*asd* | ++ | + | - | - |

| | **Medium** | | | |
|---|---|---|---|---|
| Plasmid in Chi8854 | (a) Minimal agar (Lys, Met, Thr) 0.4% (v/v) glycerol 50ug/ml DAP 0.1% Arab | (b) Minimal agar (Lys, Met, Thr) 0.4% (v/v) glycerol 0.1% Arab without DAP | (c) Minimal agar (Lys, Met, Thr) 0.4% (v/v) glycerol 50ug/ml DAP without Arab | (d) Minimal agar (Lys, Met, Thr) 0.4% (v/v) glycerol 0.1% glucose without Arab without DAP |
| pYA3615 pUC -*araC* P_{BAD} SD-ATG *murA* SD GTG-*asd* anti-*asd* | ++ | ++ | +/- | - |
| pYA3649 pUC*-araC* P_{BAD} SD-ATG *murA* SD ATG*-asd* anti-*asd* | + | + | +/- | - |
| pYA3650 pUC-*araC*P_{BAD} SD-GTG*murA* SD-GTG-*asd* anti-*asd* | ++ | ++ | - | - |
| pYA3651 pUC *-araC* P_{BAD} SD-GTG *murA* SD ATG-*asd* anti-*asd* | + | ++ | +/- | - |
| | | | | |

| | | | | |
|---|---|---|---|---|
| * ++: growth +: growth slowly . +/- :very poor growth -:no growth | | | | |

Chi8854 containing any of the plasmids with the B/r *araC* P_{BAD} sequence (prior to introducing the *E*. *coli* K-12 *araC* P_{BAD} sequence present in pYA3624) demonstrated arabinose-independent growth and grew on medium (c). In general, lysis plasmids with the lower copy number p15A *ori* demonstrated arabinose-dependant growth except for those plasmids such as pYA3613 and pYA3643 that possessed SD-ATG *murA* genes (Table 7). Chi8854 with either pYA3646 or pYA3647 was unable to grow on medium without arabinose independent of the presence of DAP. For the higher copy number DNA vaccine vectors, the most stringent arabinose-dependent growth was observed with pYA3650 in Chi8854. This plasmid has both SD-GTG *murA* and SD-GTG *asd* genes. When the plasmid, such as pYA3615 or pYA3649, has the SD-ATG *murA* sequence poor growth is observed in the absence of arabinose. Collectively, these results lead one to conclude that the chromosomal DeltaP_{murA7}:: *araC* P_{BAD} *murA* mutation-insertion is somewhat leaky and permits very low-level transcription in the absence of arabinose. To remedy this minor problem, we can readily substitute the non (less) leaky *E*. *coli* K-12 Chi289 *araC* P_{BAD} sequence in place of the *E*. *coli* B/r sequence in the DeltaPₘᵤᵣA7::TT *araC* P_{BAD} *murA* construction using pYA3624 (Figure 32) for the sequence to generate a suicide vector to yield this substitution. This construction was described in Example 7 and diagrammed in Figure 28.

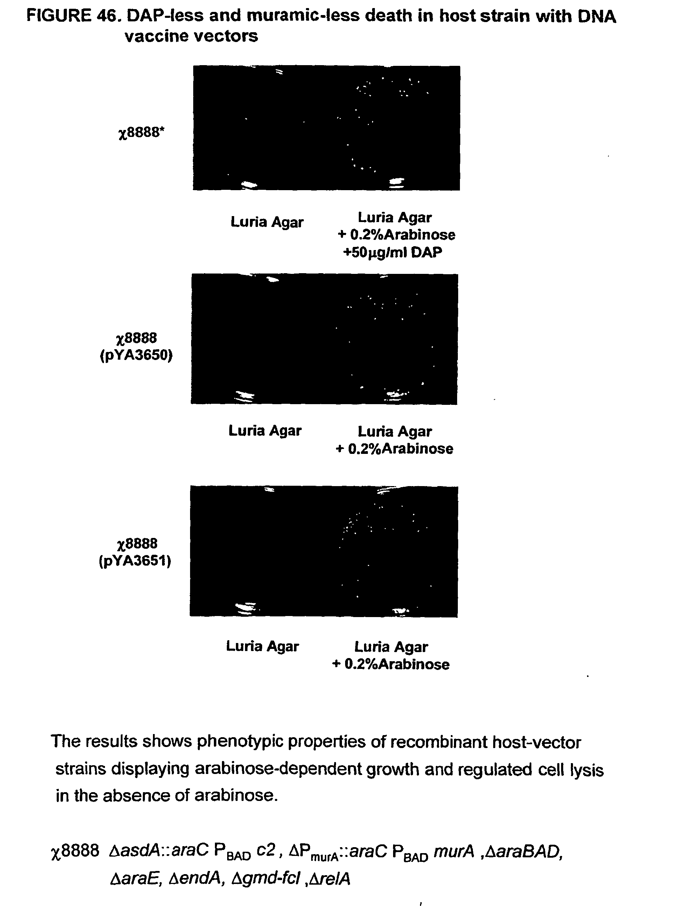

Figure 46 depicts the growth of Chi8888 on Luria agar in the absence and presence of DAP plus arabinose or arabinose alone lacking or containing the DNA vaccine vectors pYA3650 or pYA3651. Chi8888 is dependent on the presence of both arabinose and DAP whereas Chi8888(pYA3650) and Chi8888(pYA3651) are only dependent on arabinose for growth.

**Example 17**

Example 17 shows the virulence and colonizing ability of recombinant strains with DNA vaccine vectors in mice.

**Table 8.** Virulence of *S. typhimurium* strains with the pUC *ori* DNA vaccine vector pYA3615 as determined by oral inoculation into 8-week-old female BALB/c mice and immunity of surviving mice to challenge with wild-type *S. typhimurium* Chi3761.

| **Strain** | **Inoculating dose** | **Survivors/total** | **Challenge dose** | **Survivors/total after challenge** |
|---|---|---|---|---|
| Chi8804(pYA3615 ) | 1.6 x 10⁹ | 5/5 | 2.1 x 10⁹ | 2/5 |
| Chi8805(pYA3615 ) | 1.0 x 10⁹ | 5/5 | 2.1 x 10⁹ | 4/5 |
| Chi8806(pYA3615 ) | 1.1 x 10⁹ | 5/5 | 2.1 x 10⁹ | 0/5 |
| Chi8807(pYA3615 ) | 8.4 x 10⁸ | 5/5 | 2.1 x 10⁹ | 1/5 |

Bacteria were grown in Luria broth supplemented with 0.2% arabinose to OD₆₀₀ of about 0.8. Bacterial cells were collected by centrifugation and suspended in buffered saline with gelatin (BSG) for oral inoculation of mice. Morbidity and mortality were observed for 30 days. Surviving mice were challenged 30 days after the initial inoculation with virulent wild-type UK-1 Chi3761 grown in Luria broth. Morbidity and mortality observations were recorded daily for an additional 30 days post challenge. Both inoculating and challenge doses were measured in CFU.

Genotype of strains:

Chi8804 (Delta*araE25*, Delta*asdA19*::TT*araC* P_{BAD} *c2* 5ST1T2 DeltaP _{murA7}::*araC* P_{BAD} *murA*).
Chi8805 (Delta*ara*_{BAD}*1923*,Delta*asdA19*::TT*araC* P_{BAD} *c2* 5ST1T2,(DeltaP_{murA7}::*araC* P_{BAD} *murA).*
Chi8806 (Delta*asdA19*::TT*araC* P_{BAD} c2 5ST1T2,DeltaP_{murA7}::*araC* P_{BAD} *murA).*
Chi8807 (Delta*araE25*,Delta*ara*BAD*1923*,Delta*asdA19*::TT*araC* P_{BAD} c2 5ST1T2,(DeltaP_{murA7}::*araC* P_{BAD} *murA).*

The results in Table 8 reveal that the recombinant strains are totally attenuated and cause no deaths when the inoculating doses were 10,000 times what would be a lethal dose for the wild-type Chi3761 strain. Partial protective immunity against subsequent challenge is apparent with some strains but not with the arabinose-utilizing strain Chi8806, at least at the high challenge dose used. This lower immunogenicity can be due to rapid breakdown of arabinose to cause cell wall-less death to commence more rapidly to induce less of an immune response to *Salmonella* antigens. These results are very desirable, however, since the objective is to induce maximal immune responses to antigens specified by the DNA vaccine vector or released by lysing *Salmonella* cells. Some immunity to *Salmonella* is a plus but is not the objective and a very strong induction of immunity to *Salmonella* antigens could compete in the induction of desired immune responses to specified or delivered protective antigens.

Table 9 presents data demonstrating that Chi8888, Chi8888(with pYA3650) and Chi8888(with pYA3651) were avirulent when orally administered at high doses to female BALB/c mice. Chi8888 with either pYA3650 or pYA3651 conferred a modest level of immunity to challenge with the wild-type virulent S. *typhimurium* UK-1 strain Ghi3761, at a dose 10,000 times its mean lethal dose (Table 5). In contrast, Chi8888 without a vector did not induce a detectable level of immunity to challenge.

**Table 9.** Virulence of *host* strain χ8888 with DNA vaccine vectors pYA3650 and pYA3651 orally inoculated into 8-week-old female BALB/c mice and immunity of surviving mice to challenge with wide-type *S*. *typhimurium* χ3761

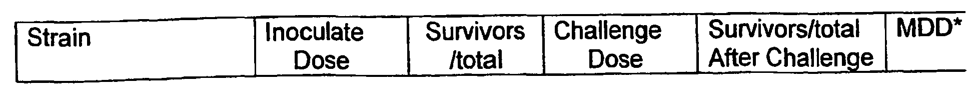

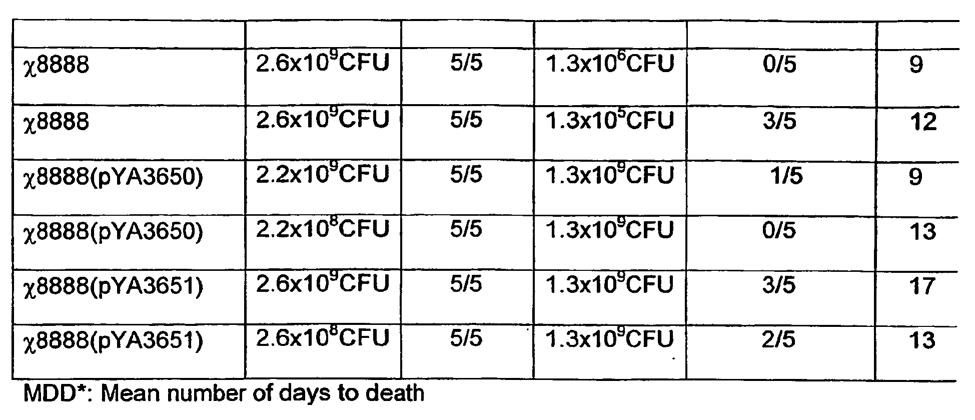

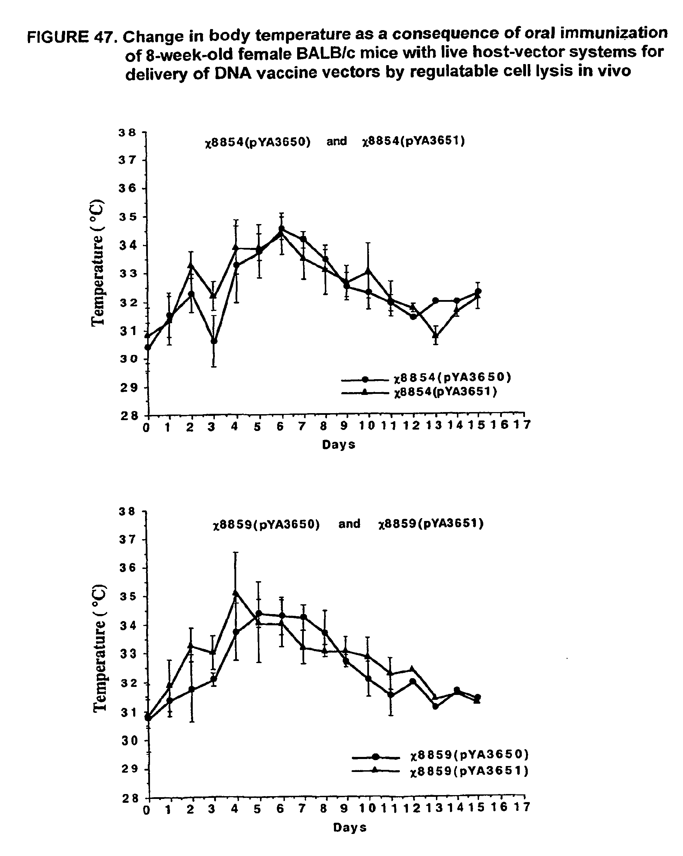

Colonization of 8-week old female BALB/c mice orally inoculated with Chi8854 (pYA3650) was investigated. The results presented in Table 10 indicate that the constructed DNA vaccine delivery host-vector systems are able to transiently colonize lymphoid tissues but at very modest numbers. These results probably represent a significant underestimation of the bacterial titers achieved in various tissues. Since these bacterial strains are being stressed by being caused to lyse in vivo, it is likely that cells are probably killed in the course of tissue maceration, vortex mixing, dilution and plating by spreading on plates. We have observed this problem before, as have others. We therefore decided to evaluate a clinical parameter indicative of systemic infection. We thus chose to measure temperatures of mice rectally (Stiles et al., Infect. Immun. 67:1521-1525 (1999); Li et al., Infect. Immun. 70:2519-2525 (2002)) under the expectation that infection and eventual lysis would cause a significant increase in temperature but that little or no temperature increase would be observed if in vivo proliferation and colonization of lymphoid tissues was minimal. We used the commercially available rectal temperature probe YSI 402. As revealed in Figures 47A and B, there was a significant increase in temperature of mice after oral inoculation with Chi8854 (pYA3650) and Chi8854 (pYA3651) due to cell lysis in vivo. The same results were observed using Chi8859 as the host. It lacks the Delta*araE25* mutation present in Chi8854. It is significant that the temperatures return to normal within two weeks after inoculation of mice. This to is a very desirable outcome. If the fever represents too much of an inflammatory response, the Delta*msbB48* mutation is introduced to the DNA vaccine delivery host strain as described in Example 5. The Delta*msbB48* mutation detoxifies the lipid A endotoxin.

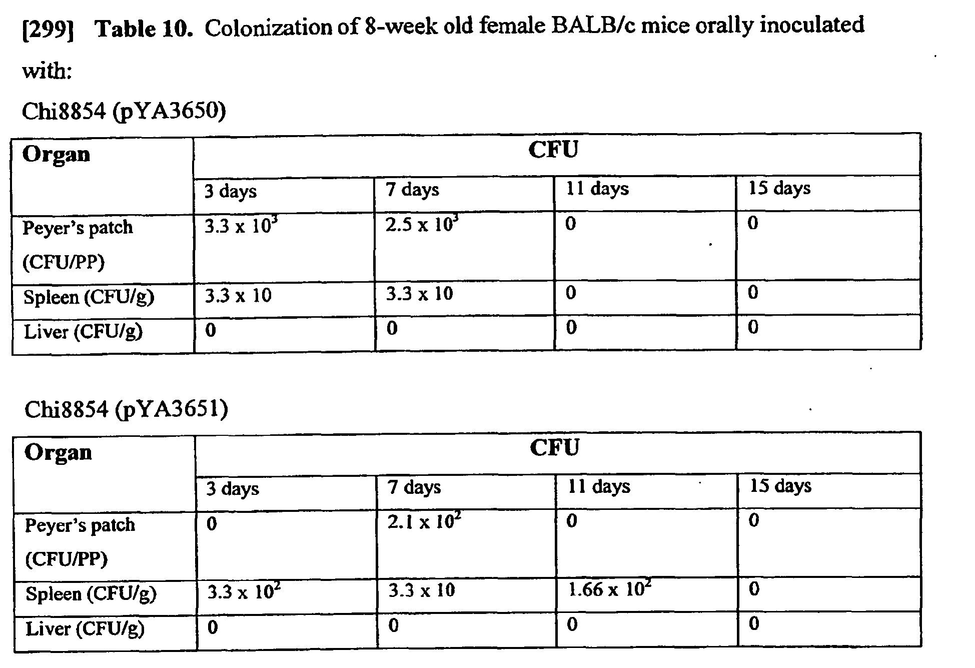

**Example 18**

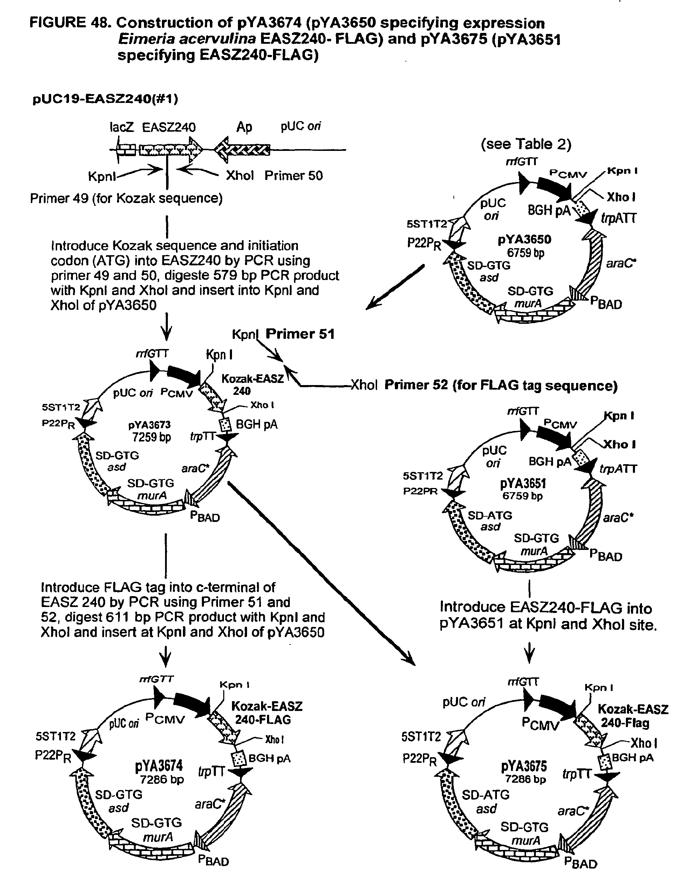

Example 18 shows construction of DNA vaccine vectors specifying the *Eimeria acervulina* sporozoite antigen EASZ240 and the merozoite antigen EAMZ250 with FLAG epitope fusions. Figure 48 diagrams the construction of pYA3650 and pYA3651 derivatives specifying the *Eimeria acervulina* sporozoite antigen EASZ. This antigen induces antibodies that react with the same surface sporozoite antigen in *E. acervulina, E. tenella,* and *E. maxima,* the three most common causes of coccidiosis on U.S. poultry farms. We PCR amplified the coding sequence for EASZ from a pUC19 recombinant plasmid. The PCR method introduced a Kozak sequence to facilitate translation (Kozak, J. Cell Biol. 115:887-903 (1991)) and an ATG start codon at the N-terminal end of the EASZ sequence. At the C-terminal end, we used PCR to introduce the FLAG peptide encoding sequence. The FLAG peptide is a strong T-cell epitope that induces a CTL response and is also recognized by a commercially-available MAb (Einhaurer and Jungbauer, J. Biochem. Biophys. Meth. 49:455-465 (2001); Kaltwasser et al, App. Environ. Microbiol. 68:2624-2628 (2002)). This provides a control for monitoring induction of T-cell immunity and also provides a means to quantitate expression of the EASZ-FLAG fusion protein in cells in culture or in vivo in and immunized animal. The resulting plasmids pYA3674 (derived from pYA3650) and pYA3675 (derived from pYA3651) are diagrammed in Figure 48.

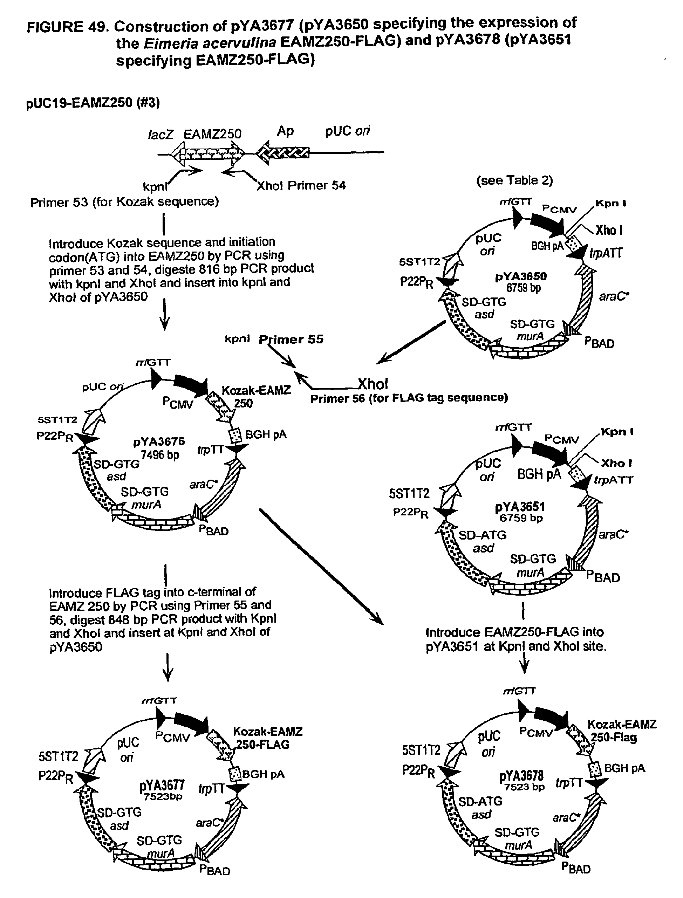

Figure 49 diagrams the construction of the pYA3677 and pYA3678 plasmids derived from pYA3650 and pYA3651, respectively, that specify the synthesis of the *E*. *acervulina* merozoite antigen EAMZ-250 with N-terminal Kozak and C-terminal FLAG sequences.

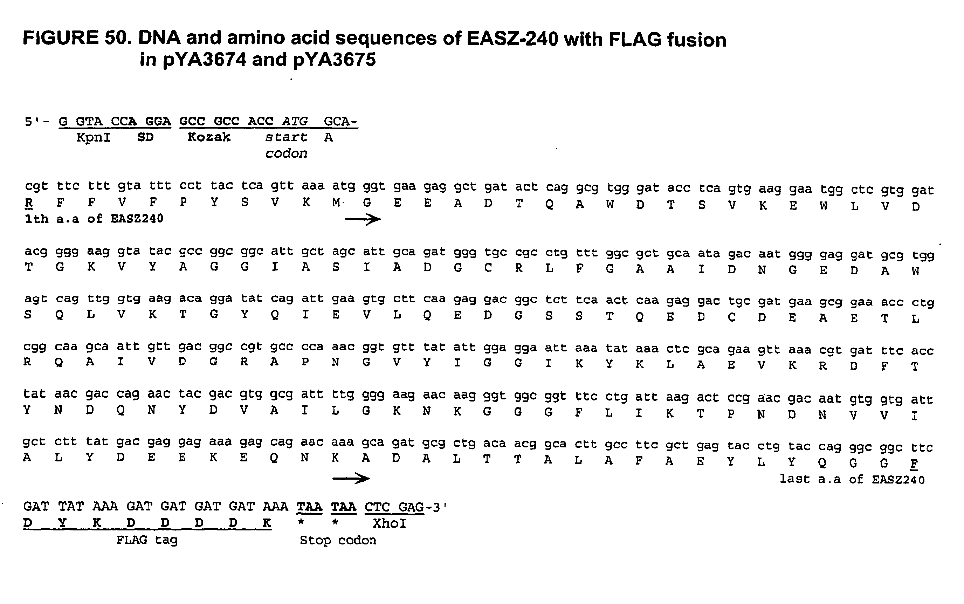

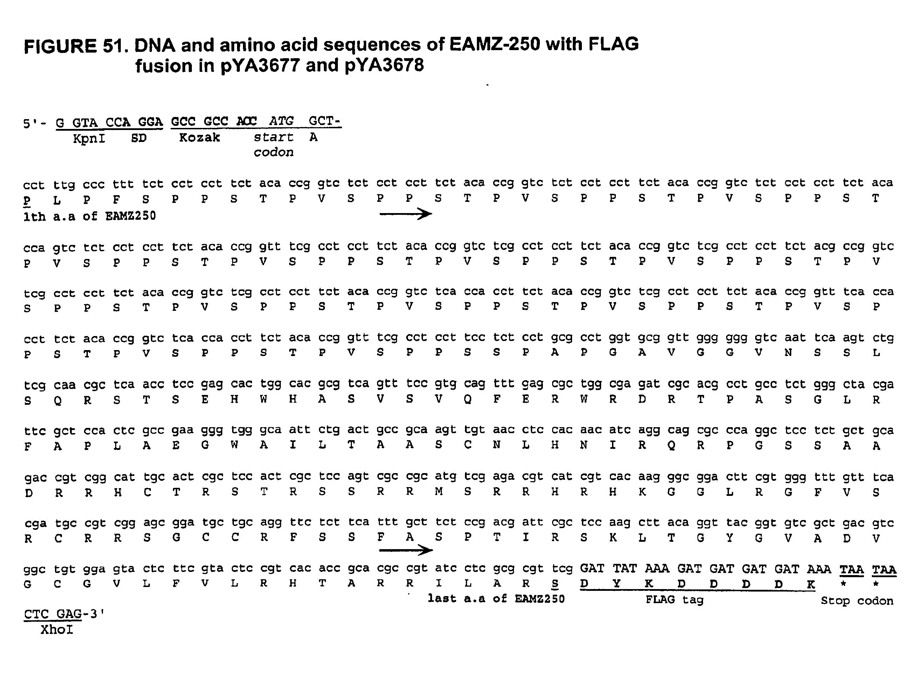

Figures 50 and 51 provide the nucleotide and amino acid sequences of the EASZ240 and EAMZ250 antigens specified by the constructed DNA vaccine vectors diagrammed in Figures 48 and 49, respectively.

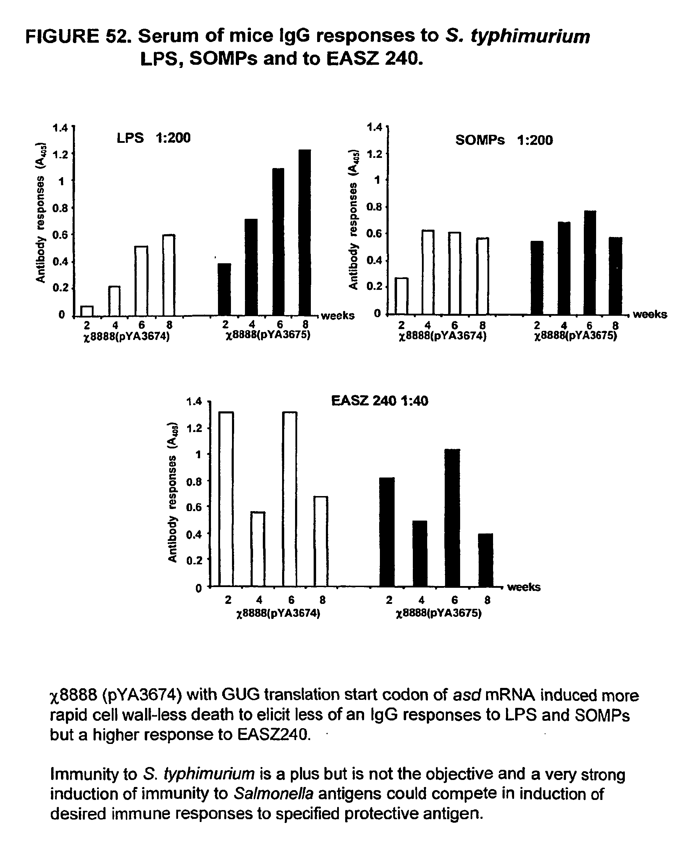

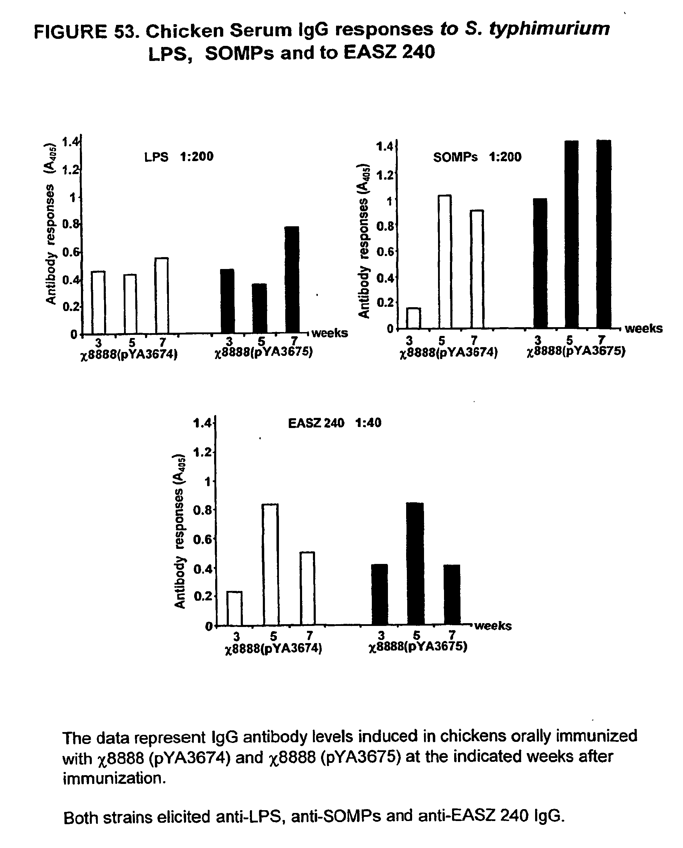

Chi8888 containing pYA3674 that was derived from pYA3650 with the GTG start codons for the *asd* and *murA* genes and encodes the *Eimeria acervulina* EASZ240 sporozoite antigen (Figure 48) to be expressed by cells within the immunized animal host was used to orally immunize 8-week old female BALB/c mice and day-of-hatch chicks. Mice were periodically bled by retroorbital bleeding and chicks by wing web veinipuncture. Sera were diluted and evaluated for IgG antibodies to *Salmonella* LPS and SOMPs and to the EASZ240 antigen. Figure 52 presents the immune response data from mice, and Figure 53 the immune response data from chickens.

**Example 19**

Example 19 shows construction of strains of bacteria with *in vivo* regulated lysis with gene attributes that enhance exit from the endosome and entry into the cytoplasm of host cells for release and function of genetic vaccines to induce CTL immune responses. We have devised two means to enhance the ability of *Salmonella* to exit the endosome.

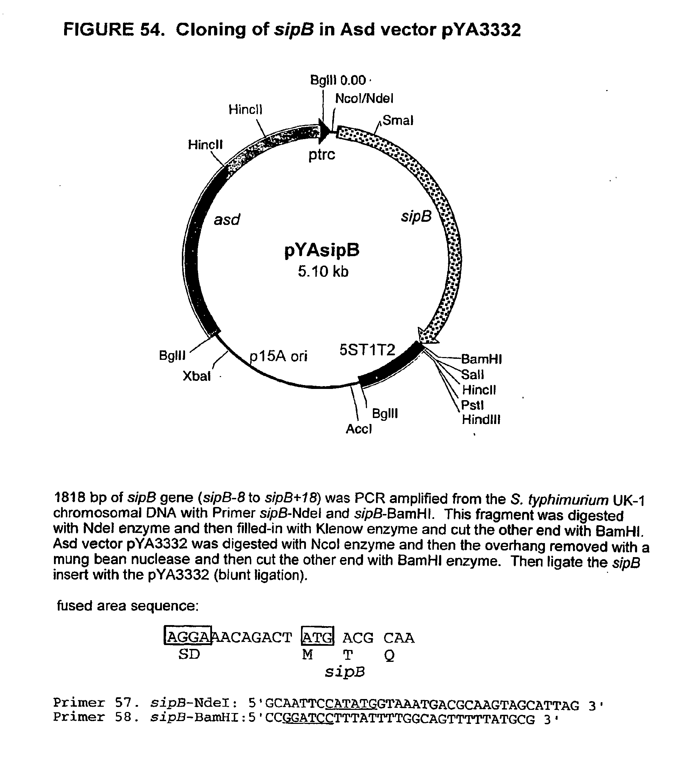

In one example, the Type III effector protein SipB is over-expressed on a plasmid such as that diagrammed in Figure 54 or the *sipB* gene is located on a DNA vaccine vector plasmid such as in pYA3650 (Figure 5A).

In another example, a means to cause *Salmonella* to escape from the endosome is to delete the *sifA* gene (Stein et al., Mol. Microbiol. 20:151-164 (1996); Brumell et al., Traffic 2002:3:407-4I5 (2002)). Figure 24 diagrams the steps used to generate the suicide vector pYA3716 to introduce the *DeltasifA26* mutation with an internal in-frame deletion of the *sifA* gene into the chromosome to generate Chi8926. To minimize the adverse effects of a deletion of the *sifA* gene, we also placed the chromosomal *sifA* gene under the control of the more tightly regulated *E. coli* K-12 *araC* P_{BAD}, such that the SifA protein would be in abundance early in infection and be diluted out as a consequence of cell division, to result in endosome escape after substantial colonization of lymphoid tissues has occurred. Figure 25 diagrams the construction of the suicide vector pYA3719 to introduce this chromosomal DeltaP_{sifA196}::TT *araC* P_{BAD} *sifA* deletion-insertion mutation. The DeltaP_{sifA196}::TT *araC* P_{BAD} *sifA* construction (Figure 4 aa) is introduced into Chi8888 using the transductional method described in Example 1. Escape from the endosome followed by lysis of the host-vector strain will release the DNA vaccine into the cytoplasm, which should enhance the transit of the DNA vaccine to the nucleus for transcription. Collectively, this should enhance synthesis of antigens, including protective antigens, encoded within the eukaryotic expression cassette and in turn, stimulate an enhanced immune response. The synthesis of antigen in the cell cytoplasm should ensure presentation by MHC class I and induction of a CD8⁺ dependent CTL response. This is further enhanced by encoding a protein sequence as a fusion to a selected antigen that is readily ubiquinated and thus rapidly targeted to the proteosome for more efficient presentation by MHC class I. This is accomplished by using the N-terminal ends of the SopE or SopE2 proteins that are very rapidly ubiquinated in the cytoplasm of eukaryotic cells and are thus rapidly proteolytically processed.

**Example 20**

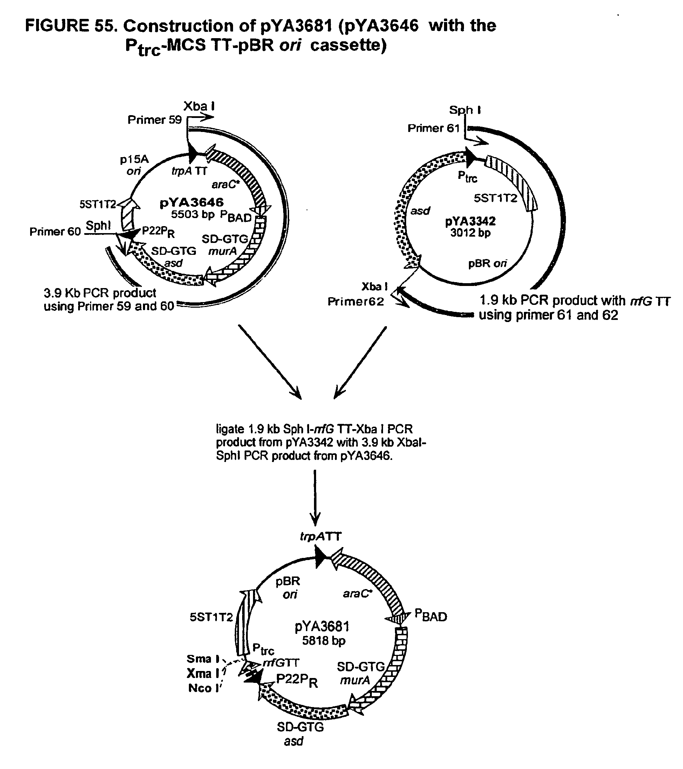

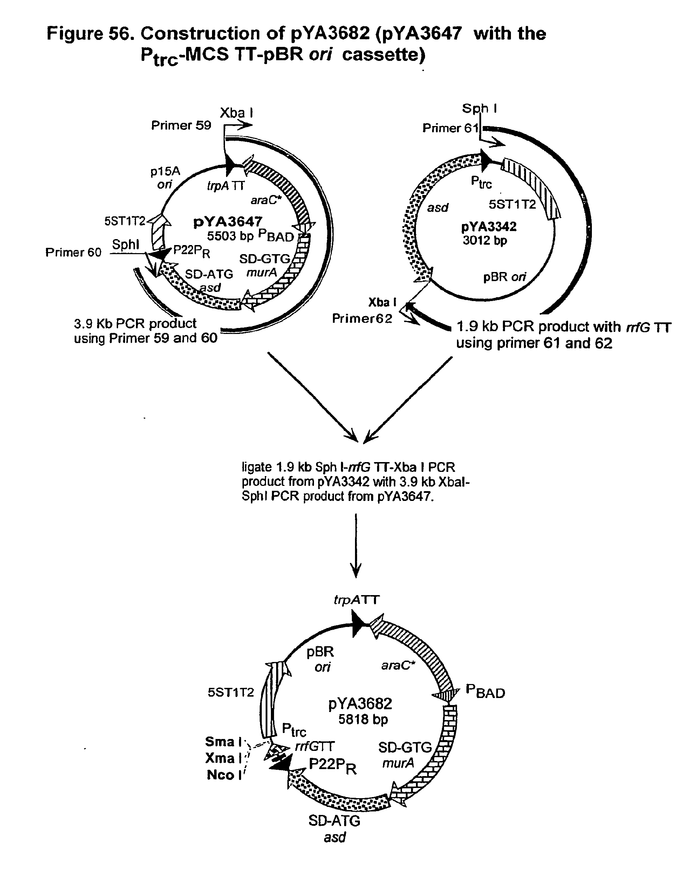

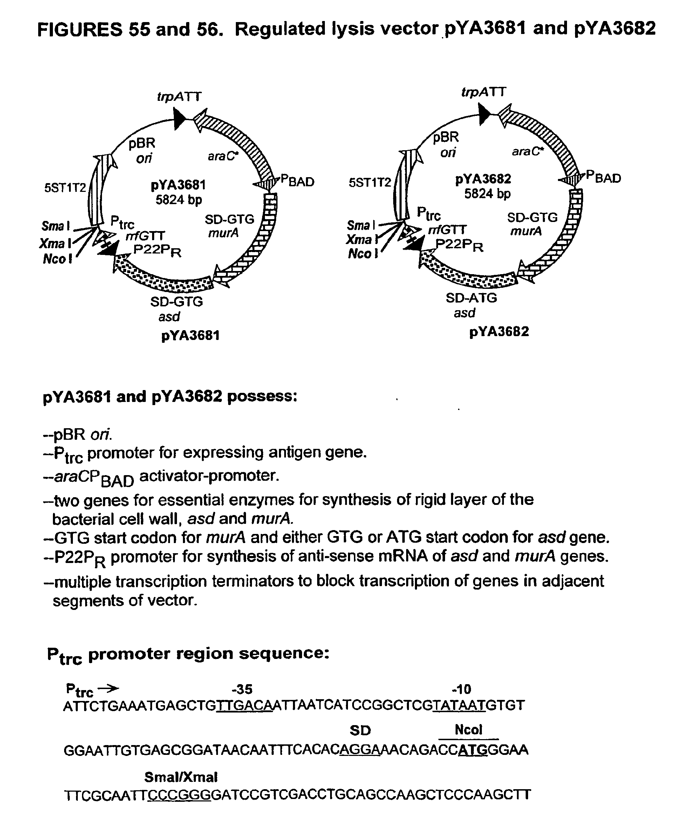

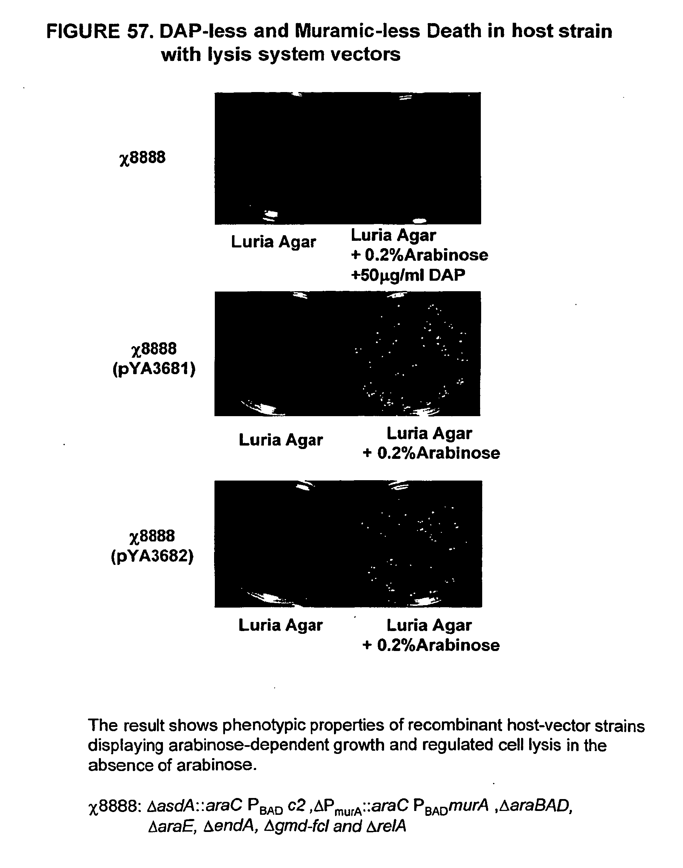

Example 20 shows construction of vectors to enable regulated lytic release of protective antigens specified by cloned genes and synthesized by the bacterial delivery host. The plasmids pYA3646 (Figures 30 and 31G) and pYA3647 (Figure 35) have the same gene components as in the DNA vaccine vectors pYA3650 (Figure 5A) and pYA3651 (Figure 5B), respectively. To convert them into vectors to specify synthesis of protective antigens within *Salmonella* before regulatable delayed lysis in immunized host lymphoid tissues, we inserted a regulatable prokaryotic promoter in association with multiple cloning sites. Since the level of protective antigen synthesis in bacteria is gene copy number dependent, it is generally desirable to have high copy number plasmids with either pBR *ori* or pUC *ori* rather than afforded by the lower copy number pSC101 *ori* and p15A *ori.* We therefore show in Figures 55 and 56 the construction of pYA3646 and pYA3647 derivatives, respectively, eliminating the p15A *ori* and replacing it with a PCR generated cassette encoding the regulatable P_{trc} promoter (ATTCTGAAATGAGCTGTTGACAATTAATCATCCGGCTCGTATAATGTGTGGA ATTGTGAGCGGATAACAATTTCACACAGGAAACAGACCATGGGAATTCGCAA TTCCCGGGGATCCGTCGACCTGCAGCCAAGCTCCCAAGCTT), multiple cloning sites, the transcription terminator 5ST1T2 (from the *E. coli rrnB* gene), and the pBR *ori* derived from the SD*-asd* vector pYA3342 (Table 2). Table 4 shows the oligonucleotide primers used for the PCR reactions. The constructed plasmids pYA3681 and pYA3682 are diagrammed in Figures 55 and 56. A very similar construction can be made using sequence from pYA3341 (Table 2) that has the same DNA sequence as pYA3342, except it has the pUC *ori* instead of the pBR *ori.* The nucleotide sequence for the P_{trc} promoter and multiple cloning sites in the regulatable expression lysis plasmids (Figures 55 and 56) that are also present in pYA3342 and pYA3341 are given at the bottom of Figure 44. The *Nco*I site facilitates cloning of entire coding sequences for protective antigens because it provides the ATG start codon within the restriction enzyme recognition sequence. Because some of the restriction cleavage sites in the multiple cloning site of pYA3342 are no longer unique due to the sequences present in the regulatable lysis vectors pYA3681 and pYA3682, we did not list them among the multiple cloning sites for the two regulated lysis vectors (Figures 55 and 56).

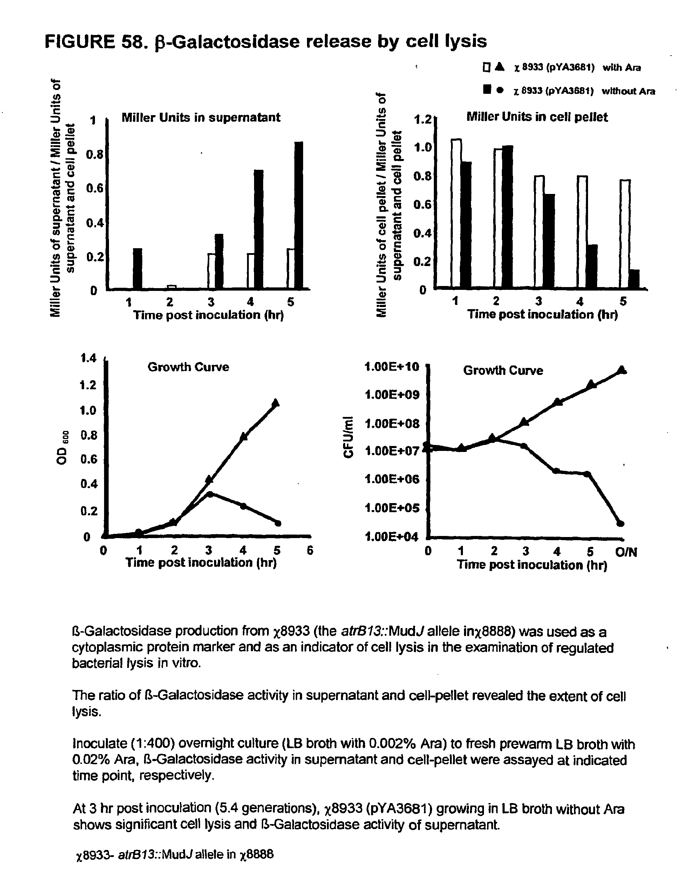

Figure 57 shows that Chi8888 with either plasmid is dependent on arabinose in Luria agar for growth. Cell lysis and release of cell contents were measured against time after strains like Chi8888 were deprived of arabinose. We introduced the chromosomal *atrB13::MudJ* element (Kang *et al.,* Infect. Immun. 70:1739-1749 (2002)) by P22-mediated transduction to generate Chi8933. The MudJ encodes constitutive synthesis of beta-galactosidase, which is retained in the cytoplasm and is only released into the medium upon cell lysis. We measured the amount of beta-galactosidase in the cell pellet versus the amount released into the supernatant fluid for Chi8933 (pYA3681) growing in medium with and without arabinose. An overnight culture grown in LB broth with 0.002% arabinose was diluted 1:400 in either prewarmed LB broth containing 0.02% arabinose or prewarmed LB broth containing no arabinose. At each time interval, samples were taken and the amounts of beta-galactosidase measured in cell pellets and in the supernatant fluid. The amounts measured were evaluated by comparing ratios of the beta-galactosidase activities in the two fractions, one in the presence and one in the absence of arabinose in the medium. Figure 58 shows the results. Chi8933 (pYA3681) growing in the absense of arabinose showed considerable lysis and release of beta-galactosidase into the supernatant fluid as a function of time after inoculation into medium lacking arabinose. Conversely, the strain grown in medium with arabinose retained essentially all beta-galactosidase intracellularly.

**Example 21**

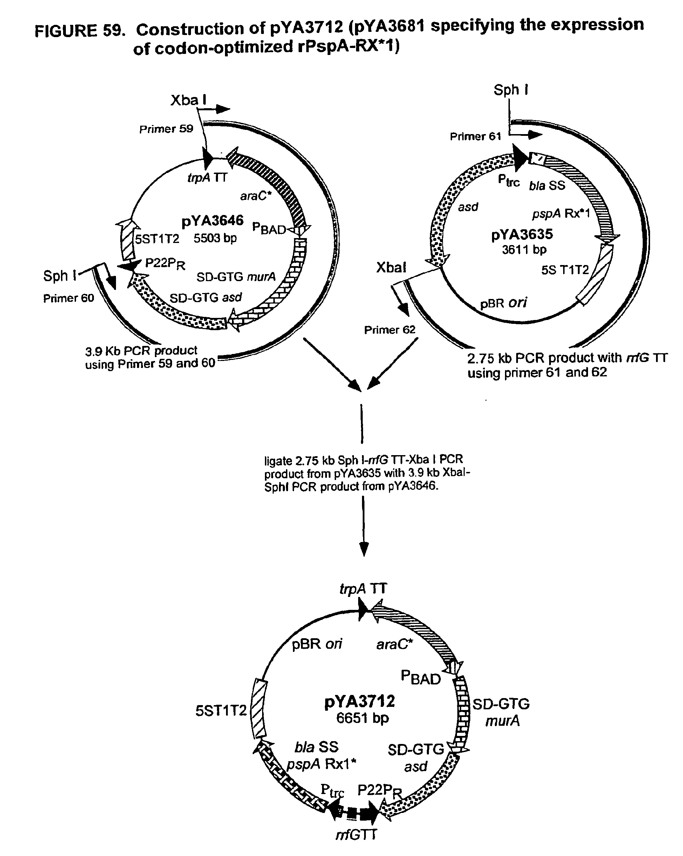

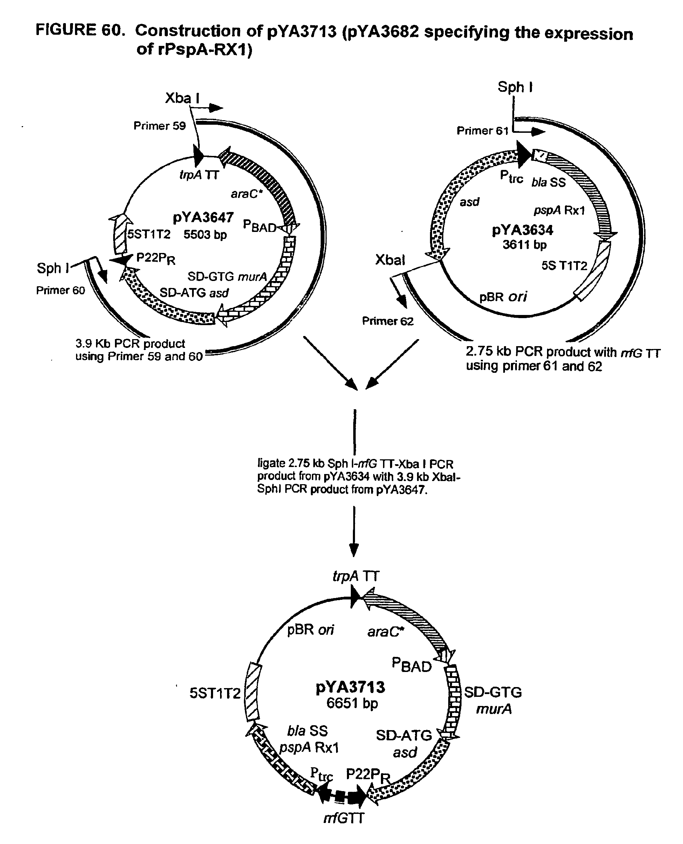

To evaluate the efficacy of a host-vector system for release of a protective antigen synthesized within the bacterial host prior to lysis *in vivo* after colonization of lymphoid tissues, we constructed recombinant plasmids to specify synthesis of the *S*. *pneumoniae* protective PspA antigen (Kang et al. (2002)). Specifically, we specified the synthesis of the alpha-helical domain of PspA that contained the protective B-cell epitopes fused to the signal sequence for beta-lactamase which caused export of some PspA antigen to the periplasmic space and resulted in enhanced immunogenicity (Kang et al. (2002)). We further used gene splicing techniques to insert a PspA-encoding sequence that had been codon optimized. That is, we selected codons that are used in highly expressed genes in *E. coli* and *Salmonella* to result in pYA3712 (Figure 59) and inserted the native sequence encoding the same segment of the *S*. *pneumoniae* strain RX1 PspA protein in pYA3713 (Figure 60). The introduction of these plasmids into Chi8888 resulted in the release of large quantities of PspA antigen as a consequence of inoculation into medium lacking arabinose.

Because expression of protective antigens during growth of a vaccine strain and during the initial phases of infection after orally administration of the vaccine to a host may potentially interfere with successful colonization of the internal lymphoid tissues, we subjected the antigen expression to regulation. Since the P_{trc} promoter, which is used to control expression of the beta-lactamase-PspA fusions in pYA3712 and pYA3713 (Figures 59 and 60), is repressible by the LacI repressor, a host component of the regulatable host-vector lysis system can be modified by inserting into the chromosome an *araC* P_{BAD} *lacI* construction such that the LacI repressor protein is produced in abundance during growth of the vaccine strain and during the initial phase of infection and colonization of the immunized animal or human host. During subsequent cell divisions of the regulatable lysis host-vector system, the LacI repressor concentration decreases and transcription of the DNA sequences encoding the protective antigen commences from the de-repressed P_{trc} promoter. Thus a vaccine vector strain, such as Chi8888, is further modified by insertion of the Delta*ilv*G3::TT *araC* P_{BAD} *lacI* deletion-insertion mutation present in Chi8623 (Table 1) using the suicide vector pMEG-249 (Table 2) and the transductional transfer method described in Example 1. Alternatively, or in addition to, the Delta*araBAD23 c2 lac*I::*rrf*G or Delta*araBAD23 lacI*::*rrf*G deletion-insertion mutation and their suicide vectors (Figure 12) are used to replace the Delta*araBAD1923* mutation in the chromosome of Chi8888 or its derivatives. As described in Example 2, we construct additional Delta*endA*::TT *araC* P_{BAD} *lacI* and Delta*relA*::TT *araC* P_{BAD} *lacI* deletion-insertion mutations that are further optimized by use of an ideal AGGA SD sequence and an ATG start codon instead of the native GTG start codon (Figures 4 y, 14 and 18). Thus, we have multiple options to construct strains with multiple genes encoding the LacI repressor or with high-level synthesis of LacI that would provide additional generations of cell division prior to de-repression of the pYA3681 or pYA3682 vector P_{trc} promoter. These *lac*I genes are regulated by the *S. typhimurium araC* P_{BAD} activator-promoter, the *E. coli* B/r *araC* P_{BAD} activator-promoter and/or the *E. coli* K-12 *araC* P_{BAD} activator-promoter. Derepression of P_{trc} with synthesis of the antigen would commence several generations prior to the onset of lysis due to higher numbers of plasmid-encoded Asd and MurA enzyme molecules that need to be diluted by cell division. The number of generations of growth prior to derepression or prior to lysis is further modulated by choosing the concentration of arabinose to add to the growth medium of the host-vector strain prior to immunization of an animal, such as humans.

**Example 22**

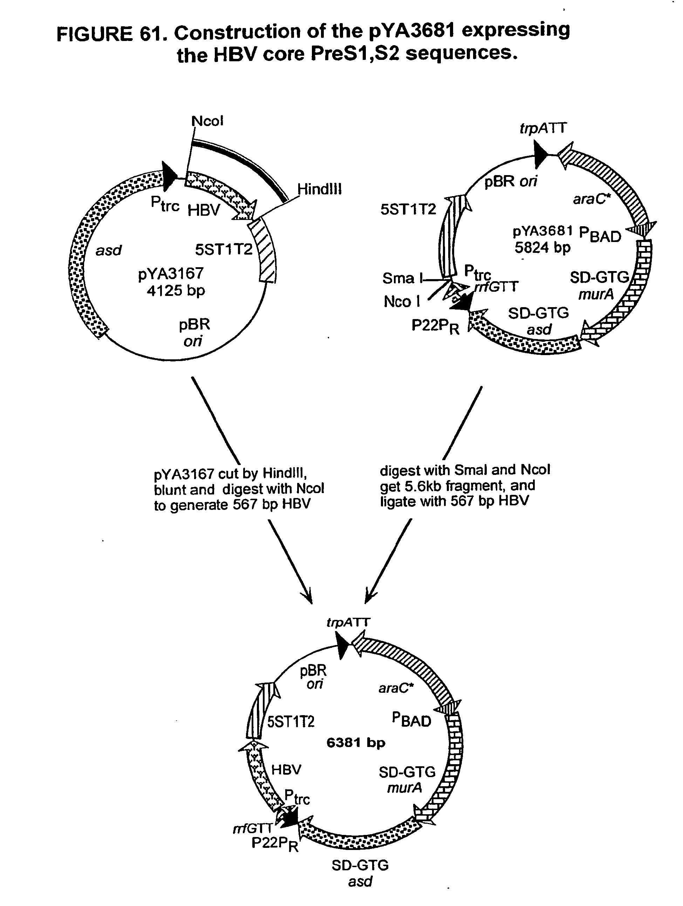

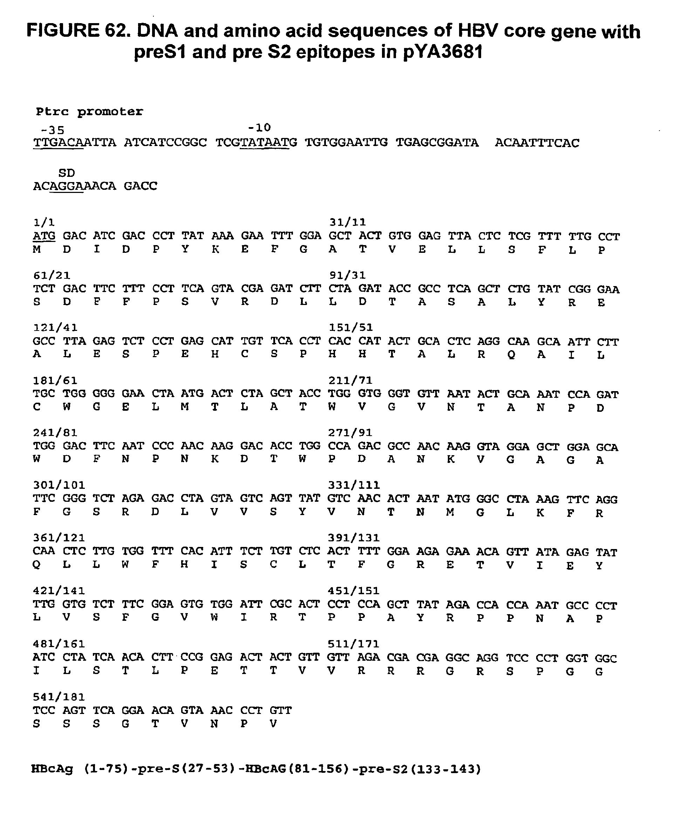

Example 22 shows construction of a recombinant vaccine strain with regulated expression of cloned genes for the hepatitis B virus core with inserted pre S1, S2 epitopes and regulated in vivo lysis to release the synthesized HBV core pre S1, S2 antigen. We have extensively used the hepatitis B virus core as a particulate antigen produced by attenuated *Salmonella typhimurium* for mice (Schodel et al., Infect. Immun. 62:1669-1676 (1994)) and in *S*. *typhi* for humans (Nardelli-Haefliger et al., Infect. Immun. 64:5219-5224 (1996)). The HBV core gene product self assembles when synthesized in bacteria and the core gene can be engineered to express protective B-cell and T-cell epitopes from various pathogens including HBV preS1, S2 and *Plasmodium falciparum* circumsporozoite antigens. They can also be used to express protective antigens of *Eimeria* species. Figure 46 shows the insertion of the DNA sequence encoding the HBV core with insertion of the pre S1 and pre S2 epitopes as encoded in the vector pYA3167 (Nardelli-Haefliger et al.) into the multiple cloning site in the regulatable lysis plasmid pYA3681 or pYA3682vector described in Figure 61. Such a plasmid construct introduced into a derivative of Chi8888 with one or more chromosomal regulatable *araC* P_{BAD} *lacI* deletion-insertion mutations (Example 13) is expected to induce with high efficacy immunity to the protective preSI and pre S2 epitopes due to synthesis and assembly of HBV core particles in the host-vector and their release by regulatable lysis into lymphoid tissues of the immunized animal or human host. Figure 62 shows the nucleotide sequence of the inserted DNA encoding the HBV core with the inserted pre S1 and pre S2 epitopes and the amino acid sequence specified by this sequence.

The HBV surface (S) antigen is a protective antigen present in current commercially-available HBV vaccines. The S antigen is glycosylated and must be synthesized in eukaryotic cells to elicit protective immunity, and a DNA vaccine specifying the HBV S antigen can stimulate immune responses. (Oka *et al.,* Immunology 103:90-97 (2001)). It follows that a DNA sequence encoding the HBV S antigen could be inserted into the DNA vaccine vector pYA3650 (Figure 5A), for example, to be introduced into Chi8888 or a derivative. The vaccine is further improved by stimulating immune responses to the protective preS1 and preS2 antigens.

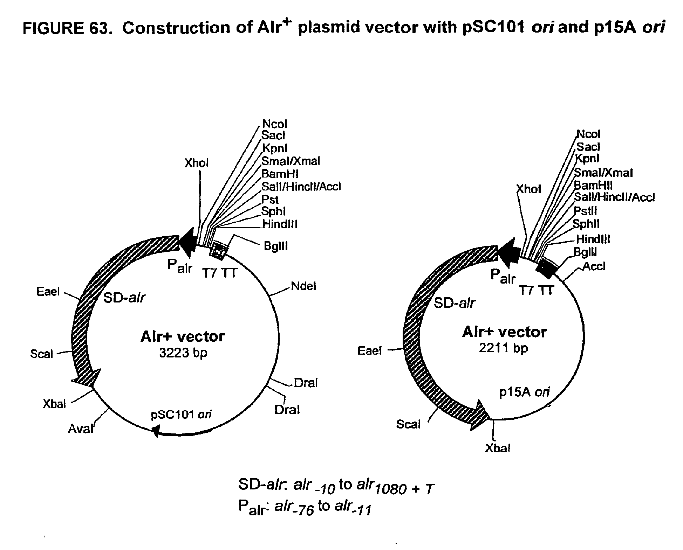

As described in Example 6, we are able to introduce both the Delta*alr*-3 and Delta*dadB4* mutations into Chi8888 or a derivative to impose a requirement for D-alanine. An Alr⁺ or DadB⁺ plasmid is introduced into the Chi8888 derivative. The Alr⁺ or DadB⁺ plasmid contains a replicon that is compatible with the pBR *ori* and pUC *ori* plasmids, such as pYA3681 and pYA3682 for synthesis of antigens within the bacterial host or pYA3650 and pYA3651 for synthesis of antigens in the immunized host. This is accomplished by using a mini-F *ori,* a mini-I-alpha *ori,* the pSC101 *ori* or the p15A *ori* as the replicon for the Alr⁺ or DadB⁺ plasmid. The design of Alr⁺ plasmids with the pSC101 *ori* and p15A *ori* is diagrammed in Figure 63. These plasmids enable insertion of genes with their own promotor or genes linked to a regulatable promoter, such as lambda P_{L}, can be used. Lamda P_{L} is regulated with a transiently expressed lambda C1 repressor. A unique transcription terminator that is not homologous to any transcription terminators in *Salmonella* can be used at the end of the gene insertion. Such transcription terminator can derive from a phage genome.

The inventors contemplate that an improved vaccine against HBV can be made using the HBV core and preS1 and preS2 fusions as the antigen. The fusion is placed in an Alr⁺ plasmid with the p15A *ori.* The S antigen derives from pYA3650 (pUC *ori).* Both plasmids are introduced into a Chi8888 derivative having at a minimum both the *Deltaalr-3* and *DeltadadB4* mutations. Other desired mutations to provide gene regulation functions and the like as described in the preceding Examples can also be included. This strain would display regulated delayed lysis *in vivo* after colonization of lymphoid tissues to release a bolus of recombinant HBV core particles, which would be synthesized and assembled in the bacterial host prior to lysis. This would stimulate protective immune responses to the preS1 and preS2 antigens, and the release of the DNA vaccine vector that would direct the synthesis of S antigen within cells in the immunized host to also induce a protective immune response to the S antigen.

**Example 23**

The inventors contemplate that the host-vector system of the present invention can also be used to develop a vaccine against *M. tuberculosis.* The Mtb 39A antigen is a 39 kDa protein belonging to the PPE family of conserved proteins of *M. tuberculosis.* Human T-cell epitopes have been identified in this protein, and the gene encoding this antigen has been inserted in a DNA vaccine vector, delivered by injection, to elicit protective immune responses in mice (Dillon et al.,Infect. Immun. 67:2941-2950 (1999)) and monkeys. Through PCR methods, we insert the DNA sequence encoding the Mtb 39A antigen with a Kozak sequence into the DNA vaccine vector pYA3650 (Figure 5A). The resulting product is introduced into a derivative of Chi8888, which has the Delta*alr*-3 and Delta*dadB4* mutations and other useful mutations. This DNA vaccine vector can be released into the cytoplasm of cells within the immunized individual where synthesis of the Mtb 39A antigen would take place. This would lead to MHC class I presentation and enhance induction of a CD8⁺-dependent CTL response. This process would be facilitated if the derivative Chi8888 strain used as the host strain also possessed the DeltaP_{sifA196}::TT *araC* P_{BAD} *sifA* construction to cause the host-vector strain to escape the endosome to ensure induction of a CD8⁺-dependent CTL response.

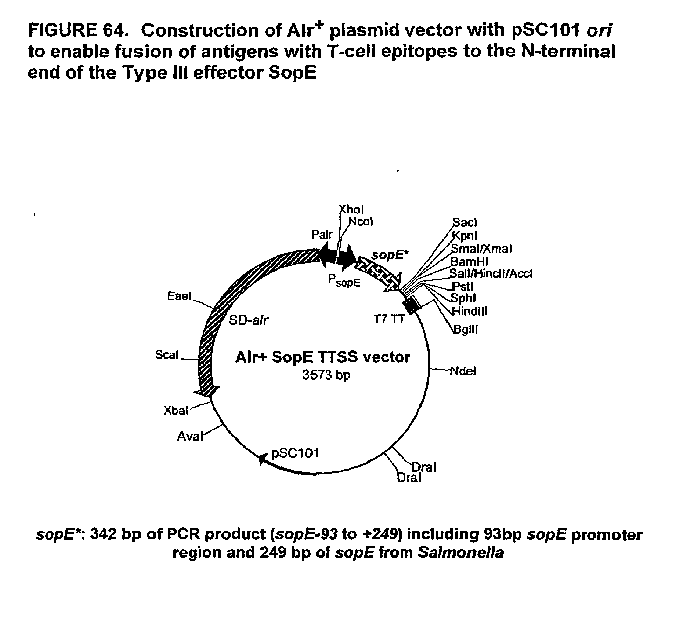

The ESAT-6 antigen and its co-expressed Cfp-10 antigen (and probably their homologs) contain human T-cell epitopes. We used the SPI-1 encoded TTSS of *Salmonella* to deliver these antigens to the cytoplasm of cells within an immunized individual. This means of delivery leads to MHC class I presentation to enhance induction of a CD8⁺-dependent CTL response. Sequences encoding ESAT-6 or Cfp-10 are introduced into an Alr⁺ vector derived from the construct shown in Figure 63), which possesses the N-terminal 77 amino acids of the Type III effector protein SopE (shown in Figure 64). The SopE protein is rapidly ubiquinated upon entry into the host cell cytoplasm and rapidly targeted to the proteosome for processing and presentation with MHC class I antigen. A further improvement in this vaccine construction is to include the N-terminal 60 or so amino acids of the SopE2 protein fused to the sequence encoding Mtb 39A in the pYA3650 DNA vaccine vector. This would ensure rapid ubiquination, processing and presentation of Mtb 39A T-cell epitopes by MHC class I antigen for later uptake and presentation by macrophages or dendritic cells.

**Example 24**

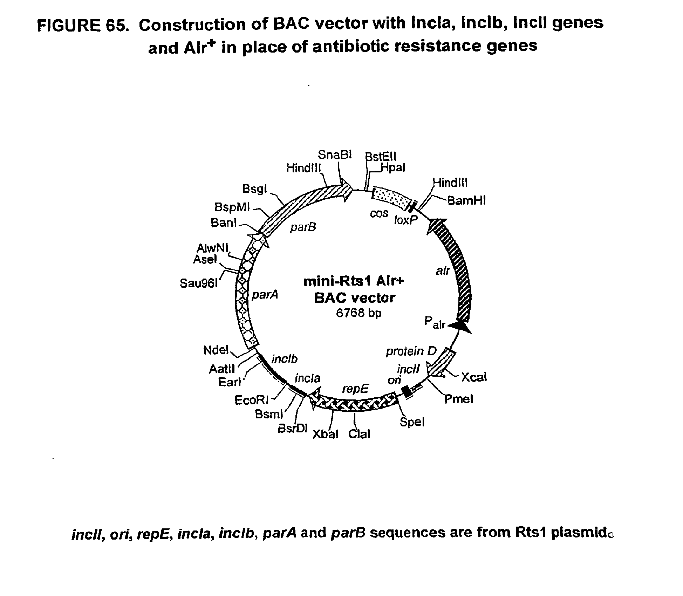

The host-vector strains with the regulated delayed lysis *in vivo* phenotype can also be used to deliver attenuated viral vaccines. This can be accomplished by having the entire genome of the attenuated virus cloned into a BAC vector such that the viral genome sequence excises from the BAC vector in the cytoplasm of cells to replicate and express viral antigens to induce protective immunity. This type of construction has been successfully developed for the delivery of a BAC containing an attenuated Marek's disease virus delivered as a DNA vaccine to chickens (Tischer *et al.,* J. Gen. Virol. 83:2367-2376 (2002)). Marek's disease virus vaccination is universally used in the poultry industry. Because currently available BAC vectors are derived from the mini-F plasmid and these plasmids are incompatible with the virulence plasmids of *Salmonella,* it is necessary to engineer the BAC vector to replace the IncF encoding genes with the IncI encoding genes for IncI-alpha, IncII and IncI-beta from the mini-Rts 1 plasmid. In addition, to avoid use of antibiotic resistance genes in live bacterial vaccines, we replace the drug resistance genes prevalent in BAC vectors with the wild-type *alr*^{*+*} gene. The construction of this IncI Alr⁺BAC plasmid is diagrammed in Figure 65. This vector is used to replace the IncF and drug resistance genes in the BAC20 recombinant clone containing the serotype 1 Marek's disease attenuated virus genome (Tischer et al.). The resulting construct is introduced into a Chi8888 derivative with the Delta*alr*-3, Delta*dadB4* and DeltaP_{sifA196}::TT *araC* P_{BAD} *sifA* deletion and deletion-insertion mutations and pYA3646 (Figure 31) for regulated delayed lysis. This vaccine construction is administered to day-of-hatch chicks through a course spray in a hatchery.

Table 11 shows the bacterial strains and host-vector systems that have been deposited with ATCC.

**Table 11. ATCC Deposited Strains**

| ATCC No | Strain | Date Deposited |
|---|---|---|
| 68169 | *S. typhimurium* UK-1. Chi3761 | 11-2-89 |
| 55114 | *S. typhi* ISP2822 Chi3745 | 10-31-90 |
| 55116 | *S. typhi* ISP1820 Chi3744 | 10-31-90 |
| 202182 | *S. typhi* Ty2 RpoS⁺ Chi8438 | 11-18-98 |
| PTA-4616 | *S. typhimurium* UK-1 Chi8854 (pYA3650) | August 2002 |
| PTA-4615 | *S. typhimurium* UK-1Chi8854 (pYA3651) | August 2002 |

The examples provided above are for illustrative purposes only, and not to limit the scope of the present invention. In light of the present disclosure, numerous embodiments within the scope of the claims will be apparent to those of ordinary skill in the art.

**REFERENCES**
1. Aggarwal, A., S. Kumar, R. Jaffe, D. Hone, M. Gross, and J. Sadoff. 1990. Oral *Salmonella:* malaria circumsporozoite recombinants induce specific CD8+ cytotoxic T cells. J. Exp. Med 172:1083-1090.
2. Alpuche-Arunda, et al., 1992, Proc. Nat. Acad. Sci. USA. 85:7972-7976.
3. Branstrom et al., U.S. Patent No. 5,824,538, filed on Sept. 6, 1995, issued on Oct. 20, 1998.
4. Brown, P., and R. Curtiss III. 1996. Unique chromosomal regions associated with virulence of an avian pathogenic *Escherichia coli* strain. Proc. Nail. Acad. Sci. USA 93:11149-11154.
5. Brumell et al., 2002, Traffic 2002:3:407-415.
6. Cardenes, L., and J.D. Clements. 1992. Oral immunization using live attenuated *Salmonella* spp. as carriers of foreign antigens. Clin. Microbiol. Rev. 5:328-342.
7. Chen, L.M., K. Kaniga, and J.E. Galán. 1996. *Salmonella* spp. are cytotoxic for cultured macrophages. Mol. Microbiol. 21:1101-1115.
8. Cookson, B.T. and M. J. Bevan. 1997. Indentification of a natural T cell epitope presented by *Salmonella*-infected macrophages and recognized by T cells from orally immunized mice. J. Immunol. 158(9):4310-9.
9. Czerkinsky, et al., 1983, J. Immunol. Methods. 65:109-121.
10. Curtiss, R. III, and S.A. Tinge. 1996. Recombinant bacterial vaccine system with environmentally limited viability. Patent WO 96/40947.
11. Curtiss, R. III, and S.M. Kelly. 1987. *Salmonella typhimurium* deletion mutants lacking adenylate cyclase and cyclic AMP receptor protein are avirulent and immunogenic. Infect. Immun. 55:3035-3043.
12. Curtiss, R. III, R. Goldschmidt, S.M. Kelly, M. Lyons, S. Michalek, R. Pastian, and S. Stein. 1987. Recombinant avirulent *Salmonella* for oral immunization to induce mucosal immunity to bacterial pathogens, p. 261-271. In H. Kohler, and P. T. LoVerde (ed.), Vaccines: New Concepts and Developments - Proceedings of the Tenth International Convocation on Immunology. Longman Scientific and Technical, Harlow, Essex, Great Britain.
13. Curtiss, 1990, New Generation Vaccines, p. 715-740.
14. Curtiss, 1965. J. Bacteriol. 89:28-40.
15. Denis et al., 1998, Infect. Immun. 66:1527-1533.
16. Doggett, T.A., E.K. Jagusztyn-Krynicka, and R. Curtiss III. 1993. Immune responses to Streptococcus sobrinus surface protein antigen A expressed by recombinant *Salmonella typhimurium.* Infect. Immun. 61:1859-1866.
17. Einhaurer and Jungbauer, J. Biochem. Biophys. Meth. 49:455-465.
18. Finlay et al. (1996, Mol. Microbiol. 20:151-164.
19. Frey et al., 2001, Clin. Immunol. 101:32-37.
20. Galan et al., U.S. Patent No. 6,306,387 B1, filed on Dec. 9, 1997, issued on Oct. 23,2001.
21. Guzman, L.-M., D. Belin, M.J. Carson, and J. Beckwith. 1995. Tight regulation, modulation, and high-level expression by vectors containing the arabinose PBAD promoter. J. Bacteriol. 177:4121-4130.
22. Hassan, J.0., and R. Curtiss III. 1990. Control of colonization by virulent Salmonella typhimurium by oral immunization of chickens with avirulent Deltacya Deltacrp S. typhimurium. Res. Microbiol. 141:839-850.
23. Hassan, J.O., S.B. Porter, and R. Curtiss III. 1993. Effect of infective dose on humoral immune responses and colonization in chickens experimentally infected with Salmonella typhimurium. Avian Dis. 37:19-26.
24. Hassan and Curtiss, 1994, Infect. Immun. 62:2027-2036.
25. Hassan and Curtiss, 1996, Infect. Immun. 64:938-944.
26. Hitchcock and Brown, 1983, J. Bacteriol. 154:269-277.
27. Kaltwasser et al, 2002, App. Environ. Microbiol. 68:2624-2628.
28. Kang et al. 2002 Infect. Immun. 70:1739-1749.
29. Kang et al (2002, J. Bacteriol. 184:307-312.
30. Kozak,1991, J. Cell Biol. 115:887-903.
31. Krieg, 2002, Annu. Rev. Immunol. 20:709-760.
32. Lewis P J and Babiuk L A, Adv Virus Res 54:129-88, 1999.
33. Li et al., 2002, Infect. Immun. 70:2519-2525.
34. Lietner W W et al., Vaccine 18(9-10):765-777,1999.
35. Lockman, H.A. and R. Curtiss III. 1992. Infect. Immune. 60:491-496.
36. Lockman, H.A., and R. Curtiss III. 1990. Occurrence of secondary attenuating mutations in avirulent *Salmonella typhimurium* vaccine strains. J. Infect. Dis. 162:1397-1400.
**37.** Luria, S.E., and JW. Burrous. 1957. Hybridization between Escherichia coli and *Shigella.* J. Bacteriol. 74:461-476.
38. Miller and Mekalanos, 1988, J. Bacteriol. 170:2575-2583.
39. Miller, G.A., B.S. Bhogal, R. McCandliss, R.L. Strausberg, E.J. Jessee, A.C. Anderson, C.K. Fuchs, J. Nagle, M.H. Likel, J.M. Strasser, and S. Strausberg. 1989. Characterization and vaccine potential of a novel recombinant coccidial antigen. Infect. Immun. 57:2014-2020.
40. Nardelli-Haefliger et al., 1996, Infect. Immun. 64:5219-5224.
41. Nakayama, K., S.M. Kelly, and R. Curtiss III. 1988. Construction of an Asd⁺ expression-cloning vector: stable maintenance and high level expression of genes in a *Salmonella* vaccine strain. Bio/Tech. 6:693-697.
42. Nayak, A.R., S.A. Tinge, R.C. Tart, L.S. McDaniel, D.E. Briles, and R. Curtiss III. 1998. A live recombinant avirulent oral *Salmonella* vaccine expressing pneumococcal surface protein A (PspA) induced protective responses against Streptococcus pneumoniae. Infect. Immun. 66:3744-3751.
43. Nicolet CM, et al. Cancer Gene Ther 2:161-70, 1995.
44. Pardoll D M and Beckerleg A M, Immunity, 3:165-169, 1995
45. Powell et al., U.S. Patent No. 5,877,159, filed on May 3, 1995, issued on Mar. 2, 1999.
46. Raetz and Whitfield, 2002, Annu. Rev. Biochem. 71:635-700.
47. Roth, J. et al., J Natl Cancer Inst 89: 21-39, 1997.
48. Sadoff, J.C., W.R. Ballou, L.S. Baron, W.R. Majarian, R.N. Brey, W.T. Hockmeyer, J.F. Young, J.J. Cryz, J. Ou, G.H. Lowell, and J.D. Chulay. 1988. Oral *Salmonella typhimurium* vaccine expressing circumsporozoite protein protects against malaria. Science 240:336-338.
49. Sambrook, J., E.F. Fritsch, and T. Maniatis. 1989. Molecular cloning: a laboratory manual. Cold Spring Harbor Laboratory, Cold Spring Harbor.
50. Schmieger, 1972, Molec. Gen. Genet. 119:75-88.
51. Schodel, F., and R. Curtiss III. 1995. *Salmonella* as oral vaccine carriers. Dev. Biol. Stand. 84:245-253.
52. Schodel et al., 1994, Infect. Immun. 62:1669-1676.
53. Sizemore, D.R., A.A. Branstrom, and J.C. Sadoff. 1995. Attenuated *Shigella* as a DNA delivery vehicle for DNA-mediated immunization. Science. 270:299-303.
54. Sizemore, D.R., A.A. Branstrom, and J.C. Sadoff. 1997. Attenuated bacteria as a DNA delivery vehicle for DNA-mediated immunization. Vaccine. 15:804-807.
55. Webster et al, Vacc., 12:1495-1498 (1994).
56. Sobol R, and Scanlon K J. Clinical protocols list. Cancer Gene Ther. 2:225-34, 1995.
57. Stein et al., 1996, Mol. Microbiol. 20:151-164.
58. Stiles et al., 1999, Infect. Immune. 67:1521-1525.
59. Tacket et al., 1992, Infect. Immun. 60:536-541.
60. Tacket et al., Infect. Immun. 65:3381-3385.
61. Wick, M.S., C.V. Harding, N.J. Twesten, S.J. Normark, and J.D. Pfeifer. 1995. The phoP locus influences processing and presentation of Salmonella typhimurium antigens by activated macrophages. Mol. Microbiol. 16:465-476.
62. Wick et al, Infect. Immun., 62:4542-4548 (1994).
63. Wu and Kipps, 1997, J. Immunol. 159:6037-6043.
64. Yang, W., G.J. Waine, and D.P. McMannus. 1995. Antibodies to Schistosoma-japonicum (asian bloodfluke) paramyosin induced by nucleic-acid vaccination. Biochem. Biophys. Res. Commun. 212:1029-1039.

## Claims

1. A host-vector system, which comprises:
a. a host chromosome comprising:
i. a first activatible control sequence, wherein the activatible control sequence is activatible by an inducer;
ii. a sequence that encodes a repressor, wherein the sequence is operably-linked to the first activatible control sequence; and
iii. at least one essential gene, wherein the essential gene encodes a polypeptide that is necessary for synthesis of a rigid layer of a cell wall of a prokaryote, and wherein the essential gene is inactivated, operably linked to the first activatible control sequence, or operably linked to a second activatible control sequence that is activated by a second inducer;
AND
b. a vector comprising:
i. at least one origin of replication (ori);
ii. a eukaryotic expression cassette comprising:
1. a eukaryotic promoter sequence;
2. a site for insertion of a gene encoding a desired gene product; and
3. a polyadenylation sequence;
iii. a prokaryotic activator-promoter sequence;
iv. at least one essential gene, wherein the essential gene is necessary for synthesis of a rigid layer of a cell wall of a prokaryote, that functions to complement the essential gene of (a. iii.) and is operably-linked to the prokaryotic-activator promoter sequence of (b.iii);
v. a regulatable prokaryotic promoter sequence, which is repressible by the repressor and oriented to cause synthesis of antisense RNA to the essential gene of (b.iv);
vi. at least one transcription terminator sequence operably-linked to the essential gene, ori, and/or eukaryotic expression cassette; and
vii. at least one CpG sequence motif, wherein the CpG sequence motif enhances immunogenicity.

2. A host-vector system, which comprises:
a. a host chromosome comprising:
i. a first activatible control sequence, wherein the activatible control sequence is activatible by a first inducer;
ii. at least one sequence that encodes at least one repressor, wherein the sequence is operably-linked to the first activatible control sequence;
iii. at least one essential gene, wherein the essential gene encodes a polypeptide that is necessary for synthesis of a rigid layer of a cell wall of a prokaryote, and wherein the essential gene is inactivated, operably linked to the first activatible control sequence, or operably linked to a second activatible control sequence that is activated by a second inducer;
AND
b. at least one vector comprising
i. at least one origin of replication (ori);
ii. a prokaryotic activator-promoter sequence;
iii. at least one essential gene, wherein the essential gene is necessary for synthesis of a rigid layer of a cell wall of a prokaryote, that functions to complement the essential gene of (a. iii.) and is operably-linked to the prokaryotic activator-promoter sequence of (b.ii);
iv. a first regulatable prokaryotic promotor sequence, wherein the first regulatable control sequence is repressible by said first repressor; and oriented to cause synthesis of antisense RNA to the essential gene(s) of (b.iii);
v. a second regulatable prokaryotic promotor sequence, wherein the second regulatable control sequence is repressible by a second repressor encoded by (a. ii);
vi. a site for insertion of a gene encoding a desired gene product wherein said site is operably-linked to the second regulatable prokaryotic promoter, and
vii. at least one transcription terminator sequence operably-linked to the essential gene(s), ori, and/or desired gene insertion site.

3. The host-vector system of claim 1 or 2 further comprising a gene encoding a desired gene product inserted in said site for insertion of a gene encoding a desired gene product.

4. The host-vector system of claim 3, wherein the gene encodes an antigen.

5. The host-vector system of claim 4, wherein the antigen is from a bacterial, viral, fungal, or parasitic pathogen.

6. The host-vector system of claim 4, wherein the antigen is from *Eimeria,* HBV, or *Streptococcus pneumoniae.*

7. The host-vector system of claim 3, wherein the host-vector system comprises a first vector comprising a desired gene in a eukaryotic expression cassette, a second vector comprising a desired gene in a prokaryotic expression cassette and wherein the desired gene product and the essential gene on said first vector is different from the desired gene product and the essential gene on said second vector.

8. The host-vector system of any one of claims 1 to 7, wherein the prokaryotic activator-promoter sequence is araC P_{BAD}.

9. The host-vector system of any one of claims 1 to 7, wherein the regulatable control sequence is P22 P_{R} or P_{trc}.

10. The host-vector system of any one of claims 1 to 7, wherein the repressor is C2, Lac I, or both.

11. The host-vector system of any one of claims 1 to 7, wherein the essential gene is *asd, murA, dapA,* or air.

12. The host-vector system of any one of claims 1 to 7, further comprising a mutation in a gene to enhance immunogenicity, wherein the mutation is Δ*endA2311,* Δ*relA1123,* ΔaraE25, ΔaraBAD1923, ΔaraBAD23, Δgmd-11, or Δgmd-fcl-26.

13. A microorganism comprising the host vector system of any one of claims 3 to 7 for use in the delivery of a desired gene product and/or a nucleic acid vector to a eukaryotic host.

## Patentansprüche

1. Gast-Vektor-System, welches umfasst:
a) ein Gastchromosom umfassend:
i. eine erste aktivierbare Kontrollsequenz, wobei die aktivierbare Kontrollsequenz durch einen Induktor aktivierbar ist;
ii. eine Sequenz, welche für einen Repressor kodiert, wobei die Sequenz mit der ersten aktivierbaren Kontrollsequenz operativ verknüpft ist; und
iii. mindestens ein wesentliches Gen, wobei das wesentliche Gen für ein Polypeptid kodiert, welches für die Synthese einer Stützschicht einer Zellwand eines Prokaryonten notwendig ist, und wobei das wesentliche Gen inaktiviert wird, mit der ersten aktivierbaren Kontrollsequenz operativ verknüpft ist oder mit einer zweiten aktivierbaren Kontrollsequenz operativ verknüpft ist, welche durch einen zweiten Induktor aktiviert wird;
UND
b) einen Vektor umfassend:
i. mindestens einen Replikationsanfang (ori);
ii. eine eukaryontische Expressionskassette umfassend:
1. eine eukaryontische Promotorsequenz;
2. eine Stelle zur Insertion eines Gens, welches für ein gewünschtes Genprodukt kodiert; und
3. eine Polyadenylierungssequenz;
iii. eine prokaryontische Aktivator-Promotorsequenz;
iv. mindestens ein wesentliches Gen, wobei das wesentliche Gen für die Synthese einer Stützschicht einer Zellwand eines Prokaryonten notwendig ist, welches zur Komplementierung des wesentlichen Genes von (a.iii) wirkt und mit der prokaryontischen Aktivator-Promotorsequenz von (b.iii) operativ verknüpft ist;
v. eine regulierbare prokaryontische Promotorsequenz, welche durch den Repressor reprimierbar ist und ausgerichtet ist, die Synthese von Antisense-RNA zu dem wesentlichen Gen von (b.iv) zu bewirken;
vi. mindestens eine Transkriptionsterminatorsequenz, welche mit dem wesentlichen Gen, ori, und/oder der eukaryontischen Expressionskassette operativ verknüpft ist; und
vii. mindestens ein CpG-Sequenzmotiv, wobei das CpG-Sequenzmotiv die Immunogenizität steigert.

2. Gast-Vektor-System, welches umfasst:
a. ein Gastchromosom umfassend:
i. eine erste aktivierbare Kontrollsequenz, wobei die aktivierbare Kontrollsequenz durch einen ersten Induktor aktivierbar ist;
ii. mindestens eine Sequenz, welche für mindestens einen Repressor kodiert, wobei die Sequenz mit der ersten aktivierbaren Kontrollsequenz operativ verknüpft ist;
iii. mindestens ein wesentliches Gen, wobei das wesentliche Gen für ein Polypeptid kodiert, welches für die Synthese einer Stützschicht einer Zellwand eines Prokaryonten notwendig ist, und wobei das wesentliche Gen inaktiviert wird, mit der ersten aktivierbaren Kontrollsequenz operativ verknüpft ist oder mit einer zweiten aktivierbaren Kontrollsequenz operativ verknüpft ist, welche durch einen zweiten Induktor aktiviert wird;
UND
b. mindestens einen Vektor umfassend:
i. mindestens einen Replikationsanfang (ori);
ii. eine prokaryontische Aktivator-Promotorsequenz;
iii. mindestens ein wesentliches Gen, wobei das wesentliche Gen für die Synthese einer Stützschicht einer Zellwand eines Prokaryonten notwendig ist, welches zur Komplementierung des wesentlichen Genes von (a.iii) wirkt und mit der prokaryontischen Aktivator-Promotorsequenz von (b.ii) operativ verknüpft ist;
iv. eine erste regulierbare prokaryontische Promotorsequenz, wobei die erste regulierbare Kontrollsequenz durch den ersten Repressor reprimierbar ist; und ausgerichtet ist, die Synthese von Antisense-RNA zu dem (den) wesentlichen Gen(en) von (b.iii) zu bewirken;
v. eine zweite regulierbare prokaryontische Promotorsequenz, wobei die zweite regulierbare Kontrollsequenz durch einen zweiten Repressor reprimierbar ist, welcher durch (a.ii) kodiert wird;
vi. eine Stelle zur Insertion eines Gens, welches für ein gewünschtes Genprodukt kodiert, wobei die Stelle mit dem zweiten regulierbaren prokaryontischen Promotor operativ verknüpft ist; und
vii. mindestens eine Transkriptionsterminatorsequenz, welche mit dem (den) wesentlichen Gen(en), ori, und/oder der gewünschten Geninsertionsstelle operativ verknüpft ist.

3. Gast-Vektor-System gemäß Anspruch 1 oder 2, welches weiterhin ein Gen umfasst, welches für ein gewünschtes Genprodukt kodiert, welches in die Stelle zur Insertion eines für ein gewünschtes Genprodukt kodierenden Gens insertiert wird.

4. Gast-Vektor-System gemäß Anspruch 3, wobei das Gen für ein Antigen kodiert.

5. Gast-Vektor-System gemäß Anspruch 4, wobei das Antigen aus einem bakteriellen, viralen, fungalen oder parasitären Pathogen stammt.

6. Gast-Vektor-System gemäß Anspruch 4, wobei das Antigen aus *Eimeria*, HBV oder *Streptococcus pneumoniae* stammt.

7. Gast-Vektor-System gemäß Anspruch 3, wobei das Gast-Vektor-System einen ersten Vektor, welcher ein gewünschtes Gen in einer eukaryontischen Expressionskassette umfasst, und einen zweiten Vektor umfasst, welcher ein gewünschtes Gen in einer prokaryontischen Expressionskassette umfasst, und wobei das gewünschte Genprodukt und das wesentliche Gen in dem ersten Vektor von dem gewünschten Genprodukt und dem wesentlichen Gen in dem zweiten Vektor verschieden ist.

8. Gast-Vektor-System gemäß einem der Ansprüche 1 bis 7, wobei die prokaryontische Aktivator-Promotorsequenz araC P_{BAD} darstellt.

9. Gast-Vektor-System gemäß einem der Ansprüche 1 bis 7, wobei die regulierbare Kontrollsequenz P22 P_{R} oder P_{trc} darstellt.

10. Gast-Vektor-System gemäß einem der Ansprüche 1 bis 7, wobei der Repressor C2, Lac I oder beides darstellt.

11. Gast-Vektor-System gemäß einem der Ansprüche 1 bis 7, wobei das wesentliche Gen asd, *murA*, *dapA* oder alr ist.

12. Gast-Vektor-System gemäß einem der Ansprüche 1 bis 7, welches weiterhin eine Mutation in einem Gen zur Steigerung der Immunogentzität umfasst, wobei die Mutation *ΔendA2311, ΔrelA1123,* ΔaraE25, ΔaraBAD1923, ΔaraBAD23, Δgmd-11 oder Δgmd-fcl-26 darstellt.

13. Mikroorganismus, welcher das Gast-Vektor-System gemäß einem der Ansprüche 3 bis 7 umfasst, für die Verwendung zur Lieferung eines gewünschten Genproduktes und/oder eines Nukleinsäurevektors an einen eukaryontischen Gast.

## Revendications

1. Système hôte-vecteur, comprenant :
a) un chromosome hôte comportant :
i) une première séquence de régulation activable, laquelle séquence de régulation activable est activable par un inducteur,
ii) une séquence codant un répresseur, laquelle séquence est opérationnellement liée à la première séquence de régulation activable,
iii) et au moins un gène essentiel, lequel gène essentiel code un polypeptide nécessaire à la formation d'une couche rigide de paroi de cellule de procaryote, et se trouve inactivé, opérationnellement lié à la première séquence de régulation activable ou opérationnellement lié à une deuxième séquence de régulation activable qui est activée par un deuxième inducteur ;
b) et un vecteur comportant :
i) au moins une origine de réplication ori,
ii) une cassette d'expression fonctionnant chez un eucaryote, comprenant :
1) une séquence-promoteur d'eucaryote,
2) un site adapté pour l'insertion d'un gène codant un produit de gène voulu,
3) et une séquence de polyadénylation,
iii) une séquence activateur-promoteur de procaryote,
iv) au moins un gène essentiel, lequel gène essentiel est nécessaire à la formation d'une couche rigide de paroi de cellule de procaryote, a pour rôle de complémenter le gène essentiel (a-iii), et se trouve opérationnellement lié à la séquence activateur-promoteur de procaryote (b-iii),
v) une séquence promoteur régulable de procaryote, répressible par le répresseur et orientée de manière à provoquer la synthèse d'un ARN anti-sens par rapport au gène essentiel (b-iv),
vi) au moins une séquence de terminaison de transcription, opérationnellement liée à ce gène essentiel, à l'origine de réplication et/ou à la cassette d'expression fonctionnant chez un eucaryote,
vii) et au moins un motif de séquence à CpG, lequel motif de séquence à CpG renforce l'immunogénicité.

2. Système hôte-vecteur, comprenant :
a) un chromosome hôte comportant :
i) une première séquence de régulation activable, laquelle séquence de régulation activable est activable par un premier inducteur,
ii) au moins une séquence codant au moins un répresseur, laquelle séquence est opérationnellement liée à la première séquence de régulation activable,
iii) et au moins un gène essentiel, lequel gène essentiel code un polypeptide nécessaire à la formation d'une couche rigide de paroi de cellule de procaryote, et se trouve inactivé, opérationnellement lié à la première séquence de régulation activable ou opérationnellement lié à une deuxième séquence de régulation activable qui est activée par un deuxième inducteur ;
b) et un vecteur comportant :
i) au moins une origine de réplication ori,
ii) une séquence activateur-promoteur de procaryote,
iii) au moins un gène essentiel, lequel gène essentiel est nécessaire à la formation d'une couche rigide de paroi de cellule de procaryote, a pour rôle de complémenter le gène essentiel (a-iii), et se trouve opérationnellement lié à la séquence activateur-promoteur de procaryote (b-ii),
iv) une première séquence promoteur régulable de procaryote, laquelle première séquence de régulation régulable est répressible par ledit premier répresseur et orientée de manière à provoquer la synthèse d'un ARN anti-sens par rapport au(x) gène(s) essentiel(s) (b-iii),
v) une deuxième séquence promoteur régulable de procaryote, laquelle deuxième séquence de régulation régulable est répressible par un deuxième répresseur codée par la séquence (a-ii),
vi) un site adapté pour l'insertion d'un gène codant un produit de gène voulu, lequel site est opérationnellement lié à la deuxième promoteur régulable de procaryote,
vii) et au moins une séquence de terminaison de transcription, opérationnellement liée au(x) gène(s) essentiel(s), à l'origine de réplication et/ou au site d'insertion de gène voulu.

3. Système hôte-vecteur, conforme à la revendication 1 ou 2, qui comprend en outre un gène codant un produit de gène voulu, inséré dans ledit site adapté pour l'insertion d'un gène codant un produit de gène voulu.

4. Système hôte-vecteur conforme à la revendication 3, dans lequel le gène code un antigène.

5. Système hôte-vecteur conforme à la revendication 4, ledit antigène provenant d'un agent pathogène bactérien, viral, fongique ou parasitaire.

6. Système hôte-vecteur conforme à la revendication 4, ledit antigène provenant d'*Elmeria,* du HBV ou de *Streptococcus pneumoniae.*

7. Système hôte-vecteur conforme à la revendication 3, qui comprend un premier vecteur comprenant un gène voulu dans une cassette d'expression fonctionnant chez un eucaryote et un deuxième vecteur comprenant un gène voulu dans une cassette d'expression fonctionnant chez un procaryote, le produit de gène voulu et le gène essentiel dudit premier vecteur étant différents du produit de gène voulu et du gène essentiel dudit deuxième vecteur.

8. Système hôte-vecteur conforme à l'une des revendications 1 à 7, dans lequel la séquence activateur-promoteur de procaryote est araC P_{BAD}.

9. Système hôte-vecteur conforme à l'une des revendications 1 à 7, dans lequel la séquence de régulation régulable est P_{R} de P22 ou P_{trc}.

10. Système hôte-vecteur conforme à l'une des revendications 1 à 7, dans lequel le répresseur est C2, Lac I, ou les deux.

11. Système hôte-vecteur conforme à l'une des revendications 1 à 7, dans lequel le gène essentiel est *asd, mur, dapA* ou *alr.*

12. Système hôte-vecteur conforme à l'une des revendications 1 à 7, qui comporte en outre une mutation dans un gène pour renforcer l'immuno-génicité, laquelle mutation est *ΔendA2311, ΔrelA1123, ΔaraE25, ΔaraBAD-1923, ΔaraBAD23, Δgmd11* ou *Δgmd-fcl-26.*

13. Microorganisme contenant un système hôte-vecteur conforme à l'une des revendications 3 à 7, utilisable pour délivrer un produit de gène voulu ou un acide nucléique vecteur à un hôte eucaryote.
